# EUROPEAN PATENT APPLICATION

(11) **EP 4 438 629 A1**
(43) Date of publication of application: **02.10.2024**
(21) Application number: 21965062.9
(22) Date of filing: 24.11.2021
(51) Int. Cl.: C07K 19/00, C07K 14/71, C12N 15/09, A61K 38/17, A61P 35/00

(54) **MULTISPECIFIC LIGAND BINDING MOLECULE AND APPLICATION THEREOF**

(71) Applicant: Suzhou Light Ferry Biomed. Co., Ltd, Suzhou, Jiangsu 215000 (CN); Hk Light Ferry Biomed. Limited, Kowloon, Hong Kong (HK)
(72) Inventor: CHANG, Chien-Hsing, Suzhou, Jiangsu 215000 (CN); YIN, Qingqing, Suzhou, Jiangsu 215000 (CN)
(74) Representative: Manfredi, Irene
(86) International application number: PCT/CN2021/132735
(87) International publication number: WO 2023/092324

(57) **Abstract**

The present invention relates to ligand-binding molecules that can bind to one or more growth factors including VEGF-A, VEGF-B, VEGF-C, VEGF-D, PlGF, PDGF-AA, PDGF-AB, PDGF-BB and PDGF-CC, and glycosylation-modified forms of the ligand-binding molecules, fusion proteins and compositions containing such ligand-binding molecules, as well as preparation methods and uses thereof.

## Description

### FIELD

The present invention relates to the field of proteins, and in particular relates to ligand-binding molecules, and glycosylation-modified forms, fusion proteins and conjugates of such ligand-binding molecules, nucleic acid sequences encoding such molecules, as well as preparation methods and uses thereof.

### BACKGROUND

The vascular endothelial growth factor (also known as VEGF) related gene family encompasses a variety of secreted glycoproteins, including VEGF-A, VEGF-B, VEGF-C, VEGF-D and VEGF-E. VEGF-A is a 45,000 Da glycoprotein with extensive angiogenic activity, and its gene is composed of 8 exons and 7 introns. VEGF-A may be divided into 5 monomers based on different splicing patterns, namely VEGF121, VEGF145, VEGF165, VEGF189 and VEGF206, among which VEGF165 is one of the most important homologous monomers of VEGF-A.

VEGF-A plays a significant role in early angiogenesis, and it can be expressed in all vascularized tissues. In addition, many other cells can also express VEGF-A under hypoxic stress conditions, such as macrophages, platelets, dendritic cells (DC), activated T cells, retinal pigment epithelial cells, Muller cells in the retina, astrocytes, osteoblasts, bronchial and alveolar epithelial cells, pericytes, vascular smooth muscle cells, myofibroblasts, keratinocytes, renal mesangial cells and tumor cells.

VEGF-C and VEGF-D play critical roles in embryonic and postnatal lymphangiogenesis. Mice homozygously deficient in the VEGF-C gene are fatal in the embryonic stage, and heterozygous deficiency leads to defective lymphatic vascular development in postnatal mice. VEGF-C and VEGF-D may also play an important role in neovascularization, especially during pathological stages such as tumor growth. VEGF-E is isolated from a group infected with sheep Orf virus, which can stimulate the proliferation and migration of endothelial cells, and also enhance the permeability of blood vessels (Vladimir Joukov et al., The EMBO Journal 15(2):290-98, Feb 1996).

VEGF ligands mediate angiogenesis through multiple different receptors (i.e., through ligand-receptor interactions). Two receptors were initially confirmed in endothelial cells as special receptor tyrosine kinase (RTK) VEGFR-1 (FIt-1) and VEGFR-2 (FiK-1). It was later confirmed that they were also expressed on a variety of hematopoietic cell lineages in adults. These two receptors consist of an extracellular region with seven immunoglobulin-like structures, a membrane region and a tyrosine kinase region, both are transmembrane receptors belonging to RTK type III, and their common feature is the presence of a tyrosine kinase insertion region within the catalytic domain, the activity of this tyrosine kinase is activated by the binding of receptors to ligands, and receptor phosphorylation can lead to a lot of intracellular enzymatic and other reactions that play an important role in cell growth and differentiation. Recently, an additional RTK, VEGFR3 (Fit-4), has been confirmed and found to be closely related to lymphangiogenesis. Different VEGF family members have different receptor binding sites.

Inhibition of angiogenesis is an effective treatment option for angiogenesis-related diseases such as tumor growth and ophthalmic vascular proliferation. Over the past few decades, extensive research has been conducted on VEGF-related signaling, and there have been many drugs used for treatment, such as Lucentis (Ranibizumab), Eylea (Aflibercept), Avastin (bevacizumab), Cyramza (Ramucirumab), Inlyta (Axitinib), Stivarga (regorafenib), Cometriq (cabozantinib), OFEV (Nintedanib) and Lenvim (envatinib).

Wet age-related macular degeneration (wAMD) and diabetic macular edema (DME) affect a wide range of patients, progress rapidly and cause serious consequences after onset of illness (vision loss), which are the most important inducements for blindness. The current main treatment modality is targeting VEGF-A, which has significantly improved therapeutic efficacy over traditional treatment means (laser photocoagulation, etc.), and the corresponding targeted drugs (e.g., Ranibizumab and Aflibercept) have also achieved huge market success. However, targeting VEGF-A alone is still unable to completely conquer these diseases, for example, after AMD treatment, only one-third of patients are able to fully benefit from the treatment, while the rest of patients obtain no significant increase in vision, and one-third of patients still have vision close to blindness (Rofagha S, Bhisitkul RB, Boyer DS, Sadda SR, Zhang K. Seven-Year Outcomes in Ranibizumab-Treated Patients in ANCHOR, MARINA, and HORIZON: A multicenter cohort study (SEVEN UP) [J]. Ophthalmology, 2013, 120(11):2292-2299). Currently, multiple drugs targeting VEGF-A on the market and in clinical trial have all demonstrated similar efficacy in clinical treatment, with affinities (K_{D}) ranging from pM to nM levels, indicating that the single targeted VEGF-A therapy may have reached the upper limit of its therapeutic efficacy.

In summary, although extensive research has been conducted on VEGF-related signaling in the past few decades, there are still obvious unmet clinical needs in related diseases such as neovascularization and tumors. The present invention presents a new solution to this need. The present invention provides novel molecules capable of targeting the VEGF, PIGF and PDGF families, which have important clinical significance for the treatment of neovascularization-related diseases.

### SUMMARY OF THE INVENTION

The present invention relates to ligand-binding molecules (i.e., multi-specific ligand-binding molecules) that can bind to one or more growth factors including VEGF-A, VEGF-B, VEGF-C, VEGF-D, PlGF, PDGF-A, PDGF-B and PDGF-C, and glycosylation modified forms thereof, compositions comprising such ligand-binding molecules, as well as preparation methods and uses thereof. The invention further validates effects of the ligand-binding molecules in vivo and in vitro, especially the effect for use in anti-angiogenesis.

In some aspects, the present disclosure provides an isolated ligand-binding molecule comprising an amino acid sequence derived from VEGFR2 and having an enhanced binding capacity to at least one, more preferably two, more preferably three, more preferably four, more preferably five, more preferably six, and most preferably seven of VEGF-A, VEGF-C, VEGF-D, PDGF-AA, PDGF-AB, PDGF-BB and PDGF-CC, as compared to native VEGFR2 (for example, native full VEGFR2 extracellular domain, such as extracellular sequence Met1-Glu764). The VEGFR2-derived sequence is an amino acid sequence that has at least 95% identity to any of the following amino acid sequences:
(a) Amino acid sequence defined by positions 117-218 of SEQ ID NO: 2 (e.g., LFV-A);
(b) Amino acid sequence defined by positions 117-327 of SEQ ID NO: 2 (e.g., LFV-B);
(c) Amino acid sequence defined by positions 123-327 of SEQ ID NO: 2 (e.g., LFV-C, LFV-G, LFV-H, LFV-I, LFV-J, LFV-K and LFV-L);
(d) Amino acid sequence defined by positions 117-421 of SEQ ID NO: 2 (e.g., LFV-D);
(e) Amino acid sequence defined by positions 23-327 of SEQ ID NO: 2 (e.g., LFV-E); and
(f) Amino acid sequence defined by positions 23-421 of SEQ ID NO: 2 (e.g., LFV-F).

In some embodiments, the amino acid sequence derived from VEGFR2 is or consists of an N-terminal and/or C-terminal truncated fragment of any one of (a)-(f), for example, with 20, 15, 10, 9, 8, 7, 6, 5, 4, 3, 2 or 1 amino acid(s) truncated.

In some embodiments, the amino acid sequence derived from VEGFR2 contains insertion, substitution or deletion of one or more amino acids, such as the insertion, deletion or substitution at glycosylation sites, as compared to the sequence at the corresponding position in the native VEGFR2 extracellular domain.

In some embodiments, various modifications can be performed at glycosylation sites to remove glycosylation, especially to eliminate known glycosylation sites such as "NXT" and/or "NXS" triamino acids, for example, by mutating N to Q or A. Specific glycosylation sites can be selected from, for example, the positions corresponding to amino acid positions 46, 66, 96, 143, 158, 245 and 318 of SEQ ID NO: 2. In some embodiments, Asn at the above positions is replaced with Gln or Ala or other suitable amino acids. Specifically, the substitution of glycosylation sites can be selected from the following: N143A, N143Q, N158A, N158Q, N245Q and N318Q.

In some embodiments, the amino acid sequence derived from VEGFR2 comprises or consists of an amino acid sequence selected from SEQ ID Nos: 4-15 and 18.

In some embodiments, the ligand-binding molecules comprise an amino acid sequence derived from VEGFR1 in addition to the amino acid sequence derived from VEGFR2. The VEGFR1-derived sequence can be selected from the following group:
(i) An amino acid sequence having at least 95% identity to the amino acid sequence as set forth at positions 132-230 of SEQ ID NO: 1; and
(ii) An N-terminal and/or C-terminal truncated amino acid sequence of the amino acid sequence of (i), for example, with 20, 15, 10, 9, 8, 7, 6, 5, 4, 3, 2 or 1 amino acid(s) truncated.

In some embodiments, the ligand-binding molecules comprise an amino acid sequence having at least 95% identity to the amino acid sequence as set forth at positions 117-327 or 123-327 of SEQ ID NO: 2 operably linked to the VEGFR1-derived sequence. For example, the ligand-binding molecules comprise or consist of an amino acid sequence selected from SEQ ID Nos: 16-17 and 19-21.

In some other embodiments, the ligand-binding molecules include an amino acid sequence derived from VEGFR3 in addition to the amino acid sequence derived from VEGFR2. The VEGFR3-derived sequence can be selected from the following group:
(i) An amino acid sequence having at least 95% identity to the amino acid sequence as set forth at positions 25-216, 25-115, 47-115, 154-210, 248-314 or 25-329 of SEQ ID NO: 3; and
(ii) An N-terminal and/or C-terminal truncated amino acid sequence of the amino acid sequence of (i), for example, with 20, 15, 10, 9, 8, 7, 6, 5, 4, 3, 2 or 1 amino acid(s) truncated.

In some other embodiments, the ligand-binding molecules comprise an amino acid sequence having at least 95% identity to the amino acid sequence as set forth at positions 117-327, 123-327 or 221-312 of SEQ ID NO: 2 operably linked to the VEGFR3-derived sequence. For example, the ligand-binding molecules comprise or consist of an amino acid sequence selected from SEQ ID Nos: 22-30.

In some embodiments, the ligand-binding molecules further comprise an Fc region of an immunoglobulin selected from the group consisting of IgA, IgM, IgE, IgD and IgG, including IgG1, IgG2, IgG3 and IgG4, preferably an Fc region of human IgG1 or a variant thereof, and optionally also comprises a hinge region. The hinge region and Fc region of the immunoglobulin may be operably linked to the C-terminal of the VEGFR2-derived sequence, VEGFR1-derived sequence, or VEGFR3-derived sequence, either via a linker (e.g., GPG) or without a linker. In some embodiments, the Fc region or variant thereof is present in a polymeric form comprising macromolecular compounds such as phospholipids.

In some embodiments, the Fc region is as set forth in the sequence of amino acids 104-330 of SEQ ID No: 31.

In some specific embodiments, the ligand-binding molecules disclosed herein comprise or consist of any sequence of SEQ ID Nos: 32-59.

In some embodiments, the ligand-binding molecules further comprise a signal peptide.

In some embodiments, the ligand-binding molecules can bind to at least one, preferably at least two, more preferably at least three, more preferably at least four, more preferably at least five, more preferably at least six, more preferably at least seven, more preferably at least eight, and more preferably all nine of VEGF-A, VEGF-B, VEGF-C, VEGF-D, PlGF, PDGF-AA, PDGF-AB, PDGF-BB and PDGF-CC proteins. Preferably, the ligand-binding molecules can bind to multiple targets, that is, they are multi-specific ligand-binding molecules. The VEGF-A, VEGF-B, VEGF-C, VEGF-D, PlGF, PDGF-AA, PDGF-AB, PDGF-BB and PDGF-CC proteins can be from or derived from a human, mouse, rat or cynomolgus monkey. For example, the above-mentioned proteins may be of human origin, including processed or unprocessed forms thereof, as well as phosphorylated or unphosphorylated forms thereof.

In some embodiments, the ligand-binding molecules can bind to at least one, preferably at least two, more preferably at least three, and most preferably all four of VEGF-A, VEGF-C, VEGF-D and PDGF-AB with a K_{D} of 5 nM or less, as determined by SPR.

In some embodiments, the ligand-binding molecules can bind to at least one of VEGF-A and VEGF-C with a K_{D} of 50 pM or less, preferably 40 pM or less, more preferably 30 pM or less, more preferably 20 pM or less, more preferably 10 pM or less, more preferably 5 pM or less, more preferably 4 pM or less, more preferably 3 pM or less, or more preferably 1 pM or less, as determined by SPR.

In some embodiments, the ligand-binding molecules can bind to VEGF-D with a K_{D} of 20 nM or less, preferably 15 nM or less, more preferably 10 nM or less, more preferably 5 nM or less, and even more preferably 3 nM or less, as determined by SPR.

In some embodiments, the ligand-binding molecules can bind to at least one, preferably at least two, preferably at least three, and more preferably all four of PDGF-AA, PDGF-AB, PDGF-BB and PDGF-CC with a K_{D} of 50 nM or less, preferably 40 nM or less, more preferably 30 nM or less, more preferably 28 nM or less, more preferably 26 nM or less, more preferably 24 nM or less, more preferably 22 nM or less, more preferably 20 nM or less, more preferably 18 nM or less, more preferably 15 nM or less, and even more preferably 10 nM or less, as determined by SPR.

In some embodiments, the ligand-binding molecules can inhibit or block the binding of at least one of VEGF-A, VEGF-B, VEGF-C, VEGF-D, PlGF, PDGF-AA, PDGF-AB, PDGF-BB and PDGF-CC proteins to their corresponding receptors.

In some embodiments, the receptor is selected from VEGFR-1 receptor, VEGFR-2 receptor, VEGFR-3 receptor, PDGFR-α receptor and PDGFR-β receptor. The receptor can be present in the form of a monomer, a homodimer, or any heterodimer formed between each other, depending on the binding form in which they function in physiology and pathology.

In some aspects, the present invention also provides a fusion protein comprising the ligand-binding molecules disclosed above operably linked to a heterologous peptide. The heterologous peptide can be selected from the following: Additional ligand-binding molecules, such as the extracellular domain (ECD) of common human receptor proteins or truncated and recombinant peptides thereof; antibody variable region fragments and combinations thereof, such as antibody Fab, single-chain antibody (scFv), single-domain antibody (nanobody), human heavy chain variable region single-domain antibody (V_{H}); active polypeptides and protein aptamers.

In some embodiments, the heterologous peptide is a heterologous peptide targeting VEGF-A, VEGF-B, VEGF-C, VEGF-D, PDGF-AA, PDGF-AB, PDGF-BB, PDGF-CC, PDGF-DD, Ang-2, TGF-β, CTGF (connective tissue growth factor), EGF (epidermal growth factor), S1P, Galectin, Decorin, or a receptor for the aforementioned protein.

In some embodiments, the operable linkage is either a direct linkage or a linkage via a linker.

In some embodiments, the ligand-binding molecule is linked to the N-terminal or C-terminal of the heterologous peptide directly or through a linker. For example, it is directly linked to the N-terminal of the heterologous peptide through a peptide bond at its C-terminal, or directly linked to the C-terminal of the heterologous peptide through a peptide bond at its N-terminal. In some embodiments, the linker is a polypeptide or chemical group.

In some aspects, the invention also provides a conjugate comprising the ligand-binding molecule or fusion protein as described above conjugated to at least one moiety selected from the following: a modification moiety, a toxin (e.g., a chemotherapeutic agent), a detectable label (e.g., a radioactive isotope, a lanthanide element, a luminescent label, a fluorescent label, or an enzyme-substrate label), or a purification moiety. For example, the modification moiety is a polyethylene glycol moiety, and optionally the polyethylene glycol moiety is attached to the amino terminal of the ligand-binding molecules.

In some aspects, the invention also provides an isolated nucleic acid molecule comprising a polynucleotide sequence encoding the ligand-binding molecule or fusion protein. The nucleic acid molecule may further comprise a promoter sequence or other regulatory sequence(s) linked to the polynucleotide sequence.

In some aspects, the invention also provides a vector comprising the nucleic acid molecule(s). The vector may include, but is not limited to, a lentiviral vector, an adeno-associated viral vector, an adenoviral vector, a liposomal vector and any combination thereof. In some embodiments, the vector is a replication-deficient adenoviral vector, wherein the nucleic acid molecule is operably linked to a promoter and flanked by adenoviral polynucleotide sequences.

In some aspects, the present invention also provides a host cell transformed or transfected with the polynucleotide or vector described above. The host cell is preferably a eukaryotic cell, such as a Chinese hamster ovary (CHO) cell or a human cell.

In some aspects, the invention also provides a method for preparing the ligand-binding molecules, which comprises the following steps:
Culturing the host cell as described above under suitable conditions to express the ligand-binding molecule; and isolating the ligand-binding molecule from the culture supernatant of the host cell.

In some aspects, the present invention also provides a pharmaceutical composition comprising the ligand-binding molecule or fusion protein or nucleic acid molecule encoding the ligand-binding molecule as disclosed above, as well as a pharmaceutically acceptable adjuvant or carrier, such as a diluent or excipient. The composition may be formulated for topical administration or intravitreal injection or implant administration. The composition can be in the form of solid, paste, ointment, gel, liquid, aerosol, spray, polymer, film, emulsion or suspension.

In some aspects, the present invention also provides a method for preventing, inhibiting and/or treating neovascularization and diseases or conditions caused by neovascularization in a subject, comprising administering the ligand-binding molecule or pharmaceutical composition as disclosed herein to the subject in an amount effective to inhibit neovascularization in the subject.

In some embodiments, the neovascularization is retinal neovascularization and/or choroidal neovascularization.

In some embodiments, the disease or condition is selected from choroidal vasculopathy or neovascularization, retinal neovascularization, macular neovascularization and ocular disorders associated with fundus leakage.

In some embodiments, the composition is administered topically to the eye of the subject, e.g. it is administered by intravitreal injection, intravitreal implant, or by topical administration.

In some aspects, the present invention also provides use of the ligand-binding molecules or fusion proteins in the manufacture of a medicament for treating neovascularization and diseases or conditions caused by neovascularization in a subject.

In some aspects, the present invention also provides the ligand-binding molecules or fusion proteins for use in treating neovascularization and diseases or conditions caused by neovascularization in a subject.

In some aspects, the invention provides a kit comprising the ligand-binding molecule or fusion protein or pharmaceutical composition as described herein.

The foregoing is an overview and thus simplifications, generalizations, and omissions of details are included if necessary; consequently, those skilled in the art will appreciate that the overview is illustrative only and is not intended to be in any way limiting. Other aspects, features and advantages of the methods, compositions and uses and/or other subject matters described herein will become apparent in the teachings set forth herein.

### DESCRIPTION OF THE DRAWINGS

Figure 1 shows ELISA results for the binding of various fusion protein ligand-binding molecules derived from VEGFR2 and VEGFR3 to VEGF-A165 (Figure 1a), VEGF-C (Figure 1b) or VEGF-D (Figure 1c).
Figure 2 shows ELISA results for the binding of various ligand-binding molecules derived from VEGFR2 to VEGF-C.
Figures 3-4 show the blocking ELISA results for various ligand-binding molecules on the binding between VEGFR2-VEGF C (Figure 3a), between VEGFR2-VEGF D (Figure 3b), between VEGFR3-VEGF C (Figure 4a) and between VEGFR3-VEGF D (Figure 4b), respectively.
Figure 5 shows ELISA results for the binding of various ligand-binding molecules derived from VEGFR2 to VEGF-A 165 (Figure 5a) and VEGF-A 121 (Figure 5b).
Figure 6 shows the blocking ELISA results for various ligand-binding molecules on the binding between VEGFR2-VEGFA165 (Figure 6a) and between VEGFR2-VEGF-A121 (Figure 6b), respectively.
Figure 7 shows pharmacokinetic results for ligand-binding molecules in mice.
Figure 8 shows the therapeutic effect of ligand-binding molecules in a rat CNV model.
Figure 9 shows the blocking ELISA results for the ligand-binding molecules with removal of glycosylation modifications on VEGFA 165-VEGFR2 (Figure 9a), VEGFC-VEGFR2 (Figure 9b), VEGFD-VEGFR2 (Figure 9c) and VEGFD-VEGFR3 (Figure 9d).
Figure 10 shows the blocking ELISA results for the ligand-binding molecules derived from VEGFR1 and VEGFR2 on VEGFA165-VEGFR2 (Figure 10a), VEGFA165-VEGFR1 (Figure 10b), VEGFC-VEGFR2 (Figure 10c), VEGFD-VEGFR2 (Figure 10d) and VEGFD-VEGFR3 (Figure 10e).
Figure 11 shows ELISA results for the binding of ligand-binding molecules derived from VEGFR1 and VEGFR2 to VEGF-B (Figure 11a) and PIGF (Figure 11b).
Figure 12 shows the blocking ELISA results for the fused and glycosylation-modified ligand-binding molecules on VEGFA165-VEGFR2 (Figure 12a), VEGFC-VEGFR2 (Figure 12b) and VEGFD-VEGFR2 (Figure 12c).
Figure 13 shows ELISA results for the binding of LFV-Q to VEGF-B (Figure 13a) and PlGF (Figure 13b).
Figure 14 shows the detection results of SEC single peak purity following one-step purification for LFV-M before and after glycosylation site removal.
Figure 15 shows the blocking ELISA results for LFV-Q, LFV-S, LFV-M and LFV-R molecules on VEGFA165-VEGFR2 (Figure 15a), VEGFA165-VEGFR1 (Figure 15b), VEGFC-VEGFR2 (Figure 15c) and VEGFD-VEGFR2 (Figure 15d).
Figure 16 shows ELISA results for the binding of LFV-Q, LFV-S, LFV-M and LFV-R to VEGF-B (Figure 16a) and PIGF (Figure 16b).
Figure 17 shows the ELISA binding potency of LFV-Q, LFV-B, LFV-R and LFV-C, as compared with VEGFR2-Fc, to VEGF-A (Figure 17a), VEGF-C (Figure 17b) and VEGF-D (Figure 17c).
Figures 18a-18b show experimental results for the inhibitory effect of LFV-R and LFV-Q on the proliferation of HUVEC cells.
Figures 19a-19b show experimental results for the therapeutic effect of LFV-R and LFV-Q on laser-induced choroidal neovascularization in rats.

### DETAILED DESCRIPTION OF THE INVENTION

Unless defined otherwise, all technical and scientific terms used herein have the same meaning as commonly understood by one of ordinary skill in the art to which the present disclosure belongs. All patents, patent applications, and other publications cited herein are incorporated by reference in their entirety. To the extent that definitions set forth herein conflict with those set forth in patents, patent applications, and other publications incorporated herein by reference, the definitions set forth herein shall control.

As used herein, the term "VEGF" refers to the VEGF family, which consists of VEGF glycoproteins (VEGF-A, VEGF-B, VEGF-C, VEGF-D and VEGF-E). VEGF-A encompasses any form of VEGF-A, including the native protein of VEGF-A (processed or unprocessed forms), full-length VEGF-A, fragments (e.g., truncated forms, extracellular/transmembrane domains) or variants (e.g., splice variants, allelic variants or engineered variants) thereof, and modified forms (e.g., glycosylated) thereof. There are five isoforms of VEGF-A in the human body based on different splicing patterns, namely VEGF121, VEGF145, VEGF165, VEGF189 and VEGF206, of which VEGF165 is the most important homologous monomer of VEGF-A. Similarly, VEGF-B, VEGF-C and VEGF-D also encompass their respective native proteins (including processed or unprocessed forms), full-length or fragments (e.g., truncated forms, extracellular/transmembrane domains) or variants (e.g., splice variants, allelic variants or engineered variants) and their modified forms (e.g., glycosylated).

As used herein, the term "vascular endothelial growth factor receptor" or "VEGF receptor" includes VEGFR1, VEGFR2 and VEGFR3, which are members of the receptor tyrosine kinase family. The three receptors comprise a large extracellular region consisting of 6 or 7 immunoglobulin (Ig)-like domains (referred to as D1-D7, respectively), a single transmembrane (TM) helix and a cytoplasmic region with tyrosine kinase activity, as well as additional regulatory sequences. References to "VEGFR2", "VEGFR1" and "VEGFR3" may, depending on the context, refer herein to the full-length or to all or part of the extracellular domain thereof. Examples of full-length amino acid sequences of native proteins of human VEGFR2 and VEGFR3 are SEQ ID No: 2 and SEQ ID No: 3, respectively. All VEGF-A isoforms can bind to both VEGFR1 and VEGFR2, VEGF-B specifically binds and activates VEGFR-1, and the specificity of VEGF-E is related to VEGFR-2. VEGF-C and VEGF-D mainly act by binding to VEGFR3, and can also bind to VEGFR2, although this binding is weaker than that to VEGFR3. VEGF-A can also bind to VEGFR2, but again this binding is weaker than that to VEGFR1. Previously, due to the fact that VEGFR2 can bind to multiple VEGF molecules and its specificity was considered to be inferior to VEGFR1, the development of Aflibercept was mainly based on VEGFR1, whereas VEGFR2, especially its binding properties to VEGF-C and VEGF-D, has not attracted sufficient attention and exploration so far.

As used herein, the term "platelet-derived growth factor" or "PDGF" belongs to the family of vascular endothelial growth factors, and is a sugar chain-containing polypeptide growth factor with a relative molecular weight of about 30 kD. Generally, PDGF is a homodimer or heterodimer molecule formed by four polypeptide chains A, B, C and D through disulfide bonds, and there are five isoforms, namely PDGF-AA, BB, AB, CC and DD. PDGF was first discovered in the α-granules of platelets, and currently it has been proven that PDGF can be produced by diploid cells, such as monocytes, vascular smooth muscle cells, endothelial cells, embryonic cells, mesangial cells (MC) and collecting duct cells. PDGFs promote the migration, recruitment and distribution of pericytes, of which PDGF-B is a vital regulatory gene for vascular maturation and stabilization during the development of the body. PDGF-A, B and C have a certain promoting effect on lymphangiogenesis, especially PDGF-B is believed to have a strong promoting effect on lymphangiogenesis, and have a certain relationship with angiogenesis. In addition, PDGF is the earliest discovered connective tissue growth factor, which can promote the growth and differentiation of endothelial cells, glial cells, fibroblasts and vascular smooth muscle cells, and thus has been used clinically in the repair treatment of traumas.

As used herein, the terms "PDGF-AA", "PDGF-AB", "PDGF-BB" and "PDGF-CC" refer to the dimeric forms AA, AB, BB, CC of PDGF as well as fragments or variants thereof. They generally bind as homo- or heterodimers fixed by disulfide bonds to PDGF receptors and play their functions. PDGF A and B chains can form both homodimers and heterodimers. PDGF C chain mainly functions in the form of a homodimer. PDGF has been shown to be an important mitogenic factor with the ability to stimulate the division and proliferation of specific cell populations, and play an important role in the pathological fibrosis of tissues.

PDGF receptor is composed of two subunits α and β, with three dimer forms including αα, αβ and ββ, all of which can bind to PDGF molecules. The α unit mainly recognizes PDGF A chain, B chain and C chain, and thus the αα receptor can bind to PDGF-AA, AB, BB and CC. The β unit mainly recognizes PDGF B chain, and thus the ββ receptor mainly binds to PDGF-BB. The αβ receptor can bind to PDGF-AB and BB. After binding to PDGF receptor, PDGF molecule will promote the dimerization of the receptor, which in turn transmits signals downstream to produce effects such as promoting cell proliferation and stimulating cell chemotaxis in an autocrine and paracrine manner, mainly in vascular smooth muscle cells, fibroblasts, glial cells and other cells.

As used herein, the term "operably linked" refers to a juxtaposition (with or without a spacer or linker or insertion sequence) of two or more biological sequences of interest such that they are in a relationship permitting them to function in their intended manner. When used with respect to polypeptides, it is intended to mean that the polypeptide sequences are linked in such a way that permits the linked product to have the intended biological function. For example, a ligand-binding molecule may be operably linked to a constant region of immunoglobulin so as to provide for a stable product with ligand-binding activity. For another example, a ligand-binding molecule may be operably linked to a constant region of immunoglobulin through an insertion sequence therebetween, and such insertion sequences can be spacers or can contain longer sequences.

As used herein, the term "fusion" or "fused" when used with respect to amino acid sequences (e.g., peptide, polypeptide or protein) refers to combination of two or more amino acid sequences, for example by chemical bonding or recombinant means, into a single amino acid sequence which does not exist naturally. A fusion amino acid sequence may be produced by genetic recombination of two encoding polynucleotide sequences, and can be expressed by a method of introducing a construct containing the recombinant polynucleotides into a host cell.

As used herein, the term "isolated" refers to a state obtained from natural state by artificial means. If a certain "isolated" substance or component is present in nature, it is possible because its natural environment changes, or the substance is isolated from the natural environment, or both. For example, a certain unisolated polynucleotide or polypeptide naturally exsits in a certain living animal body, and the same polynucleotide or polypeptide with a high purity isolated from such a natural state is called isolated polynucleotide or polypeptide. The term "isolated" excludes neither the mixed artificial or synthesized substance nor other impure substances that do not affect the activity of the isolated substance.

The term "identity", as used herein, refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAMJ. Applied Math. 48:1073.

As used herein, the term "specific binding" or "specifically bind" refers to a non-random binding reaction between two molecules, such as a reaction between a ligand and a ligand-binding molecule. In certain embodiments, the ligand-binding molecules provided herein specifically bind to human VEGF-A and/or human PDGF with a binding affinity (K_{D} or written as KD) of ≤10⁻⁶ M (for example, ≤5×10⁻⁷ M, ≤2×10⁻⁷ M, ≤10⁻⁷ M, ≤5×10⁻⁸ M, ≤2×10⁻⁸ M, ≤10⁻⁸ M, ≤5×10⁻⁹ M, ≤4×10⁻⁹ M, ≤3×10⁻⁹ M, ≤2×10⁻⁹ M, or ≤10⁻⁹ M). As provided herein in the SPR experiment, K_{d} is the dissociation constant, Kₐ is the association constant, and K_{D} is the equilibrium dissociation constant (K_{d}/Kₐ). K_{D} can be determined using any routine method known in the art, including, but not limited to, surface plasmon resonance method, microscale thermophoresis method, HPLC-MS method and flow cytometry (e.g., FACS) method.

The ability to "block binding" as used herein refers to the ability of the ligand-binding molecules of the invention to inhibit the binding interaction between two molecules (for example, VEGF-A, C, D and VEGFR2, VEGF-C, D and VEGFR3) to any detectable degree. In certain embodiments, the ligand-binding molecules that block the binding between two molecules inhibit the binding interaction between the two molecules by at least 85% or at least 90%. In certain embodiments, the inhibition may be greater than 85%, or greater than 90%.

As used herein, the term "an effective amount" pertains to that amount of an active compound, or a material, composition or dosage form comprising an active compound, which is effective for producing some desired therapeutic effect, commensurate with a reasonable benefit/risk ratio, when administered in accordance with a desired treatment regimen. For instance, an effective amount is used herein to refer to an amount effective to inhibit neovascularization in a subject, an amount required to effectively inhibit at least one of VEGF-A, VEGF-B, VEGF-C, VEGF-D, PlGF, PDGF-AA, PDGF-BB, PDGF-AB and PDGF-CC in a subject (e.g., eyes), or an amount that stimulates the expression of at least one of VEGFR-1, VEGFR-2, VEGFR-3 and the PDGF receptor family in ocular cells or ocular blood vessels.

### I. Ligand-binding molecules comprising an amino acid sequence derived from VEGFR2

In some respects, the invention provides an isolated ligand-binding molecule capable of specifically binding to one or more VEGF/PDGF molecules. The VEGF/PDGF molecule may be selected from the group consisting of VEGF-A, VEGF-B, VEGF-C, VEGF-D, PlGF, PDGF-AA, PDGF-AB, PDGF-BB and PDGF-CC.

Preferably, the ligand-binding molecules are capable of specifically binding to at least two, more preferably at least three, more preferably at least four, more preferably at least five, more preferably at least six, more preferably at least seven, and even more preferably at least eight of VEGF-A, VEGF-B, VEGF-C, VEGF-D, PlGF, PDGF-AA, PDGF-AB, PDGF-BB and PDGF-CC. Most preferably, the ligand-binding molecules have specific binding affinity to all of VEGF-A, VEGF-B, VEGF-C, VEGF-D, PlGF, PDGF-AA, PDGF-AB, PDGF-BB and PDGF-CC.

As used herein, the expression "specifically bind(s) to..." or "specific binding affinity to...", when referring to a growth factor, includes not only specific binding to a circulating, active form of the growth factor, but also other forms of the growth factor. By way of example, VEGF-A have multiple isoforms, some of which circulate and others associate with heparin sulfate proteoglycans on cell surfaces. Ligand-binding molecules that specifically bind to VEGF-A bind to at least one circulating isoform, preferably all circulating isoforms, and more preferably bind to other isoforms as well. Therefore, references to the specific binding of ligand-binding molecules to VEGF-A encompass their ability to specifically bind to VEGF-A121, VEGF-A145, VEGF-A165, VEGF-A189 and VEGF-A206. By way of another example, VEGF-C is translated as a precursor molecule with extended amino terminal and carboxy terminal propeptide, which is cleaved to yield a "fully processed" form of VEGF-C. Ligand-binding molecules that specifically bind to VEGF-C encompasses binding to the fully processed form of VEGF-C, and preferably also encompasses binding to partly processed forms and unprocessed forms thereof.

In some embodiments, the ligand-binding molecule comprises one or more amino acid sequences derived from native VEGFR2, and its binding affinity to one or more of VEGF-A, VEGF-B, VEGF-C, VEGF-D, PlGF, PDGF-AA, PDGF-AB, PDGF-BB and PDGF-CC is significantly improved compared with that of the full extracellular domain of native VEGFR2 or a control ligand-binding molecule. For example, the binding affinity of the ligand-binding molecules to one or more of VEGF-A, VEGF-C and VEGF-D is significantly improved compared with that of the full extracellular domain of native VEGFR2 or a control ligand-binding molecule.

Preferably, the VEGF-A, VEGF-B, VEGF-C, VEGF-D, PlGF, PDGF-AA, PDGF-AB, PDGF-BB and PDGF-CC are from or derived from a mammal such as a human, a murine and a monkey, and more preferably, they are human native VEGF-A, VEGF-B, VEGF-C, VEGF-D, PlGF, PDGF-AA, PDGF-AB, PDGF-BB and PDGF-CC, respectively, including various isoforms, partly processed or unprocessed prepro-forms thereof.

In some embodiments, one or more of the VEGF-A, VEGF-B, VEGF-C, VEGF-D, PlGF, PDGF-AA, PDGF-AB, PDGF-BB and PDGF-CC are present as free soluble proteins or in anchored forms expressed on the cell surface.

The present invention also encompasses that the ligand-binding molecules are able to bind to aberrantly highly expressed forms of VEGF-A, VEGF-B, VEGF-C, VEGF-D, PlGF, PDGF-AA, PDGF-AB, PDGF-BB and PDGF-CC, thereby promoting the body to return to normal functions via binding to such aberrantly highly expressed growth factors. As known in the art, some of the VEGF/PDGF/PlGF growth factors are overexpressed in pathological states, causing these growth factors to exhibit aberrant functions in body tissues.

In some embodiments, the ligand-binding molecules preferably bind to human VEGF-A and/or human VEGF-C with a K_{D} of about 1 nM or less (for example, 500 pM, 400 pM, 300 pM, 200 pM, 100 pM, 50 pM, 10 pM, 5 pM, 1 pM or less). The ligand-binding molecules preferably bind to at least one of human VEGF-D, human PDGF-AB and human PDGF-BB with a K_{D} of about 10 nM or less (for example, 5 nM, 1 nM, 500 pM, 400 pM, 300 pM, 200 pM, 100 pM, 50 pM, 10 pM or less). The ligand-binding molecules preferably bind to human PDGF-AA and/or human PDGF-CC with a K_{D} of about 50 nM or less (for example, 30 nM, 10 nM, 5 nM, 1 nM, 500 pM, 400 pM, 300 pM, 200 pM, 100 pM, 50 pM, 10 pM or less).

In some embodiments, the ligand-binding molecules can bind to at least one of VEGF-A and VEGF-C with a K_{D} of 50 pM or less, preferably 40 pM or less, more preferably 30 pM or less, more preferably 20 pM or less, and even more preferably 10 pM or less, as determined by SPR.

In some embodiments, the ligand-binding molecules can bind to VEGF-D with a K_{D} of 20 nM or less, preferably 15 nM or less, more preferably 10 nM or less, more preferably 5 nM or less, and even more preferably 3 nM or less, as determined by SPR.

In some embodiments, the ligand-binding molecules can bind to VEGF-C with an EC₅₀ of 1 nM or less, preferably 0.5 nM or less, more preferably 0.4 nM or less, more preferably 0.3 nM or less, and even more preferably 0.2 nM or less; can bind to VEGF-A (e.g., VEGF-A165) with an EC₅₀ of 1 nM or less, preferably 0.5 nM or less, more preferably 0.4 nM or less, more preferably 0.3 nM or less, more preferably 0.2 nM or less, and even more preferably 0.1 nM or less, as determined by ELISA.

In some embodiments, the ligand-binding molecules can bind to at least one, preferably at least two, preferably at least three, and more preferably all four of PDGF-AA, PDGF-AB, PDGF-BB and PDGF-CC with a K_{D} of 50 nM or less, preferably 40 nM or less, more preferably 30 nM or less, more preferably 28 nM or less, more preferably 26 nM or less, more preferably 24 nM or less, more preferably 22 nM or less, more preferably 20 nM or less, more preferably 18 nM or less, more preferably 15 nM or less, and even more preferably 10 nM or less, as determined by SPR.

In some embodiments, the ligand-binding molecules disclosed herein are capable of blocking the binding between one or more growth factors and receptors selected from the following:
(a) VEGF-A and VEGFR-1, VEGFR-2 or PDGFR-α;
(b) VEGF-B, PlGF and VEGFR-1;
(c) VEGF-C and VEGFR-2 or VEGFR-3;
(d) VEGF-D and VEGFR-2 or VEGFR-3;
(e) PDGF-A and PDGFR-α;
(f) PDGF-B and PDGFR-α or PDGFR-β;
(g) PDGF-C and PDGFR-α;
(h) PDGF-D and PDGFR-β.

Optionally, the ligand-binding molecules are capable of blocking the binding between one or more growth factors and receptors selected from the following:
(a) binding of any one of VEGF-A, B, C, D with PDGFR-α or PDGFR-β;
(b) binding of any one of PDGF-A, B, C, D with any one of VEGFR-1, VEGFR-2, VEGFR-3, PDGFR-α and PDGFR-β.

As verified in the Examples, the ligand-binding molecules disclosed herein can block the binding of VEGF-C or VEGF-D to VEGFR-2 or VEGFR-3 with a lower EC₅₀ (for example, below 1 nM, below 0.5 nM, below 0.4 nM) compared to a native VEGFR2 molecule or a control ligand-binding molecule.

As known in the art, any one or more of VEGFR-1, VEGFR-2, VEGFR-3, PDGFR-α and PDGFR-β can bind to VEGF/PDGF growth factors in a homodimerized form or in any heterodimerized form, thereby being activated. Take human VEGFR-2 as an example, its correspondingly formed dimerized forms include, but are not limited to, human VEGFR2-human VEGFR2, human VEGFR2-human VEGFR1, human VEGFR2-human VEGFR3, human VEGFR2-human PDGFRα, and human VEGFR2-human PDGFRβ dimers. Accordingly, references herein to blocking the binding between a growth factor and a receptor encompass blocking the binding between the growth factor and the receptor in various forms (e.g., monomer, homodimer or heterodimer).

In some embodiments, the ligand-binding molecules comprise one or more amino acid sequences derived from VEGFR2 (especially the extracellular region of VEGFR2), and the amino acid sequences derived from VEGFR2 are fragments of VEGFR2 or amino acid sequences having at least 80% identity thereto. Specifically, the fragment of VEGFR2 can be selected from the following:
(a) Amino acid sequence defined by positions 20-116 of SEQ ID NO: 2, or amino acid sequence defined by positions 46-110 of SEQ ID NO: 2;
(b) Amino acid sequence defined by positions 117-218 of SEQ ID NO: 2, or amino acid sequence defined by positions 141-207 of SEQ ID NO: 2;
(c) Amino acid sequence defined by positions 219-327 of SEQ ID NO: 2, or amino acid sequence defined by positions 224-320 of SEQ ID NO: 2;
(d) Amino acid sequence defined by positions 328-421 of SEQ ID NO: 2, or amino acid sequence defined by positions 328-414 of SEQ ID NO: 2; and
(e) An N-terminal or C-terminal truncated or extended fragment of (a), (b), (c) or (d) (for example, with no more than 20, 15, 10, 9, 8, 7, 6, 5, 4, 3, 2 or 1 amino acid(s) truncated or extended).

The ligand-binding molecules may comprise two or more amino acid sequences derived from the VEGFR2 extracellular region. In some embodiments, the amino acid sequence derived from the VEGFR2 extracellular region is a fragment of the VEGFR2 (preferably human VEGFR2) extracellular region. Two or more amino acid sequences derived from the VEGFR2 extracellular region can be linked to each other directly or via a spacer, alternatively, in the case where the ligand-binding molecule is a double-stranded dimer, they can be on two separate chains.

In some embodiments, the ligand-binding molecules comprise an N-terminal truncated fragment of (b), for example, an amino acid sequence defined by positions 123-218 of SEQ ID NO: 2. In some embodiments, the ligand-binding molecules comprise both amino acid sequences of (b) and (c), for example, the amino acid sequence of (b) directly linked to the amino acid sequence of (c), i.e., the amino acid sequence defined by positions 117-327 of SEQ ID NO: 2. In some embodiments, the ligand-binding molecules comprises both the N-terminal truncated fragment of (b) and the amino acid sequence of (c), for example, the N-terminal truncated fragment of (b) directly linked to the amino acid sequence of (c), i.e., the amino acid sequence defined by positions 123-327 of SEQ ID NO: 2.

In some embodiments, the ligand-binding molecules described herein comprise the amino acid sequence as set forth at positions 20-116 of SEQ ID NO:2, the amino acid sequence as set forth at positions 117-218 of SEQ ID NO:2, the amino acid sequence as set forth at positions 219-327 of SEQ ID NO:2, the amino acid sequence as set forth at positions 328-421 of SEQ ID NO:2, or amino acid sequences having at least 80%, or at least 85%, or at least 90%, or at least 92%, or at least 95%, or at least 96%, or at least 97%, or at least 98%, or at least 99% identity with the above sequences.

In some embodiments, the ligand-binding molecules described herein comprise the amino acid sequence as set forth at positions 117-327 of SEQ ID NO:2, the amino acid sequence as set forth at positions 23-327 of SEQ ID NO:2, the amino acid sequence as set forth at positions 117-421 of SEQ ID NO:2, the amino acid sequence as set forth at positions 23-421 of SEQ ID NO:2, or amino acid sequences having at least 80%, or at least 85%, or at least 90%, or at least 92%, or at least 95%, or at least 96%, or at least 97%, or at least 98%, or at least 99% identity with the above sequences.

In some embodiments, the amino acid sequences with identity are N-terminal and/or C-terminal truncated and/or extended fragments of the amino acid sequence as set forth at positions 20-116 of SEQ ID NO:2, the amino acid sequence as set forth at positions 117-218 of SEQ ID NO:2, the amino acid sequence as set forth at positions 219-327 of SEQ ID NO:2, the amino acid sequence as set forth at positions 328-421 of SEQ ID NO:2, the amino acid sequence as set forth at positions 117-327 of SEQ ID NO:2, the amino acid sequence as set forth at positions 23-327 of SEQ ID NO:2, the amino acid sequence as set forth at positions 117-421 of SEQ ID NO:2, or the amino acid sequence as set forth at positions 23-421 of SEQ ID NO:2. Preferably, the truncation or extension is no more than 40 amino acids, such as no more than 30 amino acids, no more than 20 amino acids, no more than 15 amino acids, no more than 10 amino acids, no more than 8 amino acids, no more than 6 amino acids, no more than 5 amino acids, no more than 4 amino acids, no more than 3 amino acids, no more than 2 amino acids or no more than 1 amino acid. In some specific embodiments, the ligand-binding molecules described herein comprise the amino acid sequence as set forth at positions 123-218 of SEQ ID NO:2 or the amino acid sequence as set forth at positions 123 -327 of SEQ ID NO:2.

In some embodiments, the amino acid sequences with identity contain one or more amino acid modifications, such as amino acid substitutions, insertions or deletions, as compared to the amino acid sequence as set forth at positions 20-116 of SEQ ID NO:2, the amino acid sequence as set forth at positions 117-218 of SEQ ID NO:2, the amino acid sequence as set forth at positions 219-327 of SEQ ID NO:2, the amino acid sequence as set forth at positions 328-421 of SEQ ID NO:2, the amino acid sequence as set forth at positions 117-327 of SEQ ID NO:2, the amino acid sequence as set forth at positions 23-327 of SEQ ID NO:2, the amino acid sequence as set forth at positions 117-421 of SEQ ID NO:2, or the amino acid sequence as set forth at positions 23-421 of SEQ ID NO:2. The amino acid modification may be a mutation at a glycosylation site to eliminate the glycosylation site, such as to eliminate an N-glycosylation site or an O-glycosylation site. In some embodiments, the modification is a mutation at one or more of positions Asn46, Asn66, Asn96, Asn143, Asn158, Asn245 and Asn318 of SEQ ID NO:2.

### II. Ligand-binding molecules comprising amino acid sequences derived from VEGFR2 and other receptors

In some aspects, the ligand-binding molecules of the present invention comprise, in addition to the amino acid sequence derived from VEGFR2, amino acid sequences derived from other receptors, which improves binding to or blocking of at least one of VEGF-A, VEGF-B, VEGF-C, VEGF-D, PlGF, PDGF-AA, PDGF-AB, PDGF-BB and PDGF-CC. Other receptors can be other receptor tyrosine kinases, such as VEGFR-1, VEGFR-3, PDGFR-α and/or human PDGFR-β.

In some embodiments, the ligand-binding molecules of the present disclosure comprise one or more amino acid sequences derived from VEGFR2 and one or more amino acid sequences derived from VEGFR3 (for example, human VEGFR3, specifically the extracellular region of VEGFR3). Specifically, the amino acid sequence derived from VEGFR2 is operably linked to the amino acid sequence derived from VEGFR3. The operable linkage may be a direct linkage to each other or a linkage via a spacer.

In some embodiments, the amino acid sequence derived from VEGFR3 is a fragment of VEGFR3 or an amino acid sequence having at least 80% identity thereto. Specifically, the fragment of VEGFR3 can be selected from the following: Amino acid sequence defined by positions 25-115 of SEQ ID NO: 3; Amino acid sequence defined by positions 154-210 of SEQ ID NO: 3; Amino acid sequence defined by positions 248-314 of SEQ ID NO: 3; Amino acid sequence defined by positions 25-210 of SEQ ID NO: 3; Amino acid sequence defined by positions 154-314 of SEQ ID NO: 3; Amino acid sequence defined by positions 25-314 of SEQ ID NO: 3; and an N-terminal or C-terminal truncated and/or extended fragment of any of the above (for example, with no more than 40, 30, 20, 15, 10, 9, 8, 7, 6, 5, 4, 3, 2 or 1 amino acid(s) truncated or extended).

The ligand-binding molecules may comprise two or more amino acid sequences derived from the extracellular region of VEGFR3 (preferably human VEGFR3). In some embodiments, the amino acid sequence derived from the extracellular region of VEGFR3 is a fragment of the extracellular region of human VEGFR3. In the case of comprising two or more amino acid sequences derived from the extracellular region of VEGFR3, the amino acid sequences can be linked to each other directly or via a spacer, alternatively, in the case where the ligand-binding molecule is a double-stranded dimer, they can be on two separate chains.

In some embodiments, the ligand-binding molecules described herein comprise the amino acid sequence as set forth at positions 25-210 of SEQ ID NO: 3, the amino acid sequence as set forth at positions 25-314 of SEQ ID NO: 3, or amino acid sequences having at least 80%, or at least 85%, or at least 90%, or at least 92%, or at least 95%, or at least 96%, or at least 97%, or at least 98%, or at least 99% identity with the above sequences.

In some embodiments, the amino acid sequences with identity are N-terminal or C-terminal truncated fragments of the amino acid sequence as set forth at positions 25 -210 of SEQ ID NO: 3 or the amino acid sequence as set forth at positions 25-314 of SEQ ID NO: 3. For example, the truncation is no more than 40 amino acids, such as no more than 30 amino acids, no more than 20 amino acids, no more than 15 amino acids, no more than 10 amino acids, no more than 8 amino acids, no more than 6 amino acids, no more than 5 amino acids, no more than 4 amino acids, no more than 3 amino acids, no more than 2 amino acids or no more than 1 amino acid. In some specific embodiments, the ligand-binding molecules described herein comprise the amino acid sequence as set forth at positions 47-210 of SEQ ID NO: 3.

In some embodiments, the amino acid sequences with identity comprise one or more amino acid modifications, such as amino acid substitutions, insertions or deletions, as compared to the amino acid sequence as set forth at positions 25-210 of SEQ ID NO: 3 or the amino acid sequence as set forth at positions 25-314 of SEQ ID NO: 3.

As known in the art, the spacer can be adopted in various forms. In some embodiments, the spacer is a peptide sequence with a length between 1-100 amino acids, preferably of 1-50 amino acids. In some embodiments, the spacer is derived from linking sequences at corresponding positions in native VEGFR-2 or VEGFR-3 (i.e., sequences flanking the fragment in the sequence from which the fragment is derived). In some embodiments, the spacer comprises an amino acid sequence that is at least 80%, or at least 85%, or at least 90%, or at least 92%, or at least 95%, or at least 96%, or at least 97%, or at least 98%, or at least 99% identical to the native peptide sequence or portion thereof flanking the fragments in native human VEGFR-2 or human VEGFR-3.

Alternatively, the spacer may or may not be used, and may be a linker sequence commonly used in the art, such as(G4S)n, AAA or other short peptide sequences. As will be appreciated by those skilled in the art, the spacer may have a wider range of amino acid sequences.

In some specific embodiments, an amino acid sequence derived from human VEGFR3 is linked to an amino acid sequence derived from human VEGFR2 via a spacer, wherein the amino acid sequence derived from human VEGFR3 is the amino acid sequence defined by positions 25-210 of SEQ ID NO: 3, the amino acid sequence derived from human VEGFR2 is the amino acid sequence defined by positions 219-327 of SEQ ID NO: 2, and the spacer comprises an amino acid sequence having at least 80%, or at least 85%, or at least 90%, or at least 92%, or at least 95%, or at least 96%, or at least 97%, or at least 98%, or at least 99% identity to the amino acid sequence defined by positions 211-231 of SEQ ID NO: 3 or positions 211-220 of SEQ ID NO: 2. The amino acid sequence derived from human VEGFR3 may be at the N-terminal or the C-terminal of the amino acid sequence derived from human VEGFR2.

In some embodiments, the ligand-binding molecules of the present disclosure comprise one or more amino acid sequences derived from VEGFR2 and one or more amino acid sequences derived from VEGFR1 (for example, human VEGFR1, in particular the extracellular region of VEGFR1). Specifically, the amino acid sequence therein derived from VEGFR2 is operably linked to the amino acid sequence derived from VEGFR1. The operable linkage may be a direct linkage to each other or a linkage via a spacer.

In some embodiments, the amino acid sequence derived from VEGFR1 is a fragment of VEGFR1 or an amino acid sequence having at least 80% identity thereto. Specifically, the fragment of VEGFR1 can be selected from the following: the amino acid sequence defined by positions 132-230 of SEQ ID NO: 1 and its N-terminal and/or C-terminal truncated and/or extended fragments (for example, with no more than 40, 30, 20, 15, 10, 9, 8, 7, 6, 5, 4, 3, 2 or 1 amino acid(s) truncated or extended).

In some embodiments, the amino acid sequences with identity contain one or more amino acid modifications, such as amino acid substitutions, insertions or deletions, as compared to the amino acid sequence as set forth at positions 132-230 of SEQ ID NO: 1.

In some aspects, the ligand-binding molecules comprise one or more amino acid sequences derived from VEGFR2 and one or more amino acid sequences derived from VEGFR1. Specifically, the amino acid sequence derived from VEGFR2 is operably linked to the amino acid sequence derived from VEGFR1. The operable linkage may be a direct linkage to each other or a linkage via a spacer. In some embodiments, the amino acid sequence derived from VEGFR1 is a fragment of VEGFR1 or an amino acid sequence having at least 80% identity thereto. In some embodiments, the fragment of VEGFR1 is selected from the amino acid sequence as set forth at positions 132-230 of SEQ ID NO: 1, or its N-terminal and/or C-terminal truncated and/or extended fragments.

### III. Ligand-binding molecules comprising an Fc region and optionally a signal peptide

The ligand-binding molecules of the present invention can be a monomer (i.e., a single chain), a dimer (i.e., double chains) or a multimer. In a dimeric or multimeric structure, each chain of the ligand-binding molecules is covalently or non-covalently associated to each other. In some embodiments, a linkage occurs between VEGFR-2-derived sequences of the ligand-binding molecules.

In some embodiments, the ligand-binding molecules provided herein further comprise an immunoglobulin constant region, such as a constant region of human IgG, and more specifically, may comprise a hinge region and an Fc region, such as an Fc region sequence of IgG1, IgG2, IgG3 and IgG4. The immunoglobulin constant region can be linked to the sequence derived from VEGFR1 and/or VEGFR2 and/or VEGFR3 through a linker. As known in the art, Fc region refers to a portion of an antibody consisting of a second and a third constant domains of a first heavy chain of the antibody bound to a second and a third constant domains of a second heavy chain of the antibody via disulfide bonds, and optionally the Fc region further comprises all or part of a hinge region. The Fc region herein includes a wild-type Fc region and variants thereof, with different mutations used for multiple purposes. The variant may contain one or more amino acid residue modifications, such as substitutions, in the Fc region.

In certain embodiments, the Fc region variant contains one or more amino acid substitutions that improve the pH-dependent binding to the neonatal Fc receptor (FcRn). Such a variant can have an extended pharmacokinetic half-life, as it binds to FcRn at an acidic pH which allows it to escape from degradation in the lysosome and then be translocated and released out of the cell. Methods of engineering an antibody molecule to improve the binding affinity to FcRn are well-known in the art, see, for example, Vaughn, D. et al., Structure, 6(1): 63-73, 1998; Kontermann, R. et al., Antibody Engineering, Volume 1, Chapter 27: Engineering of the Fc region for improved PK, Springer, 2010; Yeung, Y. et al., Cancer Research, 70: 3269-3277 (2010); and Hinton, P. et al., J. Immunology, 176: 346-356 (2006).

In certain embodiments, the Fc region variant contains one or more amino acid substitutions that alter the antibody-dependent cellular cytotoxicity (ADCC), or alter the complement dependent cytotoxicity (CDC) e.g. by improving or diminishing C1q binding.

In certain embodiments, the ligand-binding molecules provided herein comprise a constant region of human IgG4, in which amino acid residue 228 is altered, e.g., Ser228Pro (S228P, which may prevent or reduce chain exchange), and/or amino acid residue 235 is altered, e.g., Leu235Glu (L235E, which may alter Fc receptor interactions).

In certain embodiments, the ligand-binding molecules provided herein comprise one or more amino acid substitution(s) in the interface of the Fc region to facilitate and/or promote heterodimerization. These modifications comprise introduction of a protuberance/knob into a first Fc polypeptide and a cavity/hole into a second Fc polypeptide, wherein the protuberance can be positioned in the cavity so as to promote interaction of the first and second Fc polypeptides to form a heterodimer or a complex. Methods of generating protein molecules with these modifications are known in the art, e.g., as described in U.S. Pat. No. 5,731,168.

In certain embodiments, the ligand-binding molecules provided herein comprise a native Fc sequence of human IgG1, as set forth in Asp104-Lys330 of SEQ ID NO: 31. In some embodiments, the ligand-binding molecules provided herein comprise an amino acid sequence having at least 80%, or at least 85%, or at least 90%, or at least 92%, or at least 95%, or at least 96%, or at least 97%, or at least 98%, or at least 99% identity or having 100% identity to the amino acid sequence at positions 104-330 of SEQ ID NO: 31.

In some embodiments, the ligand-binding molecules further comprise a signal peptide at the amino terminal to facilitate secretion and purification from cells.

### IV. Properties of ligand-binding molecules

In the present disclosure, ligand-binding molecules comprising one or more amino acid sequences derived from VEGFR2 (and/or amino acid sequences derived from other receptors) have been found to have binding and blocking abilities towards VEGF-A, VEGF-C, and VEGF-D, especially towards VEGF-C and VEGF-D. In some embodiments, the ligand-binding molecules have significantly improved binding or blocking abilities towards VEGF-A, VEGF-C, and VEGF-D, of which the binding or blocking ability towards VEGF-A is superior to that of Bevacizumab (Avastin), close to or superior to that of Aflibercept (Eylea), and the binding or blocking abilities towards both VEGF-C and VEGF-D are significantly superior to those of OPT as the control. The ligand-binding molecules can effectively inhibit neovascularization by blocking VEGF-A, VEGF-C, VEGF-D, and PDGF family molecules, and thus play a role in the treatment of a variety of diseases, especially ophthalmic diseases or oncological diseases.

In the present invention, the ligand-binding molecules have been found to block the binding of VEGF-A, VEGF-C, and VEGF-D to their receptors, and also bind to PDGF molecules. As verified in the Examples:
1) The K_{D} of LFV-B binding to VEGF-C was 5.6 times stronger than that of the control article OPT, and the K_{D} of LFV-E was slightly superior to that of OPT; in VEGF-C blocking experiments, the EC₅₀ of LFV-B was 1.4-2.2 times stronger than that of OPT (blocking experiments on the binding of VEGFR2 and VEGFR3 to VEGF-C, respectively), the EC₅₀ of LFV-C was 1.3-2.4 times stronger than that of OPT, and the EC₅₀ of LFV-E was 1.57-2.2 times stronger than that of OPT, whereas Avastin and Eylea, as controls, could not block it, as determined by Biacore.
2) The K_{D} of LFV-B binding to VEGF-D was 7.8 times stronger than that of OPT, and the K_{D} of LFV-E was slightly superior to that of OPT; in VEGF-D blocking experiments, the EC₅₀ of LFV-B was 4.7-7.4 times stronger than that of OPT (blocking experiments on the binding of VEGFR2 and VEGFR3 to VEGF-D, respectively), the EC₅₀ of LFV-C was 4.6-5.9 times stronger than that of OPT, and the EC₅₀ of LFV-E was 4.8-3.8 times stronger than that of OPT, whereas Avastin and Eylea, as controls, could not block it, as determined by Biacore.
3) The K_{D} of LFV-B binding to VEGF-A was 9.3 pM, which was close to or similar to that of Eylea; in VEGF-A165-VEGFR2 blocking ELISA experiments, the EC₅₀ of LFV-B was 4.67 nM, which was between those of Avastin (6.68 nM) and Eylea (3.2 nM), and the EC₅₀ of LFV-C was 4.57 nM, which was close to that of LFV-B; in VEGF-A121-VEGFR2 blocking ELISA experiments, the EC₅₀ of LFV-B was 7.63 nM, which was superior to those of Avastin (22.37 nM) and Eylea (9.55 nM), and LFV-C had an EC₅₀ of 9.35 nM, which was close to that of Eylea and superior to that of Avastin, as determined by Biacore.
4) In murine *in vivo* ophthalmic experiments of AMD, LFV-B and LFV-C also showed a better improvement trend compared to the control drug Eylea.

Furthermore, in the present invention, novel ligand-binding molecules (for example, LFV-M, LFV-N, LFV-Q, LFV-R and LFV-S) comprising one or more amino acid sequences derived from VEGFR2 operably linked to one or more amino acid sequences derived from VEGFR1 have been discovered. In some embodiments, the ligand-binding molecules have a further improved binding or blocking ability towards VEGF-A, while nicely retaining binding or blocking abilities towards VEGF-C and VEGF-D, and having binding abilities to VEGF-B and PIGF. As verified in the Examples:
1) In VEGF-A165-VEGFR2 blocking ELISA experiments, the EC₅₀ of LFV-M and LFV-N were 1.60 and 1.87 nM, respectively, which were superior to that of the original molecule LFV-C (3.18 nM) unfused to VEGFR1, and also superior to those of the control drugs Eylea (2.23 nM) and Avastin (4.58 nM); in VEGF-A165-VEGFR1 blocking ELISA experiments, the EC₅₀ of LFV-M and LFV-N were 3.14 and 3.30 nM, respectively, which were superior to those of Eylea (4.64 nM) and Avastin (7.31 nM).
2) In VEGF-C-VEGFR2 blocking ELISA experiments, the EC₅₀ of LFV-M and LFV-N were 27.6 and 27.3 nM, respectively, which were close to that of LFV-C (26.2 nM) and slightly superior to that of the control article OPT (32.1 nM).
3) In VEGF-D-VEGFR2 blocking ELISA experiments, the EC₅₀ of LFV-M and LFV-N were 2.17 and 1.79 nM, respectively, which were close to that of LFV-C (2.30 nM) and significantly superior to that of the control article OPT (24.59 nM); in VEGF-D-VEGFR3 blocking ELISA experiments, the EC₅₀ of LFV-M and LFV-N were 2.74 and 2.31 nM, respectively, which were close to that of LFV-C (2.62 nM) and also significantly superior to that of the control article OPT (25.22 nM).
4) Additionally, in ELISA experiments for binding to VEGF-B and PIGF, LFV-M and LFV-N also showed better binding abilities, whereas LFV-C showed extremely weak binding.

Further, it has been found in the invention that after N-glycosylation removal from LFV-M, the resulting molecule LFV-R has an even further optimized blocking potency against VEGF family molecules, and a binding potency to VEGF-B and PIGF molecules, as verified in the Examples:
(1) In ELISA experiments for blocking the VEGF A165-VEGFR2 binding, the EC₅₀ of LFV-R was 1.901 nM, which was superior to that of LFV-M (2.645 nM), and also superior to that of the control drug Eylea (3.937 nM); in ELISA experiments for blocking the VEGF A165-VEGFR1 binding, the EC₅₀ of LFV-R was 6.972 nM, which was superior to that of LFV-M (7.797 nM), and close to that of the control drug Eylea (6.959 nM).
(2) In ELISA experiments for blocking the VEGF D-VEGFR2 binding, LFV-R had a EC₅₀ of 4.933 nM, superior to 5.623 nM of LFV-M.
(3) In binding experiments to VEGF-B, the EC₅₀ of LFV-R was 125.6 ng/ml, which was superior to that of LFV-M at 181.7 ng/ml.
(4) In binding experiments to PIGF, the EC₅₀ of LFV-R was 32.01 ng/ml, which was superior to that of LFV-M at 43.85 ng/ml.

### V. Polynucleotides, vectors and host cells

In some aspects, the invention also provides polynucleotides encoding such ligand-binding molecules. The polynucleotides can be used to express the ligand-binding molecules. In some embodiments, the polynucleotides can also be used as a therapeutic agent in an active form for achieving in vivo expression of a polypeptide ligand-binding molecule.

In certain embodiments, an isolated polynucleotide comprises a nucleotide sequence encoding at least one of the following amino acid sequences and/or a nucleotide sequence having at least 80% (for example, at least 85%, 88%, 90%, 92%, 93%, 94%, 95%, 96%, 97%, 98% or 99%) sequence identity thereto, and/or a variant thereof with only degeneracy substitutions:
(a) Amino acid sequence as set forth at positions 117-218 of SEQ ID NO: 2;
(b) Amino acid sequence as set forth at positions 117-327 or 123-327 of SEQ ID NO: 2;
(c) Amino acid sequence as set forth at positions 117-421 of SEQ ID NO: 2;
(d) Amino acid sequence as set forth at positions 23-327 of SEQ ID NO: 2; and
(e) Amino acid sequence as set forth at positions 23-421 of SEQ ID NO: 2.

Optionally, the polynucleotide further comprises a nucleotide sequence encoding at least one of the following amino acid sequences and/or a nucleotide sequence having at least 80% (for example, at least 85%, 88%, 90%, 92%, 93%, 94%, 95%, 96%, 97%, 98% or 99%) sequence identity thereto, and/or a variant thereof with only degeneracy substitutions:
(i) Amino acid sequence as set forth at positions 25-115 or 47-115 of SEQ ID NO: 3;
(ii) Amino acid sequence as set forth at positions 154-210 of SEQ ID NO: 3;
(iii) Amino acid sequence as set forth at positions 248-314 of SEQ ID NO: 3; and
(iv) Amino acid sequence as set forth at positions 132-230 of SEQ ID NO: 1.

The polynucleotide encoding the ligand-binding molecule is readily isolated and sequenced using conventional procedures known in the art. The polynucleotide can also be obtained by a synthetic method. Preferably, the polynucleotide is codon optimized for expression in eukaryotic host cells, in particular mammalian cells.

Polynucleotides encoding ligand-binding molecules can be inserted into vectors for further cloning (amplification of DNA) or expression using known recombinant techniques. Vector components typically include, but are not limited to, one or more of the following: a signal sequence, a replication origin, one or more marker genes, an enhancer element, a promoter (e.g., SV40, CMV, EF-1α) and a transcription termination sequence.

In some embodiments, the present disclosure provides a vector (e.g., an expression vector) comprising a polynucleotide encoding a ligand-binding molecule provided herein, at least one promoter (e.g., SV40, CMV, EF-1α) operably linked to the polynucleotide, and at least one selection marker. Examples of the vector include, but are not limited to, retroviruses (including lentiviruses), adenoviruses, adeno-associated viruses, herpesviruses (e.g., herpes simplex virus), poxviruses, baculoviruses, papillomaviruses, papovaviruses (e.g., SV40), λ phages and M13 phages, liposomes, plasmids pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM, pCDM8, pCDNA1.1 /amp, pcDNA3.1, pRc/RSV, PCR 2.1, pEF-1, pFB, pSG5, pXT1, pCDEF3, pSVSPORT, pEF-Bos, etc.

A vector comprising a polynucleotide sequence encoding a ligand-binding molecule can be introduced into a host cell for cloning or gene expression. Suitable host cells for cloning or expressing DNA in vectors herein are prokaryote, yeast, or higher eukaryote cells. Suitable prokaryotes for this purpose include eubacteria, such as Gram-negative or Gram-positive bacteria, for example, Escherichia coli. In addition to prokaryotes, eukaryotic microbes such as filamentous fungi or yeasts are suitable cloning or expression hosts for the provided vectors. Saccharomyces cerevisiae, or common baker's yeast, is the most commonly used among lower eukaryotic host microorganisms. However, a number of other genera, species, and strains are commonly available and useful herein.

Suitable host cells for the expression of ligand-binding molecules provided herein can also be derived from multicellular organisms, such as plant cells and invertebrate cells including insect cells. Numerous baculoviral strains and variants and corresponding permissive insect host cells have been identified. However, interest has been greatest in vertebrate cells, and propagation of vertebrate cells in culture (tissue culture) has become a routine procedure. Examples of useful mammalian host cell lines include monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TR1 cells (Mather et al., Annals N.Y. Acad. Sci. 383:44-68 (1982)); MRC 5 cells; FS4 cells; and a human hepatoma line (HepG2). In some preferred embodiments, the host cell is a Chinese hamster ovary (CHO) cell. In some other preferred embodiments, the host cells are other mammalian cell lines, such as human cell lines.

Host cells are transformed with the above-described expression or cloning vectors for producing ligand-binding molecules and cultured in conventional nutrient media modified as appropriate for inducing promoters, selecting transformants, or amplifying the genes encoding the desired sequences.

Host cells used to produce ligand-binding molecules provided herein may be cultured in a variety of mediums. Commercially available mediums such as Ham's F10 (Sigma), Minimal Essential Medium (MEM, Sigma), RPMI-1640 (Sigma), and Dulbecco's Modified Eagle's Medium (DMEM, Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the ligand-binding molecules can be produced intracellularly, in the periplasmic space, or directly secreted into the medium. If the ligand-binding molecules are produced intracellularly, as a first step, the particulate debris from host cells or lysed fragments is removed, for example, by centrifugation or ultrafiltration. Cell debris can be removed by centrifugation. Where the ligand-binding molecules are secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The ligand-binding molecules prepared from the cells can be purified using, for example, hydroxylapatite chromatography, gel electrophoresis, dialysis, DEAE-cellulose ion exchange chromatography, ammonium sulfate precipitation, salting out, and affinity chromatography, with affinity chromatography being the preferred purification technique.

In certain embodiments, protein A immobilized on a solid phase is used for immunoaffinity purification of the ligand-binding molecules. The suitability of protein A as an affinity ligand depends on the species and isotype of any immunoglobulin Fc domain that is present in the ligand-binding molecules. Protein A can be used to purify antibodies that are based on human γ1, γ2, or γ4 heavy chains (Lindmark et al., J. Immunol. Meth. 62: 1-13 (1983)). The matrix to which the affinity ligand is attached is most often agarose, but other matrices may also be used. Mechanically stable matrices (such as controlled pore glass or poly(styrenedivinyl) benzene) allow for faster flow rates and shorter processing times than those can be achieved with agarose. Other techniques for protein purification such as fractionation on an ion-exchange column, ethanol precipitation, reverse phase HPLC, chromatography on silica gel, chromatography on heparin SEPHAROSE^{™}, chromatography on an anion or cation exchange resin (such as a polyaspartic acid column), chromatofocusing, SDS-PAGE, and ammonium sulfate precipitation are also available depending on the antibody to be recovered.

### VI. Conjugates

In some aspects, the invention also provides a conjugate comprising a ligand-binding molecule(s) and a moiety conjugated thereto. The conjugate moiety is one that can be attached to a ligand-binding molecule. It is contemplated that a variety of conjugate moieties may be linked to the ligand-binding molecules provided herein (see, for example, >Conjugate Vaccines", Contributions to Microbiology and Immunology, J. M. Cruse and R. E. Lewis, Jr. (eds.), Carger Press, New York, (1989)). These conjugate moieties may be linked to the ligand-binding molecules by covalent binding, affinity binding, intercalation, coordinate binding, complexation, association, blending, or addition, among other methods.

In certain embodiments, the ligand-binding molecules disclosed herein can be engineered to contain specific sites that may be utilized for binding to one or more conjugate moieties. For example, such sites may comprise one or more reactive amino acid residues, such as cysteine residues and histidine residues, to facilitate covalent linkage to the conjugate moiety.

In certain embodiments, the conjugate comprises a linker linking the conjugate moiety to the ligand-binding molecule. In some other embodiments, the ligand-binding molecules described herein are directly attached to an N-terminal amino acid of the conjugate moiety through a peptide bond at its C-terminal, or directly attached to a C-terminal amino acid of the conjugate moiety through a peptide bond at its N-terminal. It can also be linked to the conjugate moiety through a chemical bond, including but not limited to an amide bond, to form a polypeptide chain. In certain embodiments, the ligand-binding molecules can be linked to a first conjugate moiety either indirectly or through a second conjugate moiety. For example, the ligand-binding molecules can be conjugated to biotin, and then indirectly conjugated to a second conjugate moiety that is conjugated to avidin. The conjugate moiety may be a clearance modifying module, a toxin (e.g., a chemotherapeutic agent), a detectable label (e.g., a radioactive isotope, a lanthanide element, a luminescent label, a fluorescent label, or an enzyme-substrate label), or a purification moiety.

A "toxin" can be any agent that is harmful to cells or that can damage or kill cells. Examples of toxins include, but are not limited to, paclitaxel, cytochalasin B, gramicidin D, ethidium bromide, emetine, mitomycin, etoposide, teniposide, vincristine, MMAE, MMAF, DM1, vinblastine, colchicine, doxorubicin, daunorubicin, dihydroxyanthracin dione, mitoxantrone, mithramycin, actinomycin D, 1-dehydrotestosterone, glucocorticoids, procaine, tetracaine, lidocaine, propranolol, puromycin and its analogues, antimetabolites (e.g., methotrexate, 6-mercaptopurine, 6-thioguanine, cytarabine, 5-fluorouracil, dacarbazine), alkylating agents (e.g., mechlorethamine, thiotepa, chlorambucil, melphalan, carmustine (BSNU) and lomustine (CCNU), cyclophosphamide, busulfan, dibromomannitol, streptozotocin, mitomycin C and cis-dichlorodiammine platinum(II) (DDP) cisplatin), anthracyclines (e.g., daunorubicin (formerly known as daunomycin) and adriamycin), antibiotics (e.g., actinomycin (formerly known as dactinomycin), bleomycin, mithramycin and anthramycin (AMC)), anti-mitotic agents (e.g., vincristine and vinblastine), topoisomerase inhibitors and tubulin binding agents.

Examples of detectable labels can include fluorescent labels (e.g., luciferin, rhodamine, dansyl, phycoerythrin, or Texas red), enzyme-substrate labels (e.g., horseradish peroxidase, alkaline phosphatase, luciferase, glucoamylase, lysozyme, sugar oxidase or β-D-galactosidase), radioactive isotopes (e.g., ¹²³I, ¹²⁴I, ¹²⁵I, ¹³¹I, ³⁵S, ³H, ¹¹¹In, ¹¹²In, ¹⁴C, ⁶⁴Cu, ⁶⁷Cu, ⁸⁶Y, ⁸⁸Y, ⁹⁰Y, ¹⁷⁷Lu, ²¹¹At, ¹⁸⁶Re, ¹⁸⁸Re, ¹⁵³Sm, ²¹²Bi and ³²P, other lanthanide elements), luminescent labels, chromophore moieties, digoxigenin, biotin/avidin, DNA molecules or gold for detection.

In certain embodiments, the conjugate moiety can be a clearance modifier, which helps increase the half-life of the ligand-binding molecule. Illustrative examples include water-soluble polymers such as PEG, carboxymethylcellulose, dextran, polyvinyl alcohol, polyvinylpyrrolidone, copolymers of ethylene glycol/propylene glycol, and the like. The polymer can be of any molecular weight and can be branched or unbranched. The number of polymers attached to the ligand-binding molecules can vary, and if more than one polymer is attached, they can be the same or different molecules. In some specific embodiments, the ligand-binding molecule provided herein is conjugated to at least one polyethylene glycol moiety, in which the polyethylene glycol moiety can be attached to the amino terminal of the ligand-binding molecule.

In certain embodiments, the conjugate moiety can be a purification moiety, such as a magnetic bead.

### VII. Pharmaceutical compositions

In some aspects, the present invention provides a composition, such as a pharmaceutical composition, comprising the ligand-binding molecule(s) of the invention formulated together with a pharmaceutically acceptable carrier. Such compositions comprise at least one ligand-binding molecule, fusion protein or conjugate of the invention. In some embodiments, the pharmaceutical composition is a formulation suitable for intravitreal injection.

As used herein, "pharmaceutically acceptable carriers" include any and all physiologically compatible and pharmaceutically acceptable liquid, gel or solid carriers, aqueous vehicles, non-aqueous vehicles, antimicrobial agents, isotonic agents, buffers, antioxidants, anesthetics, suspending/dispersing agents, sequestering or chelating agents, diluents, adjuvants, excipients or non-toxic auxiliary substances, other components known in the art or various combinations thereof, etc. The carrier can be suitable for intravenous, intramuscular, subcutaneous, parenteral, spinal, transepidermal, intravitreal injection or implant administration, and the like. Depending on the route of administration, the active compound, i.e., the ingredient of the present invention, may be coated in a material to protect the compound from the action of acids and other natural conditions that may inactivate the compound.

The pharmaceutical composition of the present invention may further comprise a pharmaceutically acceptable antioxidant. Examples of pharmaceutically acceptable antioxidants include: (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, α-tocopherol and the like; and (3) metal-chelating agents, such as citric acid, ethylenediaminetetraacetic acid (EDTA), sorbitol, tartaric acid, phosphoric acid and the like.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials (such as lecithin), by the maintenance of the required particle size in the case of dispersing agents, and by the use of surfactants.

These compositions may also contain auxiliary agents such as preserving agents, wetting agents, emulsifying agents and dispersing agents. Prevention of the presence of microorganisms can be ensured both by sterilization methods and by the inclusion of various antibacterial and antifungal agents, such as parabens, chlorobutanol, phenol, sorbic acid, and the like. It may also be desirable to include isotonic agents in the composition, such as sugars, sodium chloride, and the like. In addition, longer absorption of the injectable pharmaceutical form can be brought about by the inclusion of agents that delay absorption, such as aluminum monostearate and gelatin.

The pharmaceutical composition can be in the form of solid, paste, ointment, gel, liquid, aerosol, spray, polymer, film, emulsion or suspension.

In certain embodiments, the pharmaceutical composition is formulated into an injectable composition. The injectable pharmaceutical compositions may be prepared in any conventional form, such as liquid solution, suspension, emulsion, or solid forms suitable for generating a liquid solution, suspension, or emulsion. Preparations for injection may include sterile and/or non-pyretic solutions ready for injection, sterile dry soluble products such as lyophilized powders, ready to be mixed with a solvent just prior to use, including hypodermic tablets, sterile suspensions ready for injection, sterile dry insoluble products ready to be combined with a vehicle before use, and sterile and/or non-pyretic emulsions. The solutions may be either aqueous or non-aqueous.

Pharmaceutically acceptable carriers include sterile aqueous solutions or dispersions and sterile powders for the extemporaneous preparation of sterile injectable solutions or dispersions. The use of such media and agents for pharmaceutically active substances is known in the art. Unless any conventional medium or agent is incompatible with the active compound, use thereof in the pharmaceutical compositions of the invention is contemplated. Supplementary active compounds can also be incorporated into the composition.

Pharmaceutical compositions generally must be sterile and stable under the conditions of manufacture and storage. The composition may be formulated as a solution, microemulsion, liposome, or other ordered structures suitable for a high drug concentration. The carrier may be a solvent or a dispersing medium containing, for example, water, ethanol, polyols (for example, glycerol, propylene glycol, liquid polyethylene glycol, and the like), and suitable mixtures thereof. Proper fluidity can be maintained, for example, by the use of coatings (such as lecithin), by the maintenance of the required particle size in the case of dispersing agents, and by the use of surfactants. In many cases, it is preferable to include isotonic agents in the composition, for example, sugars, polyols (such as mannitol, sorbitol) or sodium chloride. Prolonged absorption of an injectable composition can be brought about by the inclusion of agents that delay absorption (such as monostearate and gelatin) in the composition.

Sterile injectable solutions can be prepared by incorporating the active compound in the required amount into an appropriate solvent together with one or a combination of the components listed above, as required, followed by sterile microfiltration of the resulting mixture. Typically, dispersions can be prepared by incorporating the active compound into a sterile carrier that contains a basic dispersing medium and other desired components from the substances listed above. In the case of sterile powders for the preparation of sterile injectable solutions, the preferred preparation methods are vacuum drying and freeze drying (lyophilization), which lead to yield a powder of the active ingredient plus any additional required ingredient from a previously sterile-filtered solution.

The amount of the active ingredient that can be combined with a carrier material to prepare a single dosage form varies depending on the subject to be treated and the particular mode of administration. The amount of the active ingredient that can be combined with a carrier to prepare a single dosage form is generally an amount of the composition that produces a therapeutic effect. Typically, on a 100% basis, this amount ranges from about 0.01% to about 99% of the active ingredient, preferably about 0.1% to about 70%, and most preferably about 1% to about 30% of the active ingredient, in combination with a pharmaceutically acceptable carrier.

The dosing regimen may be adjusted to provide the optimal desired response (e.g., therapeutic response). For example, a single bolus can be administered, several divided doses can be administered over time or the dose can be proportionally reduced or increased as indicated by the exigencies of the therapeutic situation.

It is particularly advantageous to formulate the pharmaceutical composition into a unit dosage form for ease of administration and uniformity of dosage. A unit dosage form as used herein refers to a physically discrete unit suited as a unitary dosage for the subject to be treated; each unit contains a predetermined quantity of active compound suitable for producing a desired therapeutic effect in combination with the desired pharmaceutical carrier. The specification for the unit dosage form of the present invention is dictated by and directly dependent on the following factors: (a) the unique characteristics of the active compound and the particular therapeutic effect to be achieved, and (b) sensitivity limitations inherent in the field of formulating such active compounds for the treatment of individuals.

For administration of the ligand-binding molecules, the dosage ranges from about 0.0001 to 100 mg/kg, and more usually from 0.01 to 5 mg/kg, based on the host body weight. For example, the dosage may be 0.3 mg/kg body weight, 1 mg/kg body weight, 3 mg/kg body weight, 5 mg/kg body weight or 10 mg/kg body weight, or it may be in the range of 1-10 mg/kg. Exemplary treatment regimens include administration once a week, once every two weeks, once every three weeks, once every four weeks, once a month, once every three months or once every three to six months.

Optionally, the ligand-binding molecules can be administered as a sustained release formulation, in which case a less frequent administration is required. Dosage and frequency vary depending on the half-life of the administered substance in the patient. The dosage and frequency of administration may vary, depending on whether the treatment is prophylactic or therapeutic. In a prophylactic application, a relatively low dosage is administered at relatively infrequent intervals over a long period of time. Some patients continue to receive treatment for the rest of their lives. In a therapeutic application, a relatively high dosage is sometimes required at relatively short time intervals until the progression of the disease has been slowed down or terminated, and preferably until the patient has shown partial or complete alleviation in disease symptoms. Afterwards, the patient can be given a prophylactic regimen.

The actual dosage levels of active ingredients and small molecules in the pharmaceutical composition of the present invention can be varied in order to obtain an amount of the active ingredient that is effective to achieve a desired therapeutic response for a particular patient, composition and mode of administration without being toxic to the patient. The selected dosage level depends on various pharmacokinetic factors, including the activity of the specific combination of the invention used, or esters, salts or amides thereof, route of administration, time of administration, excretion rate of the specific compound used, duration of treatment, other drugs, compounds and/or materials used in combination with the specific combination used, age, gender, weight, condition, general health and past medical history of the patient to be treated, as well as similar factors well known in the medical field.

### VIII. Methods of using the ligand-binding molecules

In some aspects, the present invention provides a method for inhibiting, preventing or treating neovascularization and diseases or conditions caused by neovascularization in a subject, comprising administering the ligand-binding molecules, conjugates or pharmaceutical compositions thereof as described herein to the subject. The neovascularization includes retinal neovascularization and/or choroidal neovascularization. The diseases or conditions include polypoidal choroidal vasculopathy, retinal neovascular formation and ocular disorders associated with fundus leakage.

In some embodiments, the ocular disorder is selected from the group consisting of senile macular degeneration, diabetic ocular disorder, polypoidal choroidal vasculopathy, fundus fibrotic disorder, retinal vein occlusion-related disease, retinopathy of prematurity and macular telangiectasia. Preferably, the pharmaceutical composition comprising a ligand-binding molecule described herein is administered topically to the eye of a subject by eye drops, by intravitreal injection, or by intravitreal implant.

In some embodiments, the ligand-binding molecules, conjugates or pharmaceutical compositions thereof disclosed herein can be administered in single dose or multiple doses. Preferably, the desired therapeutic effect can be achieved by a single administration without being administered together with other targeted therapeutic drugs.

In some embodiments, the ligand-binding molecules, conjugates or pharmaceutical compositions thereof disclosed herein can be administered alone or in combination with one or more additional therapeutic means or agents. For example, the ligand-binding molecules, conjugates or pharmaceutical compositions thereof disclosed herein can be implemented in combination with additional therapeutic agents, such as chemotherapeutic agents, or other therapeutic methods. For example, in the therapeutic implementation for ocular diseases, other therapeutic methods include laser photocoagulation, vitrectomy, etc.

In some of these embodiments, the ligand-binding molecule, conjugate or pharmaceutical composition thereof disclosed herein that is administered in combination with one or more additional therapeutic agents may be administered simultaneously or sequentially or alternately with the one or more additional therapeutic agents. The ligand-binding molecule(s) administered "in combination" with another therapeutic agent does not have to be administered simultaneously with or in the same composition as the agent. A ligand-binding molecule administered prior to or after another agent is considered to be administered "in combination" with that agent as the phrase is used herein, even in the case where the ligand-binding molecule and the second agent are administered via different routes. Where possible, additional therapeutic agents administered in combination with the ligand-binding molecule disclosed herein are administered according to the schedule listed in the product information sheet of the additional therapeutic agent, or according to the Physicians' Desk Reference 2003 (Physicians' Desk Reference, 57th Ed; Medical Economics Company; ISBN: 1563634457; 57th edition (November 2002)) or protocols well known in the art.

In some aspects, the invention also provides a kit comprising the ligand-binding molecule, conjugate or pharmaceutical composition thereof.

### IX. Advantages of the invention

Inhibition of angiogenesis is an effective treatment option for angiogenesis-related diseases such as tumor growth and ophthalmic vascular proliferation. Over the past few decades, extensive research has been conducted on VEGF-related signaling, and there have been many drugs used for treatment. In order to break through limitations of a single targeted therapy, great expectations have been placed on targeting both PDGF-BB and VEGF-A. Inhibition of PDGF-BB can lead to apoptosis of pericytes, thus breaking the drug resistance to anti-VEGF-A treatment, in addition, PDGF-BB is closely related to fibrotic lesions in tissues, and fundus fibrosis, in turn, is a late-developing form of AMD and a cause of blindness, and it is also a major reason for the ultimate failure of VEGF-A therapy (Rofagha S. et al. (2013), J. Ophthalmology. 120(11):2292-2299) (Rosenfeld P. J. et al. (2011), Ophthalmology. 118(3):523-530). More notable among the PDGF-targeting therapies are E10030, a PDGF-BB antagonist introduced by Novartis, and ABBV642, a bispecific antibody developed by AbbVie, the former of which has achieved a more effective efficacy than the use of Ranibizumab alone in Phase I and II clinical trials for AMD, and provided a more pronounced improving effect on fundus fibrosis. Furthermore, although E10030 has not demonstrated a comparative advantage over the single VEGF-A-targeting therapy in larger scale Phase III clinical trials, studies have still been conducted on its therapeutic direction for fibrosis due to the effective intervention effect on fibrosis.

Another product, OPT-302, has been developed by Opthea. OPT-302 targets both VEGF-C and VEGF-D, when used by injection in combination with products targeting VEGF-A, it has clearly demonstrated a significantly better therapeutic effect with a significantly broader response than targeting VEGF-A alone, thus introducing the treatment of diseases such as nAMD into the second generation. Moreover, this therapy has also produced a favorable therapeutic effect in patients with polypoidal choroidal vasculopathy (PCV), which is highly prevalent in Asian populations. As a subtype of wet macular degeneration, PCV somewhat differs from wet macular degeneration in that it does not respond effectively and extensively to the VEGF-A-targeting therapy, and has a high incidence in Asia area (Imamura Y. et al. (2010), Surv. Ophthalmol. 55(6):501-515), with PCV accounting for as high as 33% of patients newly diagnosed as AMD in China (Lande A. et al. (2010), Prog Retin Eye Res. 29(1):19-29) and 54.7% in Japan (Maruko I. et al. (2007), Am J Ophthalmol. 144:15-22), and there currently exists a huge treatment gap for PCV due to a larger population base in Asia area.

However, this therapy is a combined therapy, which requires the simultaneous injection of two agents, i.e., a VEGF-A targeting drug and OPT-302, into the vitreous body, resulting in a higher treatment cost, and at the same time, the therapy also requires monthly injections through the eye, which not only raises the treatment cost, but also leads to inconvenience and poor patient compliance, hence increasing the risk of accidents such as intraocular infection caused by injection. In addition, this therapy needs to target all of VEGF-A, VEGF-C and VEGF-D, with a higher affinity (to the level of 10 pM and below) for all of them, which also hinders the development of alternatives thereto using currently popular monoclonal antibody technologies (an antibody generally binds to only one target site, typically with an affinity of around 100 pM).

The inventors surprisingly found that the ligand-binding molecules comprising a specific amino acid sequence derived from VEGFR2 (e.g., positions 123-327 or 117-327 of SEQ ID NO: 2) have a better binding ability to VEGF-C or VEGF-D than that of OPT. For further study, a variety of VEGF Trap molecules comprising different fragments from VEGFR2 were constructed and expressed, and their binding and blocking abilities towards VEGF-A, VEGF-C, VEGF-D, etc. were studied and characterized in detail.

It was found that the ligand-binding molecules comprising an amino acid sequence derived from VEGFR2, in particular the amino acid sequence as set forth at positions 123-327 or 117-327 of SEQ ID NO: 2, exhibited significantly binding and blocking abilities towards VEGF-C or VEGF-D than those of OPT or the full VEGFR2 extracellular domain, and also had significantly better binding and blocking abilities towards VEGF-A.

Meanwhile, the inventors further unexpectedly characterized that the ligand-binding molecules comprising an amino acid sequence derived from VEGFR2, especially the amino acid sequence as set forth at positions 117-327 or 123-327 of SEQ ID NO: 2, also had a certain binding ability to PDGF family growth factors such as PDGF-AA, AB, BB and CC, which was the main domain involved in binding and was superior to recombinant molecules of the full extracellular domain of native VEGFR2 in terms of the binding to PDGF. Since high expression of PDGF family molecules is believed to be one of the reasons for insufficient response to the VEGF-A-targeting therapy, development of resistance and post-treatment fundus fibrosis, simultaneously targeting the PDGF family may be able to benefit more patients, especially for fundus lesions caused by diabetes, in which PDGF-AA, PDGF-AB, and PDGF-BB have been reported to be highly expressed in such fundus lesions. Consequently, the ligand-binding molecules herein have the potential to treat fundus lesions caused by diabetes.

Additionally, compared with the OPT molecule, the ligand-binding molecules comprising an amino acid sequence derived from VEGFR2, especially the amino acid sequence as set forth at positions 117-327 or 123-327 of SEQ ID NO: 2, exhibits both a significantly better initial monomer purity and a significantly higher final yield during the expression and purification process from HEK293 and CHO cells, which means that they can bring about a lower production cost and medication cost.

Moreover, also provided are novel ligand-binding molecules comprising one or more amino acid sequences derived from VEGFR2 operably linked to one or more amino acid sequences derived from VEGFR1, and glycosylation-modified forms thereof (for example, LFV-M, LFV-N, LFV-Q, LFV-R (LFV-R HPLC-SEC purity is higher than that of LFV-M), LFV-S), which have optimized binding and blocking abilities towards VEGF-A while nicely retaining binding and blocking abilities towards VEGF-C and VEGF-D. In particular, they have been found to have blocking abilities towards VEGFA-VEGFR1, and their blocking abilities towards both VEGFA-VEGFR1 and VEGFA-VEGFR2 are superior to those of the control drugs Eylea and Avastin, in addition, they also show binding abilities to VEGF-B and PlGF.

In this way, VEGF-A, VEGF-C and VEGF-D can be blocked by employing ligand-binding molecules of the present invention, and the molecules can also bind to VEGF-B, PIGF, and PDGF family molecules, which not only helps to meet unmet clinical needs and achieve excellent therapeutic effects, but also solves the high cost problem of concurrently using two or more medications. At the same time, the ligand-binding molecules have a higher affinity and blocking ability for all of VEGF-A, VEGF-C and VEGF-D, for example, LFV-B and LFV-C have stronger affinity to VEGF-C and VEGF-D than that of OPT, and their affinity and blocking ability for VEGF-A are also significantly superior to those of Avastin, and close to or superior to those of Aflibercept, which will help to realize a prolongation of the injection interval and a reduction in the injection frequency, thereby further reducing the treatment cost and addressing multiple disadvantages associated with frequent injections.

The use of the ligand-binding molecules or the pharmaceutical composition comprising the same herein allows for the replacement of a multi-drug combination with a single drug to satisfy vacancies in the therapeutic methods that do not clearly respond to VEGF-A targeting, and to reduce the injection frequency by virtue of high affinity, thus significantly lowering the burden of patients, reducing the inconvenience of treatment, improving compliance and diminishing the risks such as intraocular infections evoked by injections, while obtaining optimal therapeutic effects.

Summary of the sequences of the invention

| SEQ ID NO: 1 | Full-length amino acid sequence of human VEGFR-1 |
|---|---|
| MVSYWDTGVLLCALLSCLLLTGSSSGSKLKDPELSLKGTQHIMQAGQTLHLQCRGEAA HKWSLPEMVSKESERLSITKSACGRNGKQFCSTLTLNTAQANHTGFYSCKYLAVPTSKK KETESAIYIFISDTGRPFVEMYSEIPEIIHMTEGRELVIPCRVTSPNITVTLKKFPLDTLIPD GKRIIWDSRKGFIISNATYKEIGLLTCEATVNGHLYKTNYLTHRQTNTIIDVQISTPRPVKL LRGHTLVLNCTATTPLNTRVQMTWSYPDEKNKRASVRRRIDQSNSHANIFYSVLTIDKM QNKDKGLYTCRVRSGPSFKSVNTSVHIYDKAFITVKHRKQQVLETVAGKRSYRLSMKV KAFPSPEVVWLKDGLPATEKSARYLTRGYSLIIKDVTEEDAGNYTILLSIKQSNVFKNLT ATLIVNVKPQIYEKAVSSFPDPALYPLGSRQILTCTAYGIPQPTIKWFWHPCNHNHSEARC DFCSNNEESFILDADSNMGNRIESITQRMAIIEGKNKMASTLVVADSRISGIYICIASNKV GTVGRNISFYITDVPNGFHVNLEKMPTEGEDLKLSCTVNKFLYRDVTWILLRTVNNRT MHYSISKQKMAITKEHSITLNLTIMNVSLQDSGTYACRARNVYTGEEILQKKEITIRDQE APYLLRNLSDHTVAISSSTTLDCHANGVPEPQITWFKNNHKIQQEPGIILGPGSSTLFIER VTEEDEGVYHCKATNQKGSVESSAYLTVQGTSDKSNLELITLTCTCVAATLFWLLLTLFI RKMKRSSSEIKTDYLSIIMDPDEVPLDEQCERLPYDASKWEFARERLKLGKSLGRGAFG KVVQASAFGIKKSPTCRTVAVKMLKEGATASEYKALMTELKILTHIGHHLNVVNLLGA | |
| CTKQGGPLMVIVEYCKYGNLSNYLKSKRDLFFLNKDAALHMEPKKEKMEPGLEQGKK PRLDSVTSSESFASSGFQEDKSLSDVEEEEDSDGFYKEPITMEDLISYSFQVARGMEFLSS RKCIHRDLAARNILLSENNVVKICDFGLARDIYKNPDYVRKGDTRLPLKWMAPESIFDK IYSTKSDVWSYGVLLWEIFSLGGSPYPGVQMDEDFCSRLREGMRMRAPEYSTPEIYQIM LDCWHRDPKERPRFAELVEKLGDLLQANVQQDGKDYIPINAILTGNSGFTYSTPAFSED FFKESISAPKFNSGSSDDVRYVNAFKFMSLERIKTFEELLPNATSMFDDYQGDSSTLLAS PMLKRFTWTDSKPKASLKIDLRVTSKSKESGLSDVSRPSFCHSSCGHVSEGKRRFTYDH AELERKIACCSPPPDYNSVVLYSTPPI | |

| SEQ ID NO: 2 | Full-length amino acid sequence of human VEGFR-2 |
|---|---|
| | |

| SEQ ID NO: 3 | Full-length amino acid sequence of human VEGFR-3 |
|---|---|
| MQRGAALCLRLWLCLGLLDGLVSGYSMTPPTLNITEESHVIDTGDSLSISCRGQHPLEW AWPGAQEAPATGDKDSEDTGVVRDCEGTDARPYCKVLLLHEVHANDTGSYVCYYKYI | |
| KARIEGTTAASSYVFVRDFEQPFINKPDTLLVNRKDAMWVPCLVSIPGLNVTLRSQSSVL WPDGQEVVWDDRRGMLVSTPLLHDALYLQCETTWGDQDFLSNPFLVHITGNELYDIQL LPRKSLELLVGEKLVLNCTVWAEFNSGVTFDWDYPGKQAERGKWVPERRSQQTHTEL SSILTIHNVSQHDLGSYVCKANNGIQRFRESTEVIVHENPFISVEWLKGPILEATAGDELV KLPVKLAAYPPPEFQWYKDGKALSGRHSPHALVLKEVTEASTGTYTLALWNSAAGLR RNISLELVVNVPPQIHEKEASSPSIYSRHSRQALTCTAYGVPLPLSIQWHWRPWTPCKMF AQRSLRRRQQQDLMPQCRDWRAVTTQDAVNPIESLDTWTEFVEGKNKTVSKLVIQNA NVSAMYKCVVSNKVGQDERLIYFYVTTIPDGFTIESKPSEELLEGQPVLLSCQADSYKY EHLRWYRLNLSTLHDAHGNPLLLDCKNVHLFATPLAASLEEVAPGARHATLSLSIPRVA PEHEGHYVCEVQDRRSHDKHCHKKYLSVQALEAPRLTQNLTDLLVNVSDSLEMQCLV AGAHAPSIVWYKDERLLEEKSGVDLADSNQKLSIQRVREEDAGRYLCSVCNAKGCVN SSASVAVEGSEDKGSMEIVILVGTGVIAVFFWVLLLLIFCNMRRPAHADIKTGYLSIIMDP GEVPLEEQCEYLSYDASQWEFPRERLHLGRVLGYGAFGKVVEASAFGIHKGSSCDTVA VKMLKEGATASEHRALMSELKILIHIGNHLNVVNLLGACTKPQGPLMVIVEFCKYGNL SNFLRAKRDAFSPCAEKSPEQRGRFRAMVELARLDRRRPGSSDRVLFARFSKTEGGAR RASPDQEAEDLWLSPLTMEDLVCYSFQVARGMEFLASRKCIHRDLAARNILLSESDVVK ICDFGLARDIYKDPDYVRKGSARLPLKWMAPESIFDKVYTTQSDVWSFGVLLWEIFSLG ASPYPGVQINEEFCQRLRDGTRMRAPELATPAIRRIMLNCWSGDPKARPAFSELVEILGD LLQGRGLQEEEEVCMAPRSSQSSEEGSFSQVSTMALHIAQADAEDSPPSLQRHSLAARY YNWVSFPGCLARGAETRGSSRMKTFEEFPMTPTTYKGSVDNQTDSGMVLASEEFEQIE SRHRQESGFSCKGPGQNVAVTRAHPDSQGRRRRPERGARGGQVFYNSEYGELSEPSEE DHCSPSARVTFFTDNSY | |

| SEQ ID NO: 4 | LFV-A Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVV | |

| SEQ ID NO: 5 | LFV-B Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEK | |

| SEQ ID NO: 6 | LFV-C Trap polypeptide |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 7 | LFV-D Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEKPFVAFGSGMESLVEATVGERVRIPAK YLGYPPPEIKWYKNGIPLESNHTIKAGHVLTIMEVSERDTGNYTVILTNPISKEKQSHVV SLVVYVPP | |

| SEQ ID NO: 8 | LFV-E Trap polypeptide |
|---|---|
| GLPSVSLDLPRLSIQKDILTIKANTTLQITCRGQRDLDWLWPNNQSGSEQRVEVTECSDG LFCKTLTIPKVIGNDTGAYKCFYRETDLASVIYVYVQDYRSPFIASVSDQHGVVYITENK NKTVVIPCLGSISNLNVSLCARYPEKRFVPDGNRISWDSKKGFTIPSYMISYAGMVFCEA KINDESYQSIMYIVVVVGYRIYDVVLSPSHGIELSVGEKLVLNCTARTELNVGIDFNWEY PSSKHQHKKLVNRDLKTQSGSEMKKFLSTLTIDGVTRSDQGLYTCAASSGLMTKKNST FVRVHEK | |

| SEQ ID NO: 9 | LFV-F Trap polypeptide |
|---|---|
| GLPSVSLDLPRLSIQKDILTIKANTTLQITCRGQRDLDWLWPNNQSGSEQRVEVTECSDG LFCKTLTIPKVIGNDTGAYKCFYRETDLASVIYVYVQDYRSPFIASVSDQHGVVYITENK NKTVVIPCLGSISNLNVSLCARYPEKRFVPDGNRISWDSKKGFTIPSYMISYAGMVFCEA KINDESYQSIMYIVVVVGYRIYDVVLSPSHGIELSVGEKLVLNCTARTELNVGIDFNWEY PSSKHQHKKLVNRDLKTQSGSEMKKFLSTLTIDGVTRSDQGLYTCAASSGLMTKKNST FVRVHEKPFVAFGSGMESLVEATVGERVRIPAKYLGYPPPEIKWYKNGIPLESNHTIKAG HVLTIMEVSERDTGNYTVILTNPISKEKQSHVVSLVVYVPP | |

| SEQ ID NO: 10 | LFV-G Trap polypeptide |
|---|---|
| SPFIASVSDQHGVVYITENKAKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 11 | LFV-H Trap polypeptide |
|---|---|
| SPFIASVSDQHGVVYITENKQKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 12 | LFV-I Trap polypeptide |
|---|---|
| SPFIASVSDQHGVVYITENKNKTVVIPCLGSISNLAVSLCARYPEKRFVPDGNRISWDSK KGFTIPSYMISYAGMVFCEAKINDESYQSIMYIVVVVGYRIYDVVLSPSHGIELSVGEKL | |
| VLNCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 13 | LFV-J Trap polypeptide |
|---|---|
| SPFIASVSDQHGVVYITENKNKTVVIPCLGSISNLQVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 14 | LFV-K Trap polypeptide |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLQCTARTELNVGIDFNWEYPSSKHQHKKLVNRDLKTQSGSEMKKFLSTLTIDGVTRS DQGLYTCAASSGLMTKKNSTFVRVHEK | |

| SEQ ID NO: 15 | LFV-L Trap polypeptide |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKQSTFVRVHEK | |

| SEQ ID NO: 16 | LFV-M Trap polypeptide |
|---|---|
| GRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIGGGGSGGSPFIASVSDQHGVVYIT ENKNKTVVIPCLGSISNLNVSLCARYPEKRFVPDGNRISWDSKKGFTIPSYMISYAGMVF CEAKINDESYQSIMYIVVVVGYRIYDVVLSPSHGIELSVGEKLVLNCTARTELNVGIDFN WEYPSSKHQHKKLVNRDLKTQSGSEMKKFLSTLTIDGVTRSDQGLYTCAASSGLMTKK NSTFVRVHEK | |

| SEQ ID NO: 17 | LFV-N Trap polypeptide |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGGGGSGRPFVEMYSEIPEIIHMTEGRELVIPCR VTSPNITVTLKKFPLDTLIPDGKRIIWDSRKGFIISNATYKEIGLLTCEATVNGHLYKTNYL THRQTNTII | |

| SEQ ID NO: 18 | LFV-P Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLQCTARTELNVGIDFNWEYPSSKHQHKKLVNRDLKTQSGSEMKKFLSTLTI | |
| DGVTRSDQGLYTCAASSGLMTKKNSTFVRVHEK | |

| SEQ ID NO: 19 | LFV-Q Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLQCTARTELNVGIDFNWEYPSSKHQHKKLVNRDLKTQSGSEMKKFLSTLTI DGVTRSDQGLYTCAASSGLMTKKNSTFVRVHEKGGGGSGRPFVEMYSEIPEIIHMTEGR ELVIPCRVTSPNITVTLKKFPLDTLIPDGKRIIWDSRKGFIISNATYKEIGLLTCEATVNGHL YKTNYLTHRQTNTII | |

| SEQ ID NO: 20 | LFV-R Trap polypeptide |
|---|---|
| GRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIGGGGSGGSPFIASVSDQHGVVYIT ENKNKTVVIPCLGSISNLNVSLCARYPEKRFVPDGNRISWDSKKGFTIPSYMISYAGMVF CEAKINDESYQSIMYIVVVVGYRIYDVVLSPSHGIELSVGEKLVLQCTARTELNVGIDFN WEYPSSKHQHKKLVNRDLKTQSGSEMKKFLSTLTIDGVTRSDQGLYTCAASSGLMTKK NSTFVRVHEK | |

| SEQ ID NO: 21 | LFV-S Trap polypeptide |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEKGGGGSGRPFVEMYSEIPEIIHMTEGR ELVIPCRVTSPNITVTLKKFPLDTLIPDGKRIIWDSRKGFIISNATYKEIGLLTCEATVNGHL YKTNYLTHRQTNTII | |

| SEQ ID NO: 22 | LFV-001 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHITGYRIYDVVLSPSHGIELSVGEKLVLNCTARTELN VGIDFNWEYPSSKHQHKKLVNRDLKTQSGSEMKKFLSTLTIDGVTRSDQGLYTCAASS GLMTKKNSTFVRVHEK | |

| SEQ ID NO: 23 | LFV-010 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHAQDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHITGNELYDVVLSPSHGIELSVGEKLVLNCTARTEL NVGIDFNWEYPSSKHQHKKLVNRDLKTQSGSEMKKFLSTLTIDGVTRSDQGLYTCAAS | |
| SGLMTKKNSTFVRVHEK | |

| SEQ ID NO: 24 | LFV-002 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHIGYRIYDVVLSPSHGIELSVGEKLVLNCTARTELN VGIDFNWEYPSSKHQHKKLVNRDLKTQSGSEMKKFLSTLTIDGVTRSDQGLYTCAASS GLMTKKNSTFVRVHEK | |

| SEQ ID NO: 25 | LFV-003 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHIGGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEK | |

| SEQ ID NO: 26 | LFV-004 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFIVHVTGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEK | |

| SEQ ID NO: 27 | LFV-005 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEK | |

| SEQ ID NO: 28 | LFV-006 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS | |
| SGLMTKKNSTFVRVHEK | |

| SEQ ID NO: 29 | LFV-007 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSIMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEK | |

| SEQ ID NO: 30 | LFV-008 Trap polypeptide |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSAMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEK | |

| SEQ ID NO: 31 | Amino acid sequence of human IgG1 constant region domain (comprising Fc and hinge regions) |
|---|---|
| | |

| SEQ ID NO: 32 | LFV-A Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVGPGDKTHTCPPCPAPEL LGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKL TVDKSRWQQGNVFSCSVMHEALHNHYTQKSLSLSPGK | |

| SEQ ID NO: 33 | LFV-B Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEKGPGDKTHTCPPCPAPELLGGPSVFLF | |
| PPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLTVDKSRWQ QGNVFSCSVMHEALHNHYTQKSLSLSPGK | |

| SEQ ID NO: 34 | LFV-C Recombinant receptor molecule |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 35 | LFV-D Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEKPFVAFGSGMESLVEATVGERVRIPAK YLGYPPPEIKWYKNGIPLESNHTIKAGHVLTIMEVSERDTGNYTVILTNPISKEKQSHVV SLVVYVPPGPGDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKSLSL SPGK | |

| SEQ ID NO: 36 | LFV-E Recombinant receptor molecule |
|---|---|
| GLPSVSLDLPRLSIQKDILTIKANTTLQITCRGQRDLDWLWPNNQSGSEQRVEVTECSDG LFCKTLTIPKVIGNDTGAYKCFYRETDLASVIYVYVQDYRSPFIASVSDQHGVVYITENK NKTVVIPCLGSISNLNVSLCARYPEKRFVPDGNRISWDSKKGFTIPSYMISYAGMVFCEA KINDESYQSIMYIVVVVGYRIYDVVLSPSHGIELSVGEKLVLNCTARTELNVGIDFNWEY PSSKHQHKKLVNRDLKTQSGSEMKKFLSTLTIDGVTRSDQGLYTCAASSGLMTKKNST FVRVHEKGPGDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNG | |
| QPENNYKTTPPVLDSDGSFFLYSKLTVDKSRWQQGNVFSCSVMHEALHNHYTQKSLSL SPGK | |

| SEQ ID NO: 37 | LFV-F Recombinant receptor molecule |
|---|---|
| GLPSVSLDLPRLSIQKDILTIKANTTLQITCRGQRDLDWLWPNNQSGSEQRVEVTECSDG LFCKTLTIPKVIGNDTGAYKCFYRETDLASVIYVYVQDYRSPFIASVSDQHGVVYITENK NKTVVIPCLGSISNLNVSLCARYPEKRFVPDGNRISWDSKKGFTIPSYMISYAGMVFCEA KINDESYQSIMYIVVVVGYRIYDVVLSPSHGIELSVGEKLVLNCTARTELNVGIDFNWEY PSSKHQHKKLVNRDLKTQSGSEMKKFLSTLTIDGVTRSDQGLYTCAASSGLMTKKNST FVRVHEKPFVAFGSGMESLVEATVGERVRIPAKYLGYPPPEIKWYKNGIPLESNHTIKAG HVLTIMEVSERDTGNYTVILTNPISKEKQSHVVSLVVYVPPGPGDKTHTCPPCPAPELLG GPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | |

| SEQ ID NO: 38 | LFV-G Recombinant receptor molecule |
|---|---|
| SPFIASVSDQHGVVYITENKAKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 39 | LFV-H Recombinant receptor molecule |
|---|---|
| SPFIASVSDQHGVVYITENKQKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |
| | |

| SEQ ID NO: 40 | LFV-I Recombinant receptor molecule |
|---|---|
| SPFIASVSDQHGVVYITENKNKTVVIPCLGSISNLAVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 41 | LFV-J Recombinant receptor molecule |
|---|---|
| SPFIASVSDQHGVVYITENKNKTVVIPCLGSISNLQVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 42 | LFV-K Recombinant receptor molecule |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLQCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 43 | LFV-L Recombinant receptor molecule |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS | |
| DQGLYTCAASSGLMTKKQSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNVFS CSVMHEALHNHYTQKSLSLSPGK | |

| SEQ ID NO: 44 | LFV-M Recombinant receptor molecule |
|---|---|
| GRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIGGGGSGGSPFIASVSDQHGVVYIT ENKNKTVVIPCLGSISNLNVSLCARYPEKRFVPDGNRISWDSKKGFTIPSYMISYAGMVF CEAKINDESYQSIMYIVVVVGYRIYDVVLSPSHGIELSVGEKLVLNCTARTELNVGIDFN WEYPSSKHQHKKLVNRDLKTQSGSEMKKFLSTLTIDGVTRSDQGLYTCAASSGLMTKK NSTFVRVHEKGPGDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKS LSLSPGK | |

| SEQ ID NO: 45 | LFV-N Recombinant receptor molecule |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGGGGSGRPFVEMYSEIPEIIHMTEGRELVIPCR VTSPNITVTLKKFPLDTLIPDGKRIIWDSRKGFIISNATYKEIGLLTCEATVNGHLYKTNYL THRQTNTIIGPGDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSN KALPAPIEKTISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHNHYTQKSLSL SPGK | |

| SEQ ID NO: 46 | LFV-O Recombinant receptor molecule |
|---|---|
| SPFIASVSDQHGVVYITENKNKTVVIPCLGSISNLNVSLCARYPEKRFVPDGNRISWDSK KGFTIPSYMISYAGMVFCEAKINDESYQSIMYIVVVVGYRIYDVVLSPSHGIELSVGEKL VLNCTARTELNVGIDFNWEYPSSKHQHKKLVNRDLKTQSGSEMKKFLSTLTIDGVTRS DQGLYTCAASSGLMTKKNSTFVRVHEKGPGDKTHTCPPCPAPELLGGPSVFLFPPKPKD TLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSL | |
| TCLVKGFYPSDIAVEWESNGQPENNYKTTPPVLDSDGSFFLYSKLTVDKSRWQQGNVFS CSVMHEALHNHYTQKSLSLSPGAGGGGSGGGGSGGGGSGRPFVEMYSEIPEIIHMTEG RELVIPCRVTSPNITVTLKKFPLDTLIPDGKRIIWDSRKGFIISNATYKEIGLLTCEATVNG HLYKTNYLTHRQTNTIIDVVLSPSHGIELSVGEKLVLNCTARTELNVGIDFNWEYPSSKH QHKKLVNRDLKTQSGSEMKKFLSTLTIDGVTRSDQGLYTCAASSGLMTKKNSTFVRVH EK | |

| SEQ ID NO: 47 | LFV-P Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLQCTARTELNVGIDFNWEYPSSKHQHKKLVNRDLKTQSGSEMKKFLSTLTI DGVTRSDQGLYTCAASSGLMTKKNSTFVRVHEKGPGDKTHTCPPCPAPELLGGPSVFLF PPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLTVDKSRWQ QGNVFSCSVMHEALHNHYTQKSLSLSPGK | |

| SEQ ID NO: 48 | LFV-Q Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLQCTARTELNVGIDFNWEYPSSKHQHKKLVNRDLKTQSGSEMKKFLSTLTI DGVTRSDQGLYTCAASSGLMTKKNSTFVRVHEKGGGGSGRPFVEMYSEIPEIIHMTEGR ELVIPCRVTSPNITVTLKKFPLDTLIPDGKRIIWDSRKGFIISNATYKEIGLLTCEATVNGHL YKTNYLTHRQTNTIIGPGDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEALHNHY TQKSLSLSPGK | |

| SEQ ID NO: 49 | LFV-R Recombinant receptor molecule |
|---|---|
| GRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIGGGGSGGSPFIASVSDQHGVVYIT ENKNKTVVIPCLGSISNLNVSLCARYPEKRFVPDGNRISWDSKKGFTIPSYMISYAGMVF CEAKINDESYQSIMYIVVVVGYRIYDVVLSPSHGIELSVGEKLVLQCTARTELNVGIDFN WEYPSSKHQHKKLVNRDLKTQSGSEMKKFLSTLTIDGVTRSDQGLYTCAASSGLMTKK | |
| NSTFVRVHEKGPGDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKS LSLSPGK | |

| SEQ ID NO: 50 | LFV-S Recombinant receptor molecule |
|---|---|
| YVQDYRSPFIASVSDQHGVVYITENKNKTVVIPCLGSISNLNVSLCARYPEKRFVPDGN RISWDSKKGFTIPSYMISYAGMVFCEAKINDESYQSIMYIVVVVGYRIYDVVLSPSHGIE LSVGEKLVLNCTARTELNVGIDFNWEYPSSKHQHKKLVNRDLKTQSGSEMKKFLSTLTI DGVTRSDQGLYTCAASSGLMTKKNSTFVRVHEKGGGGSGRPFVEMYSEIPEIIHMTEGR ELVIPCRVTSPNITVTLKKFPLDTLIPDGKRIIWDSRKGFIISNATYKEIGLLTCEATVNGHL YKTNYLTHRQTNTIIGPGDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEALHNHY TQKSLSLSPGK | |

| SEQ ID NO: 51 | LFV-001 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHITGYRIYDVVLSPSHGIELSVGEKLVLNCTARTELN VGIDFNWEYPSSKHQHKKLVNRDLKTQSGSEMKKFLSTLTIDGVTRSDQGLYTCAASS GLMTKKNSTFVRVHEKGPGDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGK EYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHN HYTQKSLSLSPGK | |

| SEQ ID NO: 52 | LFV-010 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHAQDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHITGNELYDVVLSPSHGIELSVGEKLVLNCTARTEL | |
| NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 53 | LFV-002 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHIGYRIYDVVLSPSHGIELSVGEKLVLNCTARTELN VGIDFNWEYPSSKHQHKKLVNRDLKTQSGSEMKKFLSTLTIDGVTRSDQGLYTCAASS GLMTKKNSTFVRVHEKGPGDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGK EYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHN HYTQKSLSLSPGK | |

| SEQ ID NO: 54 | LFV-003 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFLVHIGGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 55 | LFV-004 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH | |
| DALYLQCETTWGDQDFLSNPFIVHVTGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 56 | LFV-005 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNPFIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 57 | LFV-006 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSNMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 58 | LFV-007 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK | |
| PDTLLVNRKDAMWVPCLVSIPGLNVTLRSQSSVLWPDGQEVVWDDRRGMLVSTPLLH DALYLQCETTWGDQDFLSIMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

| SEQ ID NO: 59 | LFV-008 Recombinant receptor molecule |
|---|---|
| YSMTPPTLNITEESHVIDTGDSLSISCRGQHPLEWAWPGAQEAPATGDKDSEDTGVVRD CEGTDARPYCKVLLLHEVHANDTGSYVCYYKYIKARIEGTTAASSYVFVRDFEQPFINK PDTLLVNRKDAMWVPCLVSIPGLNVTLRSQSSVLWPDGQEVVWDDRRGMLVSTPLLH DALYLQCETTWGDQDFLSAMYIVVVVGYRIYDVVLSPSHGIELSVGEKLVLNCTARTEL NVGIDFNWEYPSSKHQHKKLVNRDLKTQSGSEMKKFLSTLTIDGVTRSDQGLYTCAAS SGLMTKKNSTFVRVHEKGPGDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVMHEALHN HYTQKSLSLSPGK | |

### EXAMPLES

The invention will now be further described below in conjunction with specific examples. It should be understood that these examples are merely illustrative of the present invention and are not intended to limit the scope of the present invention. Experimental methods for which specific conditions are not indicated in the following examples are generally in accordance with conventional conditions, or in accordance with conditions recommended by the manufacturer. Unless otherwise indicated, all percentages and parts are given by weight.

### Example 1 - Design and expression of recombinant receptor molecules

Firstly, various Trap polypeptides comprising different sequences derived from VEGFR2 were constructed, as shown in Table 1A below. Some of the Trap polypeptides comprise fragments derived from VEGFR1 or VEGFR3. These fragments could be operably linked to each other through a linker. The numbering of the mutation sites used herein, such as N143A, N158Q, N318Q, etc., were all relative to the corresponding positions of SEQ ID NO: 2.

**Table 1A**

| Trap polypeptide nomenclature | Sequence description | SEQ ID No |
|---|---|---|
| LFV-A | Amino acids 117-218 of SEQ ID NO: 2 | 4 |
| LFV-B | Amino acids 117-327 of SEQ ID NO: 2 | 5 |
| LFV-C | Amino acids 123-327 of SEQ ID NO: 2 | 6 |
| LFV-D | Amino acids 117-421 of SEQ ID NO: 2 | 7 |
| LFV-E | Amino acids 23-327 of SEQ ID NO: 2 | 8 |
| LFV-F | Amino acids 23-421 of SEQ ID NO: 2 | 9 |
| LFV-G | LFV-C with N143A mutation | 10 |
| LFV-H | LFV-C with N143Q mutation | 11 |
| LFV-I | LFV-C with N158A mutation | 12 |
| LFV-J | LFV-C with N158Q mutation | 13 |
| LFV-K | LFV-C with N245Q mutation | 14 |
| LFV-L | LFV-C with N318Q mutation | 15 |
| LFV-M | Amino acids 132-230 of VEGFR1 operably linked to the N-terminal of LFV-C | 16 |
| LFV-N | Amino acids 132-230 of VEGFR1 operably linked to the C-terminal of LFV-C | 17 |
| LFV-O | VEGFR1 (132-230)-VEGFR2 (225-327) operably linked to LFV-C | 46 |
| LFV-P | LFV-B with N245Q mutation | 18 |
| LFV-Q | Amino acids 132-230 of VEGFR1 operably linked to LFV-P | 19 |
| LFV-R | LFV-M with N245Q mutation | 20 |
| LFV-S | Amino acids 132-230 of VEGFR1 operably linked to LFV-B | 21 |
| LFV-001 or V0 | Amino acids 25-216 of VEGFR3 operably linked to amino acids 221-327 of SEQ ID NO: 2 | 22 |

Each of the designed Trap polypeptides described above could be linked to the constant region of human immunoglobulin IgG1 (fused to the Fc and hinge regions) via a short artificial linker sequence Gly-Pro-Gly (GPG), thus being constructed together. After gene synthesis, vector construction and extraction, each of the above sequences was transiently transfected into HEK293 or CHO cells (purchased from Gibco Life), and then the resulting mixture was purified using affinity chromatography with protein A and further refinement with molecular sieve (among which, LFV-D, LFV-F and LFV-001/V0 molecules had met the purity requirements after one-step purification with protein A, without undergoing further purification with molecular sieve). The resulting fusion polypeptide of truncated VEGFR2 and Fc was also referred to as a recombinant receptor molecule or ligand-binding molecule herein.

Eylea, Avastin, R3A and OPT were used as controls. Eylea was used in accordance with the sequence disclosed in WO0075319. R3A was constructed by using amino acids 25-329 of VEGFR3 linked to the constant region of human immunoglobulin IgG1 (fused to the Fc and hinge regions). OPT was constructed as described in WO2015/123715 A1 (which used amino acids 25-329, including amino acids 47-314 of VEGFR3, and underwent glycosylation modification at positions 104-106 as described in WO2015/123715 A1 to modify the N-D-T sequence into a non Q-D-T sequence), then the resulting sequence was linked to the constant region of human immunoglobulin IgG1 (fused to the Fc and hinge regions), such that the molecule so constructed was referred to herein as OPT.

Expression and purification profiles of various recombinant receptor molecules were shown in Table 1B below.

**Table 1B**

| Trap molecules | SEC single peak purity after one-step affinity chromatography purification with protein A | SEC single peak purity after further purification with molecular sieve |
|---|---|---|
| LFV-A | 80% | 97.8% |
| LFV-B | 83.5% | 96.6% |
| LFV-C | 80% | 99.4% |
| LFV-D | 97.2% | / |
| LFV-E | 87% | 99.7% |
| LFV-F | 98.5% | / |
| LFV-Q | 66.4% | 99.9% |
| LFV-M | 52.1% | 99.7% |
| LFV-P | 83.8% | 96.7% |
| LFV-R | 78.7% | 99.6% |
| LFV-S | 85.4% | 95% |
| R3A | 69% | 95% |
| OPT | 66% | 96% |
| LFV-001/V0 | 99.6% | / |

### Example 2 - Construction, production and binding test of recombinant receptor molecules derived from VEGFR2 and VEGFR3

### 2.1 Construction and expression of recombinant receptor molecules

In order to construct novel receptor-associated trap proteins with a better binding capacity in human, a fragment from human VEGFR3 (positions 25-216 of SEQ ID NO: 3) and a fragment from human VEGFR2 (positions 221-327 of SEQ ID NO: 2) were used as constituent elements, and a series of linker sequences were designed between them (as shown in Table 2A below).

Specifically, for LFV-001 or V0, a sequence in native VEGFR3 (positions 217-227 of SEQ ID NO: 3) was used; for LFV-010, a sequence in which the sequence as used in LFV-001 was further extended a bit toward the C-terminal in VEGFR3 was used; for LFV-002, Thr was removed from the linker sequence based on LFV-001 to introduce flexibility in length; and for LFV-003, this Thr was replaced with Gly to introduce greater flexibility in backbone structure torsion. On the other hand, in LFV-004 to 008, the VEGFR3-derived linker sequence used in LFV-001 was subjected to a series of mutations towards the linker sequence in the corresponding region of VEGFR2 (as shown in Table 1 below), among which, in LFV-008, an Ile derived from VEGFR2 was mutated into a same polar Ala with smaller side chains to reduce inter-side chain steric clash. As control trap proteins, LFV-B comprising only the VEGFR2 fragment as set forth in 117-327 of SEQ ID NO: 2 and R3A comprising only the VEGFR3 fragment as set forth in 25-329 of SEQ ID NO: 3 were designed.

**Table 2A**

| Trap polypeptide nomenclature | Sequence description | Intervening linker sequences | SEQ ID No |
|---|---|---|---|
| LFV-001/V0 | Positions 25-216 of SEQ ID NO: 3 + 221-327 of SEQ ID NO: 2 | SNPFLVHITG | 22 |
| LFV-010/V0-4 | Positions 25-216 of SEQ ID NO: 3 + 221-327 of SEQ ID NO: 2 | SNPFLVHITGNELYD | 23 |
| LFV-002/V0_2 | Positions 25-216 of SEQ ID NO: 3 + 221-327 of SEQ ID NO: 2 | SNPFLVHI-G | 24 |
| LFV-003/V0_3 | Positions 25-216 of SEQ ID NO: 3 + 221-327 of SEQ ID NO: 2 | SNPFLVHIGG | 25 |
| LFV-004/V1 | Positions 25-216 of SEQ ID NO: 3 +221-327 of SEQ ID NO: 2 | SNPFIVHVTG | 26 |
| LFV-005/V2 | Positions 25-216 of SEQ ID NO: 3 + 221-327 of SEQ ID NO: 2 | SNPFIVVVVG | 27 |
| LFV-006/V3 | Positions 25-216 of SEQ ID NO: 3 + 221-327 of SEQ ID NO: 2 | SNMYIVVVVG | 28 |
| LFV-007/V4 | Positions 25-216 of SEQ ID NO: 3 + 221-327 of SEQ ID NO: 2 | SIMYIVVVVG | 29 |
| LFV-008/V5 | Positions 25-216 of SEQ ID NO: 3 + 221-327 of SEQ ID NO: 2 | SAMYIVVVVG | 30 |

Each of the above-mentioned trap polypeptides could be linked to the constant region of human IgG1 immunoglobulin (fused to the Fc and hinge regions) via a short artificial linker sequence Gly-Pro-Gly (GPG), thus being constructed together. After gene synthesis, vector construction and extraction, each of the above sequences was transiently transfected into HEK293 cells (from Gibco Life), and then the resulting mixture underwent one-step purification with protein A affinity elution to obtain fusion proteins, hereinafter referred to as recombinant receptor molecules, and their expression characteristics were shown in Table 2B below:

**Table 2B.**

| Trap polypeptides | Yield from transiently transformed HEK293 (mg/L) | SEC single peak purity |
|---|---|---|
| LFV-001/V0 | 23.6 | 99.60% |
| LFV-010/V0-4 | 127 | 95.40% |
| LFV-002/V0_2 | 11 | 93.40% |
| LFV-003/V0_3 | 63.9 | 86.80% |
| LFV-004/V1 | 74.5 | 85.90% |
| LFV-005/V2 | 52.9 | 78.20% |
| LFV-006/V3 | 11.8 | 40.00% |
| LFV-007/V4 | 2.5 | 50.60% |
| LFV-008/V5 | 9.1 | 48.70% |
| / | / | / |
| LFV-B | 38 | 82.70% |
| R3A | 11 | 69.00% |

### 2.2 Binding test to EGF-A, VEGF-C and VEGF-D

VEGF-A165, VEGF-C and VEGF-D polypeptide molecules were purchased from ACROBiosystems Inc. VEGF molecules at 100-300 ng/well prepared in PBS were used for coating overnight at 4°C, the next day, the plates were washed with PBS, and 1% BSA solution was added to block for 2 hours at room temperature, after which the plates were washed again with PBS. The recombinant receptor molecules obtained by expression in Example 1 were added to each well after gradient dilution with PBT solution (0.5% BSA dissolved in PBS solution with 0.05% Tween-20) from high to low concentrations, allowing for incubation at room temperature and 300 rpm for 1.5 hours, subsequently the plates were washed three times with PBST solution (PBS with 0.05% Tween-20). Goat anti-human Fc secondary antibody conjugated with horseradish peroxidase diluted in PBT was added, followed by incubation at room temperature and 300 rpm for 1 hour, after that, the plates were washed three times with PBST solution. Then TMB was added for color development, terminated with 5% hydrochloric acid solution, and finally, the OD values at 450 nm were read using a microplate reader.

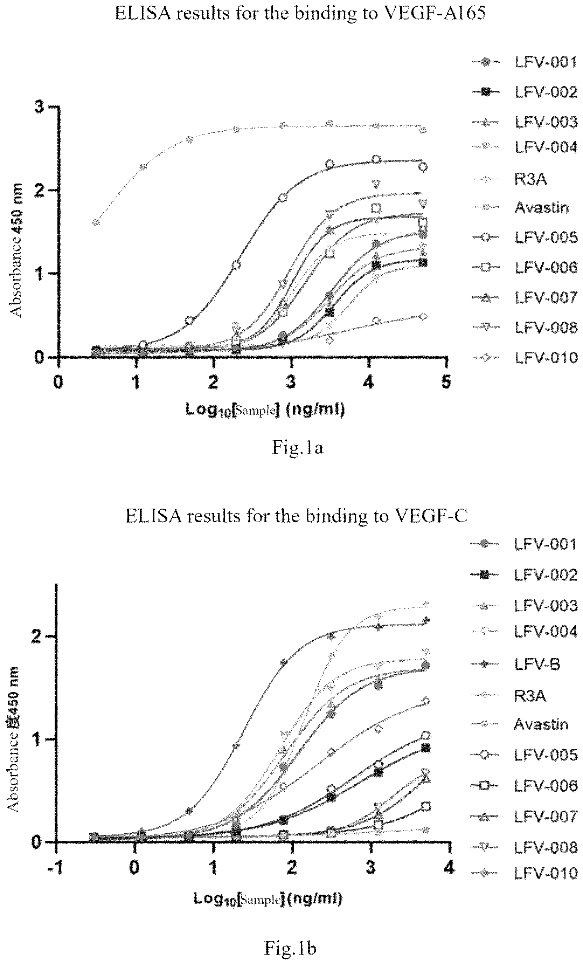

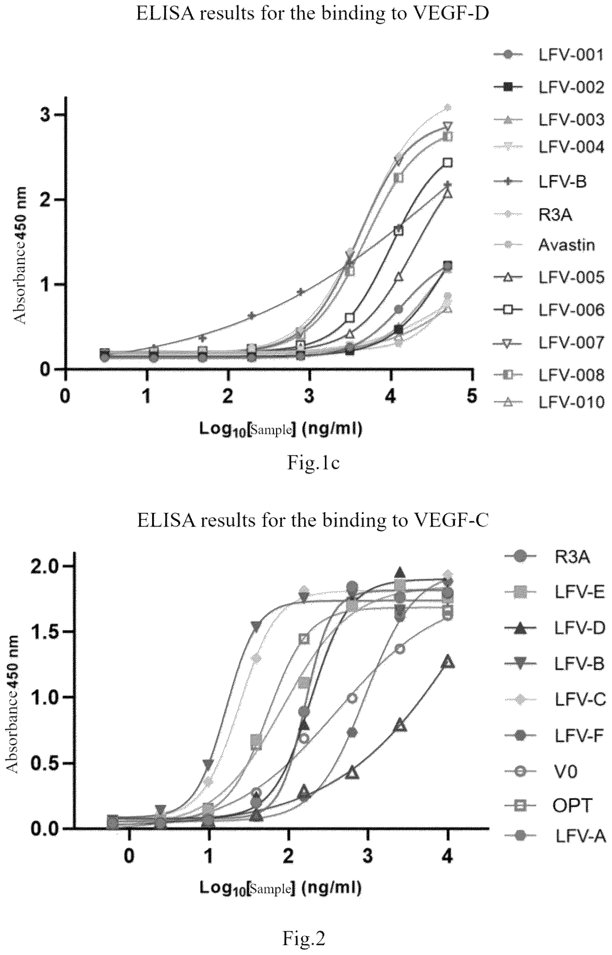

The results were shown in Figure 1, in which various recombinant ligand-binding molecules exhibited a binding ability to VEGF-A (represented by VEGF-A165), VEGF-C and VEGF-D. Besides, it was also unexpectedly found that the LFV-B molecule as a control exhibited a stronger binding ability to VEGF-C and VEGF-D than that of R3A.

### Example 3 - Binding of recombinant receptor molecules to VEGF-C

100 ng/well of VEGF-C prepared in PBS was used for coating overnight at 4°C, the next day, the plates were washed with PBS, and 1% BSA solution was added to block for 2 hours at room temperature, after which the plates were washed again with PBS. The recombinant receptor molecules obtained by expression in Example 1 were added to each well after gradient dilution from high to low concentrations, allowing for incubation at room temperature and 300 rpm for 1.5 hours, subsequently the plates were washed three times with PBST solution. Goat anti-human Fc secondary antibody conjugated with horseradish peroxidase was added, followed by incubation at room temperature and 300 rpm for 1 hour. After that, the plates were washed three times with PBST solution, then TMB was added for color development, terminated with 5% hydrochloric acid solution, and finally, the OD values at 450 nm were read using a microplate reader.

As shown in Figure 2, the related recombinant receptor molecules had a good binding ability to VEGF-C, among which LFV-B and its truncated form LFV-C had the optimal binding ability to VEGF-C, which was superior to that of OPT. LFV-E also had a binding ability close to that of OPT. Their binding EC₅₀ values were shown in Table 3 below:

**Table 3.**

| | **R3A** | **LFV-E** | **LFV-D** | **LFV-B** | **LFV-C** | **LFV-A** | **OPT** | **LFV-F** | **V0** |
|---|---|---|---|---|---|---|---|---|---|
| **EC₅₀ (ng/ml)** | **161.6** | **85.19** | **191.7** | **16.24** | **24.16** | **/** | **54.59** | **916** | **398.5** |
| **EC₅₀ (nM)** | **1.35** | **0.71** | **1.6** | **0.16** | **0.24** | **/** | **0.45** | **6.5** | **3.3** |

### Example 4 - Blocking experiments of recombinant receptor molecules on the binding of VEGF-C and VEGF-D to VEGFR2

VEGFR2-Fc (Met1-Glu764) at 500 ng/well was used for coating overnight at 4°C, the next day the plates were washed with PBS, then BSA solution was added to block for 2 hours at room temperature. During the blocking period, each recombinant receptor molecule was serially diluted from high to low concentrations, mixed evenly with 100 ng/well of VEGF-C or VEGF-D (both labeled with His-tag), allowing for incubation at room temperature for 30 minutes. Subsequently, the VEGFR2-Fc plates were washed, and the incubation solution of recombinant receptor molecules with VEGF-C and VEGF-D was added therein to allow for incubation and binding at room temperature for 1 hour. The plates were washed with PBST, then anti-polyhistidine secondary antibody conjugated with horseradish peroxidase was added to allow for incubation and binding at room temperature for 1 hour. Then the plates were washed with PBST, TMB was added for color development, terminated with 5% hydrochloric acid solution, and finally, the OD values at 450 nm were read using a microplate reader.

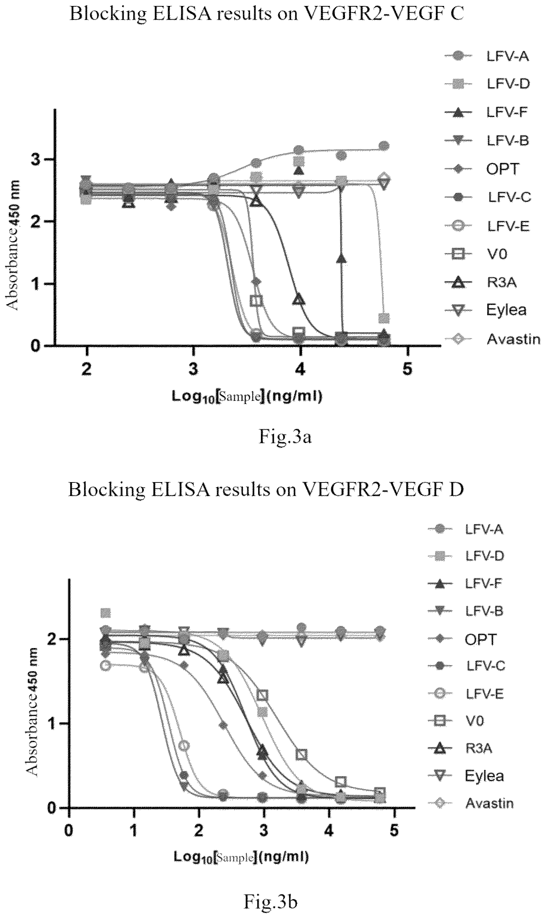

As shown in Figure 3, the related recombinant receptor molecules had a certain blocking ability, among which LFV-B and its truncated form LFV-C, as well as LFV-E had the best blocking ability, and were all superior to OPT. The V0 molecule also had a better blocking ability for VEGF-C. The blocking EC₅₀ values of each recombinant receptor molecule on the binding of VEGF-C and VEGF-D to VEGFR2 were shown in Table 4 and Table 5 below, respectively.

**Table 4. Blocking EC₅₀ values on VEGFR2-VEGFC binding**

| | **LFV-A** | **LFV-D** | **LFV-F** | **LFV-B** | **OPT** | **LFV-C** | **LFV-E** | **V0** | **R3A** | **Eylea** | **Avastin** |
|---|---|---|---|---|---|---|---|---|---|---|---|
| **EC₅₀(ng/ml)** | **/** | **58280** | **24008** | **2085** | **3567** | **2252** | **2275** | **3593** | **7808** | **/** | **/** |
| **EC₅₀(nM)** | **/** | **485.7** | **170.6** | **20.85** | **29.7** | **22.52** | **18.96** | **29.9** | **65.1** | **/** | **/** |

**Table 5. Blocking EC₅₀ values on VEGFR2-VEGFD binding**

| | **LFV-A** | **LFV-D** | **LFV-F** | **LFV-B** | **OPT** | **LFV-C** | **LFV-E** | **V0** | **R3A** | **Eylea** | **Avastin** |
|---|---|---|---|---|---|---|---|---|---|---|---|
| **EC₅₀(ng/ml)** | **/** | **920.4** | **521.6** | **26.78** | **241.3** | **34.02** | **50.54** | **1542** | **532.2** | **/** | **/** |
| **EC₅₀(nM)** | **/** | **7.7** | **3.7** | **0.27** | **2.01** | **0.34** | **0.42** | **12.85** | **4.4** | **/** | **/** |

### Example 5 - Blocking experiments of recombinant receptor molecules on the binding of VEGF-C and VEGF-D to VEGFR3

VEGFR3-Fc (Tyr25-IIe776) at 500 ng/well was used for coating overnight at 4°C, the next day, the plates were washed with PBS, then BSA solution was added to block for 2 hours at room temperature. During the blocking period, each recombinant receptor molecule was serially diluted from high to low concentrations, mixed evenly with 10 ng/well of VEGF-C or 500 ng/well of VEGF-D (both labeled with His-tag), allowing for incubation at room temperature for 30 minutes. Subsequently, the VEGFR3-Fc plates were washed, and the incubation solution was added therein to allow for incubation and binding at room temperature for 1 hour, afterwards, the plates were washed with PBST, then anti-polyhistidine secondary antibody conjugated with horseradish peroxidase was added to allow for incubation and binding at room temperature for 1 hour. The plates were washed with PBST, then TMB was added for color development, terminated with 5% hydrochloric acid solution, and finally, the OD values at 450 nm were read using a microplate reader.

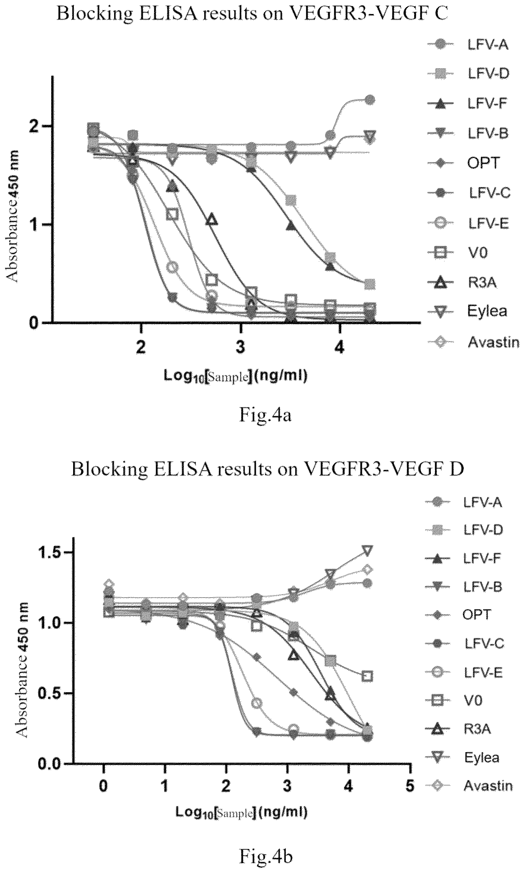

The results were shown in Figure 4, and it could be seen that the blocking results were essentially consistent with those in VEGFR2 blocking experiments. The blocking EC₅₀ values of each molecule on the binding of VEGF-C and VEGF-D to VEGFR3 were shown in Table 6 and Table 7 below, respectively.

**Table 6. Blocking EC₅₀ values on VEGFR3-VEGFC**

| | **LFV-A** | **LFV-D** | **LFV-F** | **LFV-B** | **OPT** | **LFV-C** | **LFV-E** | **V0** | **R3A** | **Eylea** | **Avastin** |
|---|---|---|---|---|---|---|---|---|---|---|---|
| **EC₅₀(ng/ml)** | **/** | **4323** | **2915** | **114.6** | **300.4** | **106.1** | **135.8** | **188.6** | **560.7** | **/** | **/** |
| **EC₅₀(nM)** | **/** | **36.03** | **20.72** | **1.15** | **2.5** | **1.06** | **1.13** | **1.57** | **4.67** | **/** | **/** |

**Table 7. Blocking EC₅₀ values on VEGFR3-VEGFD**

| | **LFV-A** | **LFV-D** | **LFV-F** | **LFV-B** | **OPT** | **LFV-C** | **LFV-E** | **V0** | **R3A** | **Eylea** | **Avastin** |
|---|---|---|---|---|---|---|---|---|---|---|---|
| **EC₅₀ (ng/ml)** | **/** | **10049** | **3440** | **122.3** | **688.1** | **124.6** | **179.5** | **2438** | **2447** | **/** | **/** |
| **EC₅₀(nM)** | **/** | **83.74** | **24.45** | **1.22** | **5.73** | **1.25** | **1.5** | **20.32** | **20.39** | **/** | **/** |

### Example 6 - Binding of recombinant receptor molecules to VEGF-A

VEGF-A 165 or VEGF-A 121 (purchased from ACROBiosystems Inc.) at 50 ng/well was used for coating overnight at 4°C, the next day, the plates were washed with PBS, and blocked at room temperature for 2 hours. The plates were washed again with PBS, the recombinant receptor molecules were added to each well after serial dilution from high to low concentrations to allow for incubation at room temperature for 1.5 hours. Subsequently the plates were washed three times with PBST solution, and horseradish peroxidase-conjugated goat anti-human Fc secondary antibody was added, followed by incubation at room temperature for 1 hour. The plates were washed three times with PBST solution, then TMB was added for color development, terminated with 5% hydrochloric acid solution, and finally, the OD values at 450 nm were read using a microplate reader.

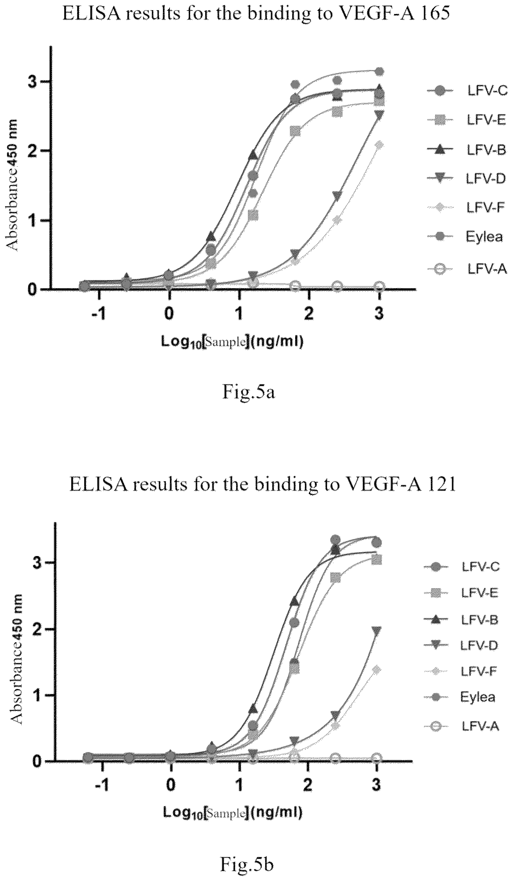

As shown in Figure 5, it could be seen that all VEGFR2-related recombinant receptor molecules, except LFV-A, LFV-D and LFV-F, had a better VEGF-A binding ability. LFV-B and its truncated form LFV-C had the optimal VEGF-A binding ability, which was superior to that of Eylea, while LFV-E had a VEGF-A binding capacity close to that of Eylea. The binding EC₅₀ values to VEGF-A 165 and VEGF-A 121 were shown in Table 8 and Table 9 below, respectively.

**Table 8. Binding EC₅₀ to VEGF-A 165**

| | **LFV-C** | **LFV-E** | **LFV-B** | **LFV-D** | **LFV-F** | **Eylea** |
|---|---|---|---|---|---|---|
| **EC₅₀ (ng/ml)** | **12.61** | **21.19** | **9.268** | **476.1** | **983.4** | **17.39** |
| **EC₅₀ (nM)** | **0.126** | **0.177** | **0.093** | **3.97** | **6.99** | **0.174** |

**Table 9. Binding EC₅₀ to VEGF-A 121**

| | **LFV-C** | **LFV-E** | **LFV-B** | **LFV-D** | **LFV-F** | **Eylea** |
|---|---|---|---|---|---|---|
| **EC₅₀ (ng/ml)** | **47.59** | **72.1** | **31.14** | **/** | **/** | **71.89** |
| **EC₅₀ (nM)** | **0.476** | **0.601** | **0.311** | **/** | **/** | **0.719** |

### Example 7 - Blocking experiments of recombinant receptor molecules on the binding of VEGF-A to VEGFR2

VEGFR2-Fc at 500 ng/well was used for coating overnight at 4°C, the next day, the plates were washed with PBS, then BSA solution was added to block for 2 hours at room temperature. During the blocking period, each recombinant receptor molecule was serially diluted from high to low concentrations, then mixed evenly with VEGF-A 165 at 60 ng/well or VEGF-A 121 at 120 ng/well (both labeled with His-tag), allowing for incubation at room temperature for 30 minutes. The VEGFR2-Fc plates were washed, and the incubation solution was added therein to allow for incubation and binding at room temperature for 1 hour. Afterwards, the plates were washed with PBST, then horseradish peroxidase-conjugated anti-polyhistidine secondary antibody was added to allow for incubation and binding at room temperature for 1 hour. Subsequently, the plates were washed with PBST, and TMB was added for color development, terminated with 5% hydrochloric acid solution. Finally, the OD values at 450 nm were read using a microplate reader.

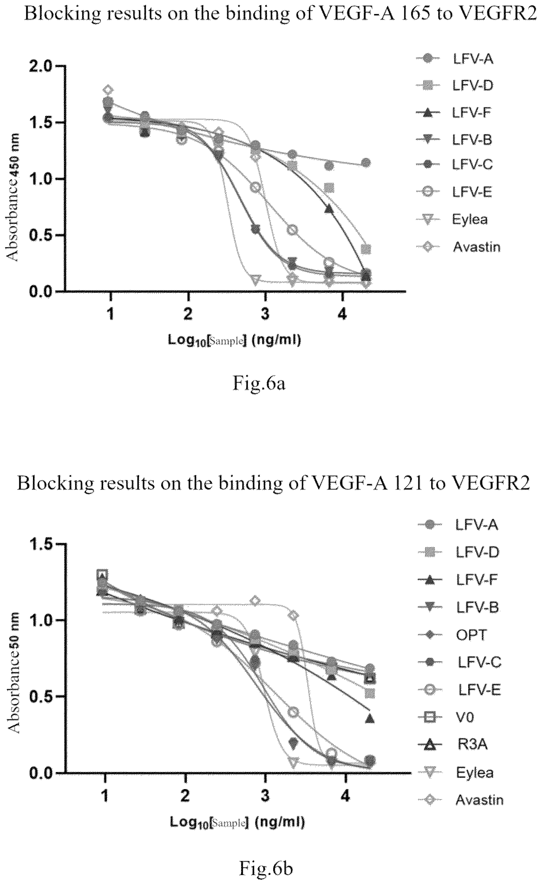

The results were shown in Figure 6, where LFV-B and its truncated form LFV-C had a better blocking ability, reaching a level superior to that of Bevacizumab and close to Eylea. The blocking EC₅₀ values on the binding of VEGF-A 165 and VEGF-A 121 to VEGFR2 were shown in Table 10 and Table 11 below, respectively.

**Table 10. Blocking EC₅₀ on VEGF-A 165 binding to VEGFR2**

| | **LFV-A** | **LFV-D** | **LFV-F** | **LFV-B** | **LFV-C** | **LFV-E** | **Eylea** | **Avastin** |
|---|---|---|---|---|---|---|---|---|
| **EC₅₀ (ng/ml)** | **/** | **/** | **/** | **467.1** | **456.8** | **1111** | **319.5** | **1002** |
| **EC₅₀(nM)** | **/** | **/** | **/** | **4.67** | **4.57** | **9.26** | **3.2** | **6.68** |

**Table 11. Blocking EC₅₀ on VEGF-A 121 binding to VEGFR2**

| | **LFV-A** | **LFV-D** | **LFV-F** | **LFV-B** | **LFV-C** | **LFV-E** | **Eylea** | **Avastin** |
|---|---|---|---|---|---|---|---|---|
| **EC₅₀ (ng/ml)** | **/** | **/** | **/** | **762.8** | **935** | **1278** | **954.9** | **3356** |
| **EC₅₀(nM)** | **/** | **/** | **/** | **7.63** | **9.35** | **10.65** | **9.55** | **22.37** |

### Example 8 - Detection experiments for binding affinity to VEGF-A, VEGF-C, and VEGF-D

Surface plasmon resonance method on a Biacore T200 instrument was used to detect affinity. The recombinant receptor molecules (including LFV-B, LFV-E, with OPT as a control) were captured by a Protein A chip, 1×HBS-EP was used as the running buffer, and antigens of various concentrations was flowed at a rate of 30 ul/min.

The detection results were shown in Table 12 below. It could be seen that LFV-B had a significantly better affinity to VEGF-C and VEGF-D than that of OPT, and its affinity to VEGF-A was also stronger, reaching 9.3 pM. LFV-E had a slightly better affinity to VEGF-C and VEGF-D than that of OPT, and due to a relatively low Ka value, LFV-E was significantly weaker in apparent affinity values compared to LFV-B, but there was not much difference between their Kd values, so they showed similar blocking potency in blocking experiments. Accordingly, the relatively lower blocking potency of OPT might be explained by its relatively significantly weaker Kd value.

**Table 12A.**

| | **VEGF-A** | | | **VEGF-C** | | | **VEGF -D** | | |
|---|---|---|---|---|---|---|---|---|---|
| | **Ka (1/Ms)** | **Kd (1/s)** | **KD (M)** | **Ka (1/Ms)** | **Kd (1/s)** | **KD (M)** | **Ka (1/Ms)** | **Kd (1/s)** | **KD (M)** |
| **LFV-B** | **1.13E+07** | **1.05E-04** | **9.341E-12** | **4.32E+06** | **3.88E-05** | **8.97E-12** | **1.51E+04** | **3.44E-05** | **2.28E-04** |
| **LFV-E** | **2.99E+06** | **1.23E-04** | **4.10E-11** | **9.38E+05** | **3.80E-05** | **4.05E-11** | **5855** | **8.67E-05** | **1.48E-08** |
| **OPT** | **N/A** | | | **3.29E+06** | **1.67E-04** | **5.07E-11** | **1.74E+04** | **3.11E-04** | **1.79E-08** |
| **V0** | **2.822E+06** | **0.001524** | **5.4E-10** | **N/A** | | | **N/A** | | |

**Table 12B.**

| | VEGF-A | | | VEGF-C | | | VEGF-D | | |
|---|---|---|---|---|---|---|---|---|---|
| | Ka(1/Ms) | Kd(1/s) | KD(M) | Ka(1/Ms) | Kd(1/s) | KD(M) | Ka(1/Ms) | Kd(1/s) | KD(M) |
| Full VEGFR2 extracellular domain | 3.727E+4 | 4.316E-4 | 1.158E-8 | 2.706E+4. | 4.521E-4 | 1.671E-8 | 3.208E+4 | 6.728E-4 | 2.098E-8 |

### Example 9 - Detection experiments for the binding to PDGF family molecules

In this example, the binding of VEGFR2 and its related truncated trap molecules to PDGF family molecules was also tested. Briefly, the binding of LFV-B, LFV-E, and VEGFR2-Fc (using the full extracellular domain sequence of VEGFR2: Met1-Glu764) to PDGF family molecules were examined using the surface plasmon resonance method. Nevertheless, it was found in the preliminary detection experiments that PDGF family molecules (purchased from R&D Systems Inc.) were prone to produce non-specific binding in the chip, resulting in a pseudo-elevation in measured values, such that the detection could not be completed. Therefore, PDGF-AA, PDGF-AB, PDGF-BB and PDGF-CC molecules were separately loaded through a coupling reaction on a CM5 chip, followed by influx of the recombinant receptor molecules, thus effectively eliminating the impact of non-specific binding on affinity determination.

The relevant detection results were shown in Table 13 below, and it could be seen that the full extracellular domain of VEGFR2 had a certain binding ability to all PDGF molecules, while LFV-C and LFV-B had significantly better binding abilities to PDGF family molecules, which unexpectedly characterized them as primary segments responsible for binding to PDGF. Therefore, by blocking PDGF family molecules through binding, they may exert more potent therapeutic effects and benefit more patients. LFV-E had a binding capacity to PDGF that was close to LFV-B and LFV-C.

**Table 13.**

| | **LFV-B** | | | **LFVE** | | | **LFV-C** | | | **VEGFR2-Fc** | | |
|---|---|---|---|---|---|---|---|---|---|---|---|---|
| | **Ka (1/Ms)** | **Kd (1/s)** | **KD (M)** | **Ka (1/Ms)** | **Kd (1/s)** | **KD (M)** | **Ka (1/Ms)** | **Kd (1/s)** | **KD (M)** | **Ka (1/Ms)** | **Kd (1/s)** | **KD (M)** |
| **PDGF-AA** | **5.79E+03** | **1.53E-04** | **2.63E-08** | **1.40E+05** | **1.34E-04** | **9.58E-10** | **3.42E+04** | **6.64E-04** | **1.94E08** | **-** | **-** | **3.113E-08** |
| **PDGF-AB** | **1.21E+05** | **1.92E-04** | **1.59E-09** | **4.68E+04** | **2.01E 04** | **4.29E-09** | **2.90E+04** | **8.60E-04** | **2.96E-08** | **-** | **-** | **3.506E-08** |
| **PDGF-BB** | **4.82E+05** | **4.70E-04** | **9.76E-09** | **5.83E+04** | **3.11E-04** | **5.33E-09** | **2.67E+04** | **5.51E-04** | **2.01E-08** | **-** | **-** | **1.410E-08** |
| **PDGF-CC** | - | - | **2.23E-08** | - | - | **4.61E-08** | - | - | **6.00E-08** | - | **-** | **7.364E-08** |

### Example 10 - In vivo pharmacokinetic experiments in mice

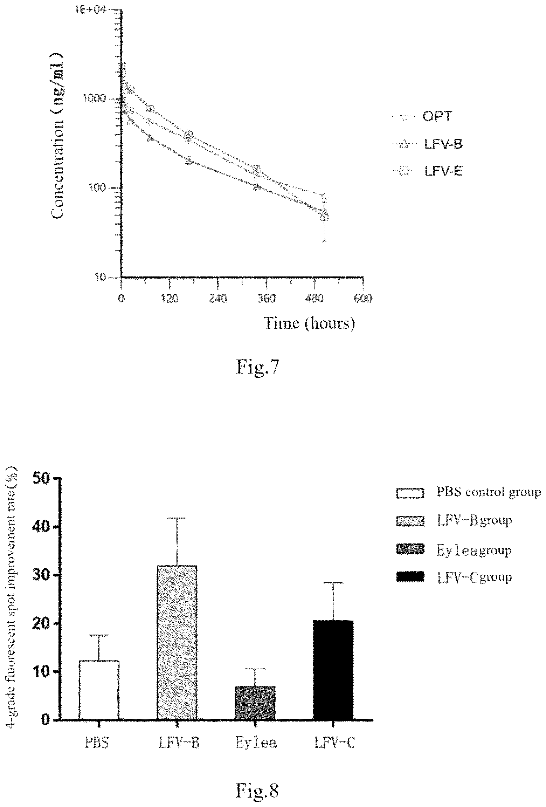

C57BL/6 mice were divided into 3 groups, with 6 mice in each group, and each group was injected via tail vein with LFV-B, LFV-E, the control article OPT at 4 mg/Kg, respectively. Blood was collected at 1h, 2h, 12h, 24h, 72h (3 days), 168h (7 days), 336h (14 days), and 504h (21 days) after the injection, and the serum was obtained by centrifugation. The molecules of interest were quantified using a capture ELISA method with anti-human Fc antibodies, and pharmacokinetic calculations were performed using WinNonlin (PhoenixTM, version 8.0) and other relevant softwares. The resulting PK data were shown in Figure 7 and Table 14 below:

**Table 14.**

| Group | T_{1/2} (hr) | AUC₀₋₃₈₄ₕ (hr*ng/mL) |
|---|---|---|
| G1: OPT | 147.79 | 154722.19 |
| G2: LFV-B | 176.09 | 106657.38 |
| G3: LFV-E | 104.22 | 205515.11 |

It could be seen that the three molecules had similar *in vivo* PK characteristics, with LFV-E having a relatively highest serum concentration and LFV-B having a relatively better half-life.

### Example 11 - Treatment experiments on laser-induced choroidal neovascularization in rats

After Brown Norway rats were anesthetized, a laser photocoagulator was used to perform photocoagulation around the optic disc, and the appearance of bubbles indicated that the Bruch's membrane had been broken. Animal grouping was performed 12 days after photocoagulation, then the drugs were administered by intraocular injection on the second day after grouping, at a volume of 4 ul and a dosage of 40 ug/eye. Fluorescence fundus angiography examination and fundus photography were performed on day 10 or day 14 after administration. The number of the 4-grade fluorescent spots for each group was counted, followed by statistical analysis.

As shown in Figure 8, it could be seen that LFV-B and LFV-C demonstrated a mitigation trend in 4-grade spots compared to the PBS and Eylea control group.

### Example 12 - Glycosylation modification design and detection

N-glycosylation sites in LFV-E (Asn46, Asn66, Asn96) and N-glycosylation sites within LFV-B, LFV-C (Asn143, Asn158, Asn245, Asn318) were subjected to point mutations to remove N-glycosylation, and Asn was preferentially mutated into Gln or Ala.

The sequences designed by point mutations were subjected to gene synthesis, vector construction, extraction, as well as cell transfection, expression and purification. Their abilities to block VEGFR2/R1-VEGFA, VEGFR2/R3-VEGFC, VEGFR2/R3-VEGFD binding were detected by ELISA, compared with those of the unmutated original molecules, and subjected to a comprehensive assessment in combination with expression levels and single peak purity. In this way, several variants were selected. HEK 293 cells were used for transfection, expression and purification, after which in vivo experiments for PK detection were performed in mice, and the results obtained were compared with those of the unmutated original molecules. The above glycosylation-modified recombinant proteins were also used to recombine with heterologous peptide, and relevant ELISA experiments, cell experiments, and animal experiments were conducted for further optimized selection.

Four N-glycosylation sites (Asn143, Asn158, Asn245 and Asn318) of LFV-C were subjected to point mutations, and their mutation designs as well as expression and purification profiles were shown in Table 15 below:

**Table 15.**

| **Trap Molecule** | **Mutation** | **Protein A one step affinity chromatograpy purified** | | **Molecular seive secondary purifed** | |
|---|---|---|---|---|---|
| | | **yield (mg/L)** | **SEC single peak purity** | **yield (mg/L)** | **SEC single peak purity** |
| **LFV-G** | **N143->A** | **30. 7** | **41%** | **/** | **/** |
| **LFV-H** | **N143->Q** | **32.8** | **48.7%** | **2** | **91.8%** |
| **LFV-I** | **N158->A** | **14.1** | **21.2%** | **/** | **/** |
| **IFV-J** | **N158->Q** | **17.3** | **42.5%** | **0.33** | **97.1%** |
| **LFV-K** | **N245->Q** | **28** | **81.4%** | **7.3** | **99.4%** |
| **LFV-L** | **N318->Q** | **54** | **76.6%** | **17.4** | **98.7%** |

Next, according to the conditions described in the Examples above, the blocking abilities of different concentrations of LFV-H, LFV-J, LFV-K and LFV-L, compared to the non-mutated LFV-C, on the binding of VEGF-A 165, VEGF-C and VEGF-D to receptors were detected by ELISA. The experimental conditions used for VEGF-A 165-VEGFR1 blocking ELISA were as follows: VEGFR1-Fc (Met1-Asn756) at 200 ng/well was used for coating overnight at 4°C, the next day, the plates were washed with PBS, then BSA solution was added to block for 2 hours at room temperature. During the blocking period, each recombinant receptor molecule was serially diluted from high to low concentrations, then mixed evenly with VEGF-A 165 (labeled with His-tag) at 40 ng/well, allowing for incubation at room temperature for 30 minutes. The VEGFR1-Fc plates were washed, and the incubation solution was added therein to allow for incubation and binding at room temperature for 1 hour, then the plates were washed with PBST, and horseradish peroxidase-conjugated anti-polyhistidine secondary antibody was added to allow for incubation and binding at room temperature for 1 hour. Subsequently, the plates were washed with PBST, and TMB was added for color development, terminated with 5% hydrochloric acid solution, and finally, the OD values at 450 nm were read using a microplate reader.

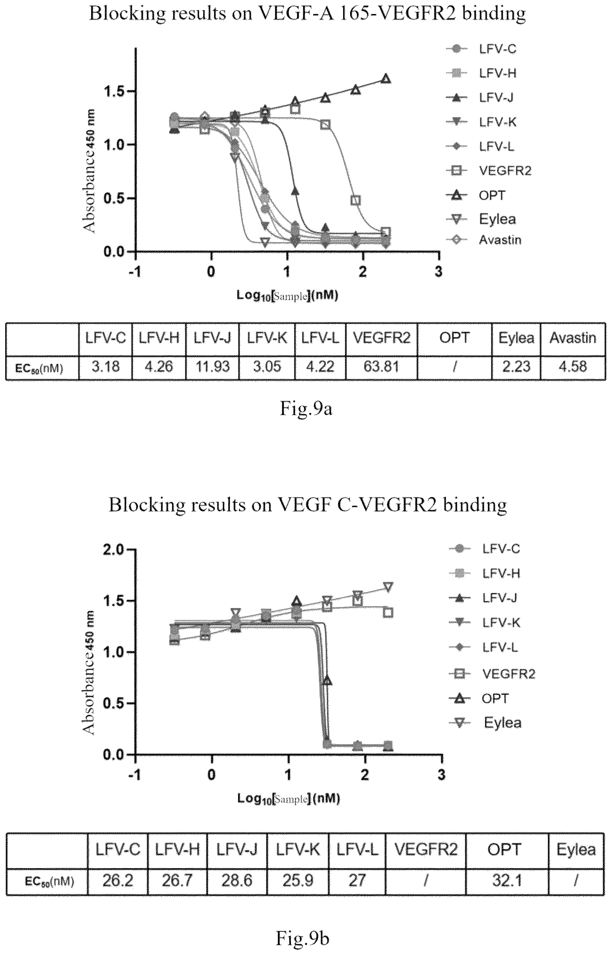

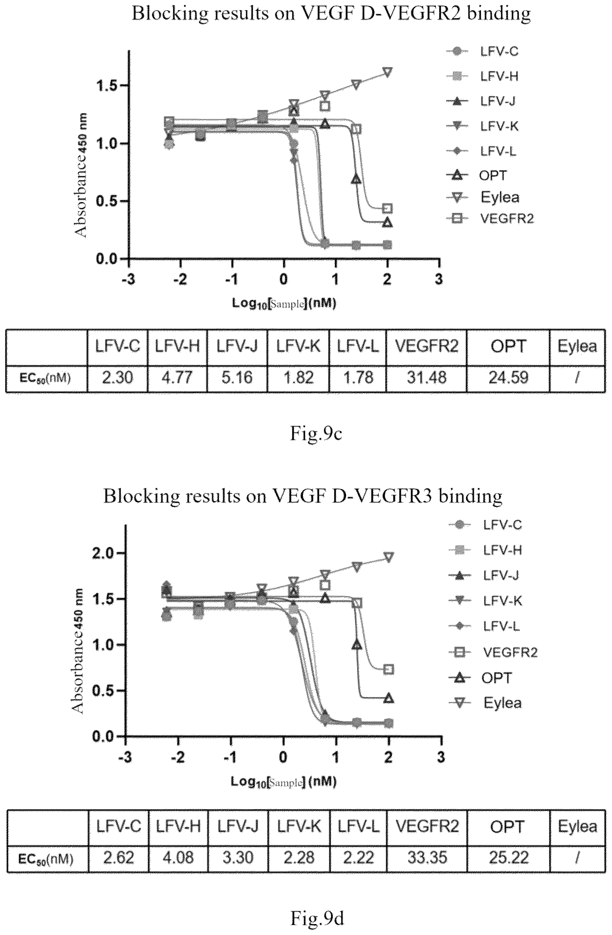

The detection results were shown in Figure 9, and it could be seen that the LFV-K molecule had a superior blocking ability on the binding of VEGF-A, VEGF-C and VEGF-D to receptors compared to that of the molecules without glycosylation engineering, thus conferring a relatively greater potential for drug development.

### Example 13 - Design of novel VEGFR2-VEGFR1 fusion proteins

To further enhance the blocking ability of recombinant receptor molecules on the binding of VEGF-A to the receptor, the region at positions 132-230 of VEGFR1, or a combined form of the region at positions 132-230 of VEGFR1 and the region at positions 225-327 of VEGFR2 was included into the LFV-B or LFV-C molecule, or glycosylation-modified molecules thereof, and transient transfection as well as expression and purification were performed in CHO cells. They were then detected for their blocking abilities on VEGFR2/R1-VEGFA, VEGFR2/R3-VEGFC and VEGFR2/R3-VEGFD using ELISA, and detected for their blocking abilities on VEGF and PDGF family molecules using the system described in Examples 8 and 9, to explore whether they possessed enhanced blocking abilities on the binding of VEGF-A to the receptor, and possessed binding abilities to VEGF-B and PlGF, while retaining better blocking abilities on the binding of VEGF-C, VEGF-D and PDGF family molecules to receptors. Moreover, Biacore was used to detect their binding abilities to PDGF family molecules, and cell or animal experiments were conducted on preferred fusion proteins.

In one experiment, the region at positions 132-230 of VEGFR1 was linked to the N terminal and C terminal of the LFV-C molecule, respectively. A combined form of the region at positions 132-230 of VEGFR1 and the region at positions 225-327 of VEGFR2 was constructed into the LFV-C molecule, with a GS linker used for linking. In this way, the full sequences of the constructed molecules, LFV-M, LFV-N, and LFV-O, were set forth in SEQ ID NO: 16, SEQ ID NO: 17, and SEQ ID NO: 46, respectively, of which LFV-M was a form in which the region at positions 132-230 of VEGFR1 was constructed at the N terminal of LFV-C, LFV-N was a form in which the region at positions 132-230 of VEGFR1 was constructed at the C terminal of LFV-C, and LFV-O was a recombinant form of VEGFR1 (132-230)-VEGFR2 (225-327) with LFV-C. Moreover, their expression and purification profiles in CHO cells were shown in Table 16:

**Table 16**

| **Trap Molecule** | **Protein A one step affinity chromatograpy purified** | | **Molecular seive secondary perifed** | |
|---|---|---|---|---|
| | **yield (mg/L)** | **SEC single peak puriry** | **yield (mg/L)** | **SEC single peak puriry** |
| **LFV-M** | **135.8** | **52. 1%** | **39.5** | **99.7%** |
| **LFV N** | **101.6** | **55.8%** | **31.8** | **99.7%** |
| **LFV-O** | **65** | **Poor** | **/** | **/** |

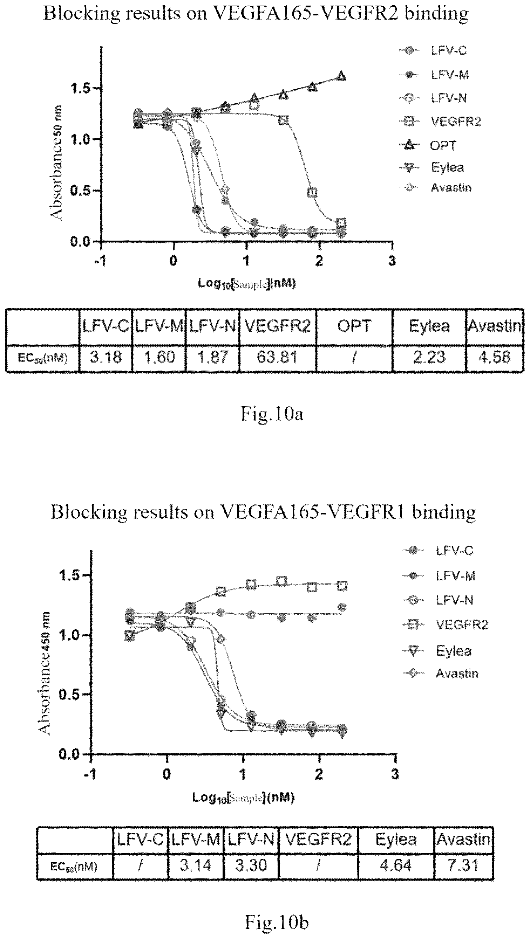

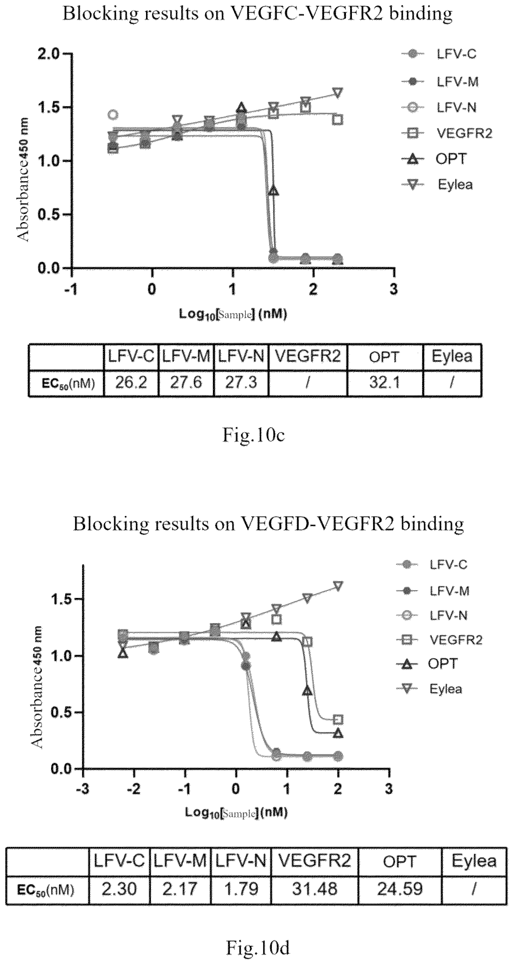

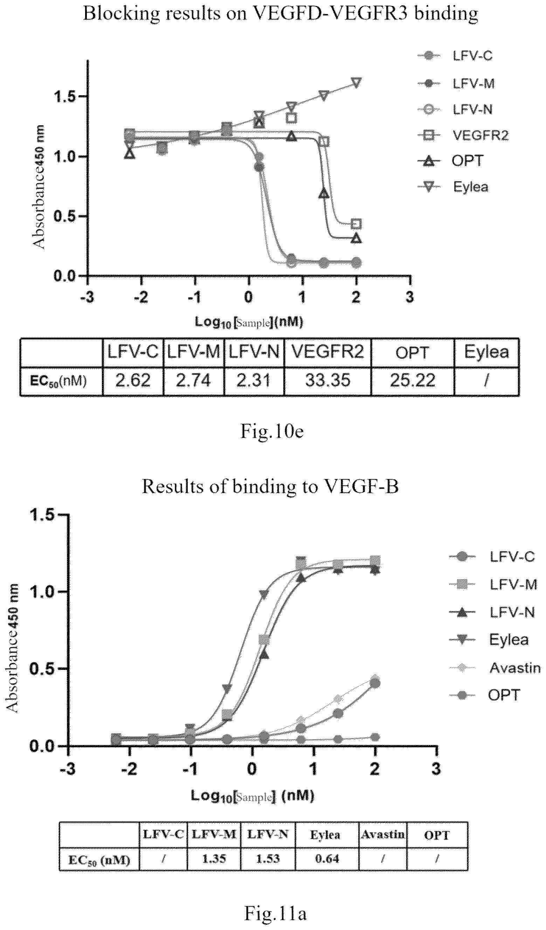

They were then detected for their blocking abilities on VEGF-A, VEGF-C, VEGF-D using ELISA, according to the conditions described in the above Examples. The results were shown in Figure 10, which showed that LFV-M and LFV-N had blocking abilities on the binding of VEGF-C, VEGF-D to receptors that were consistent with or slightly superior to those of LFV-C, and they also had significantly improved blocking abilities on the binding of VEGF-A to the receptor compared to those of LFV-C. Especially in VEGFA-VEGFR1 blocking experiments, LFV-M and LFV-N demonstrated blocking abilities superior to Eylea.

Additionally, they were detected for their binding abilities to VEGF-B and PlGF: VEGF-B or PIGF (both purchased from ACROBiosystems Inc.) at 200 ng/well prepared in PBS was used for coating overnight at 4°C, the next day, the plates were washed with PBS, and 1% BSA solution was added to block for 2 hours at room temperature, after which the plates were washed again with PBS. The recombinant receptor molecules were added to each well after serial dilution from high to low concentrations to allow for incubation at room temperature and 300 rpm for 1.5 hours, subsequently the plates were washed three times with PBST solution, and horseradish peroxidase-conjugated goat anti-human Fc secondary antibody was added, followed by incubation at room temperature and 300 rpm for 1 hour. Afterwards, the plates were washed three times with PBST solution, then TMB was added for color development, terminated with 5% hydrochloric acid solution, and finally, the OD values at 450 nm were read using a microplate reader.

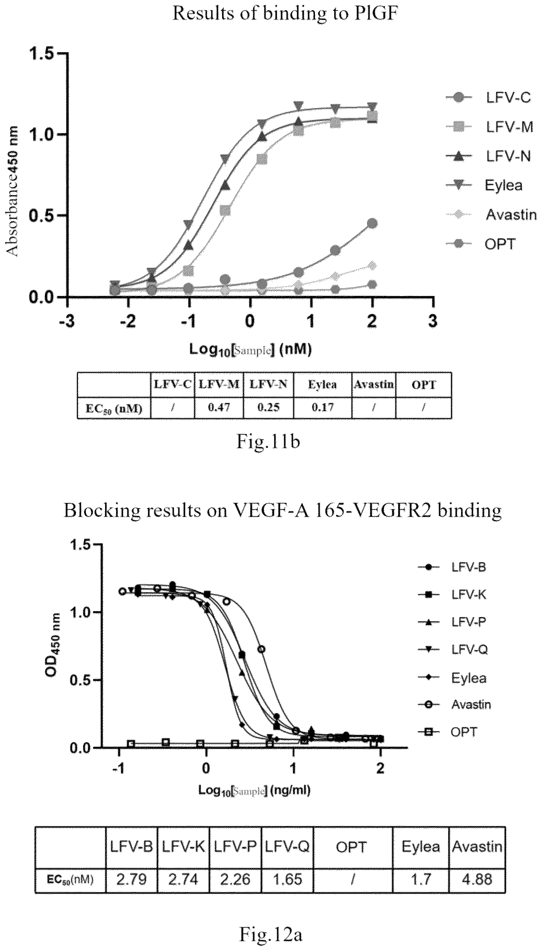

As shown in Figure 11, both LFV-M and LFV-N exhibited a certain degree of binding to VEGF-B and PlGF.

### Example 14 - Design and test of glycosylation-modified fusion proteins of VEGFR2 sequences and VEGFR1 sequences

The glycosylation-modified forms used in the LFV-K and LFV-L molecules were introduced into the LFV-B molecule, or truncated or extended forms thereof, and the obtained sequences were recombined with the region at positions 132-230 of VEGFR1 to produce new fusion proteins. The fusion proteins were expressed and purified, and subjected to characterization analyses at molecular and cellular levels as well as to animal experiments so as to obtain more potent therapeutic molecules.

In one experiment, the LFV-B molecule with N245Q mutation, named LFV-P, was used as a control, and LFV-P was combined with the 132-230 region of VEGFR1 to generate a new fusion protein, named LFV-Q. The sequence of LFV-P was set forth in SEQ ID NO: 18 and the sequence of LFV-Q was set forth in SEQ ID NO: 19. LFV-P and LFV-Q were expressed and purified, and then using the conditions described in the above examples, their abilities to block the binding of VEGF-A, VEGF-C, and VEGF-D to receptors, and their abilities to bind to VEGF-B and PIGF were detected by ELISA.

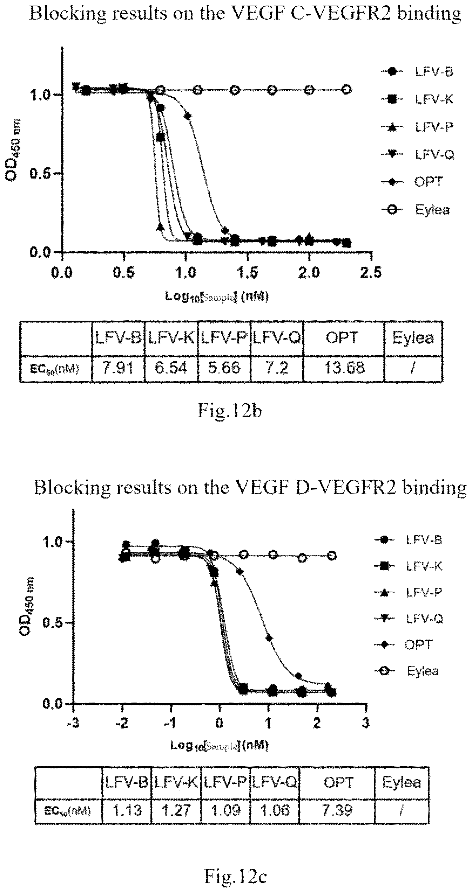

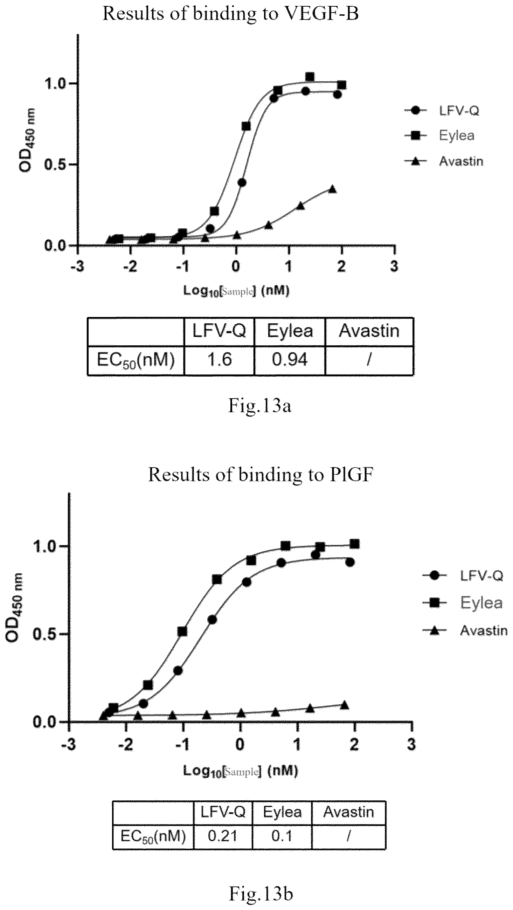

The blocking results on the binding of VEGF-A, VEGF-C and VEGF-D to VEGFR2 were shown in Figure 12, and it could be seen that after glycosylation modification, LFV-P had a superior ability to block the binding of VEGF-A, VEGF-C, and VEGF-D to receptors compared to that of LFV-B. After fusion with the 132-230 region of VEGFR1, LFV-Q had the optimal ability to block the binding of VEGF-A to the receptor. The binding results to VEGF-B and PIGF were shown in Figure 13, which showed that after fusion with the 132-230 region of VEGFR1, LFV-Q also had a better binding ability to VEGF-B and PlGF.

### Example 15 - Detection experiments for the binding of LFV-Q to PDGF family molecules

The surface plasmon resonance method was used to detect the binding of the LFV-Q molecule to PDGF family molecules, and the specific implementation steps were consistent with those described in Example 9 above. Specifically, PDGF was loaded through a coupling reaction followed by influx of the fusion protein molecules to be detected, to remove non-specific binding of the PDGF molecules to the chip.

The relevant detection results were shown in Table 17 below:

**Table 17**

| | **LFV-Q** | | |
|---|---|---|---|
| | **Ka (1/Ms)** | **Kd (1/s)** | **KD (M)** |
| **PDGF-AA** | **4.26E+04** | **4.42E-04** | **1.04E-08** |
| **PDGF-AB** | **2.96E+04** | **3.62E-03** | **1.22E-07** |
| **PDGF-BB** | **2.48E+04** | **1.70E-03** | **6.8SEOS** |
| **PDGF-CC** | **2.16E+04** | **12.14E-03** | **9.93E-08** |

It could be seen that LFV-Q had a clear binding capacity to all PDGF family molecules.

### Example 16 - Further studies on glycosylated variants of LFV-M and LFV-Q

As described above, LFV-Q was generated using a deglycosylated form at the N245 position of sequence 117-327 derived from VEGFR2, i.e., the form in which N245 was mutated to Q. To further investigate glycosylation, the N245 position within LFV-M was also mutated to Q to remove glycosylation, the resulting new molecule was named LFV-R, whose sequence was set forth in SEQ ID NO: 20. In the meantime, the Q245 position of LFV-Q was back-mutated to N for functional comparison before and after glycosylation, and the resulting molecule was named LFV-S.

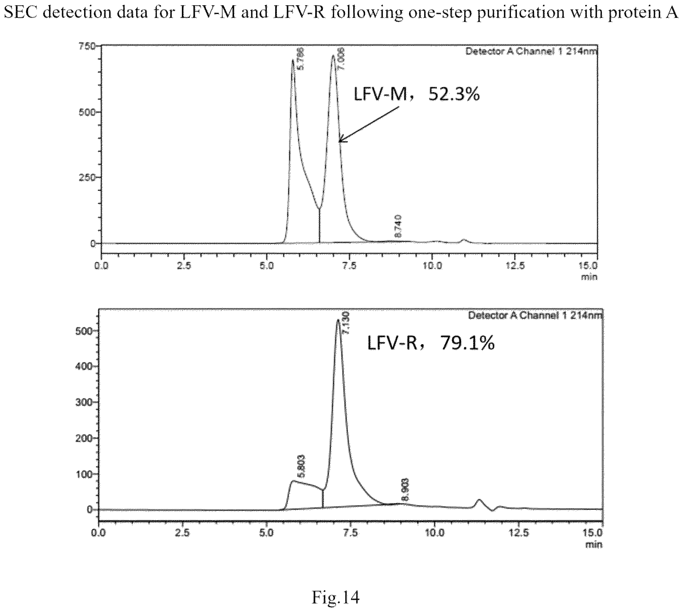

Firstly, LFV-R and LFV-S were subjected to transient transfection, expression and purification, and it was found during this process that compared with LFV-M without removal of glycosylation at position N245, LFV-R with its removal of N245 glycosylation unexpectedly demonstrated an increase in SEC single peak purity from 52.1% to 78.7% after transient transfection into CHO cells and one-step purification with protein A (as shown in Figure 14). In addition, LFV-R was concentrated in PBS after secondary purification by molecular sieve to facilitate intralenticular injection at later stage, and it was found that it still exhibited good solubility at a concentration up to 19.2 mg/ml, which reflected a better downstream development potential of LFV-R.

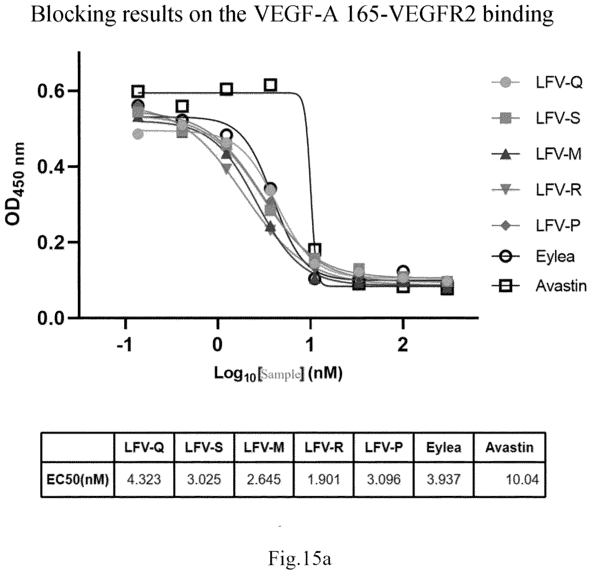

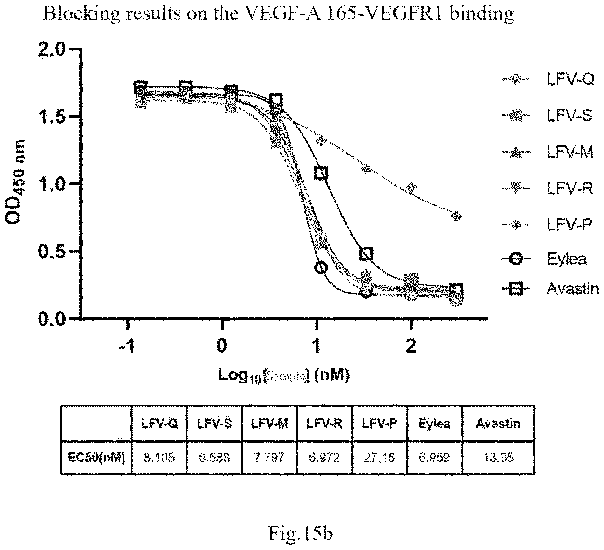

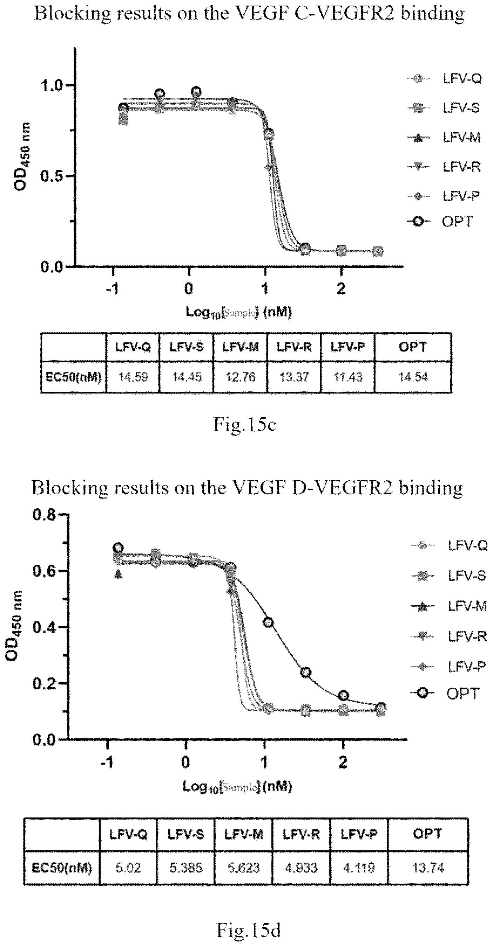

The blocking abilities of these molecules on the binding between VEGFR2/VEGFR1 and VEGF-A, between VEGFR2 and VEGF-C, and between VEGFR2 and VEGF-D were additionally detected using ELISA, and the results were shown in Figure 15. It showed that LFV-R had the optimal blocking ability on the binding between VEGFR2 and VEGF-A, significantly better than that of the control drug Eylea. Furthermore, the blocking abilities of these four molecules on the binding of VEGFR2 to VEGF-C and VEGF-D were relatively close, especially on VEGF-D, their blocking abilities were significantly better than those of the control article OPT.

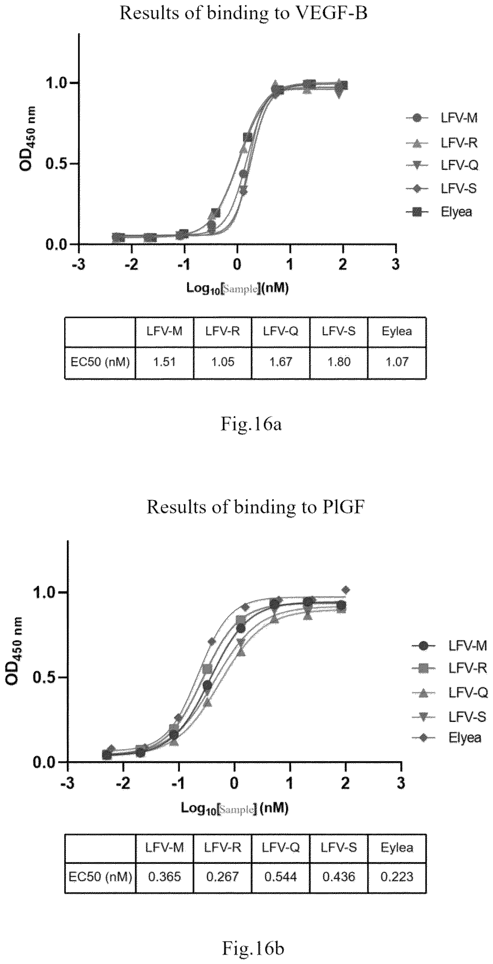

In addition, their binding abilities to PLGF and VEGF-B were also detected, and the results were shown in Figure 16, which showed that LFV-R had relatively an optimal binding ability to PIGF and VEGF-B.

### Example 17 - ELISA binding experiments of LFV-R, LFV-Q, LFV-B, LFV-C, etc. to VEGF family molecules

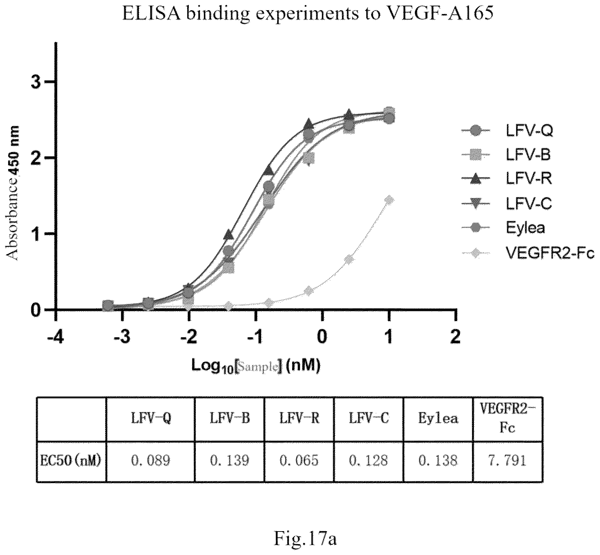

The binding of LFV-R, LFV-Q, LFV-B, and LFV-C molecules to VEGF-A was detected using ELISA methods, while VEGFR2-Fc with the full extracellular domain and Eylea were used as controls. The experimental results were shown in Figure 17a, which indicated that LFV-R had a obviously optimal binding ability to VEGF-A, higher than that of the control drug Eylea. All four molecules, LFV-R, LFV-Q, LFV-B and LFV-C, had significantly higher binding abilities to VEGF-A than those of VEGFR2-Fc with the full extracellular domain.

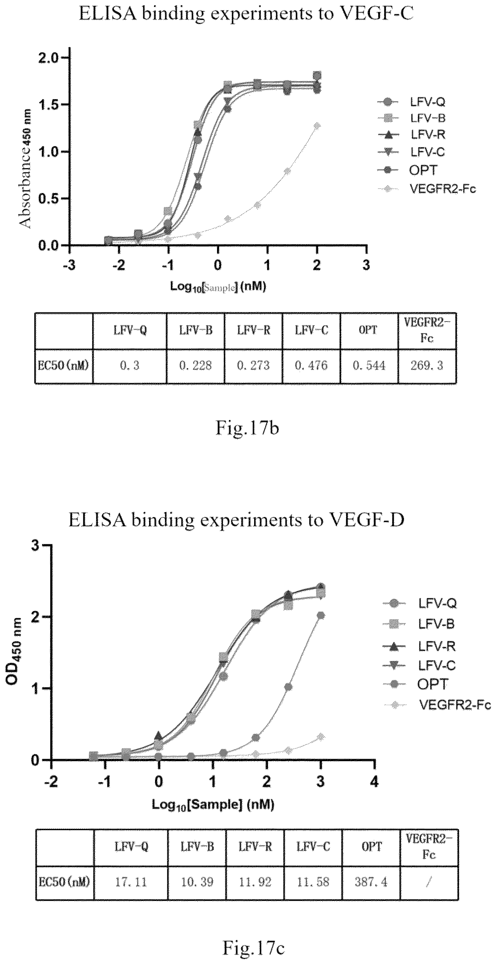

The binding of LFV-R, LFV-Q, LFV-B, and LFV-C molecules to VEGF-C was detected using ELISA methods, while VEGFR2-Fc with the full extracellular domain and OPT were used as controls. The experimental results were shown in Figure 17b, which indicated that LFV-R and LFV-Q had similar binding abilities to those of LFV-B, higher than the control article OPT. All four molecules, LFV-R, LFV-Q, LFV-B and LFV-C, had significantly higher binding abilities to VEGF-C than those of VEGFR2-Fc with the full extracellular domain.

The binding of LFV-R, LFV-Q, LFV-B, and LFV-C molecules to VEGF-D was detected using ELISA methods, while VEGFR2-Fc with the full extracellular domain and OPT were used as controls. The experimental results were shown in Figure 17c, which indicated that LFV-R and LFV-Q had similar binding abilities to those of LFV-B, significantly higher than the control article OPT. All four molecules, LFV-R, LFV-Q, LFV-B, and LFV-C, had significantly higher binding abilities to VEGF-D than those of VEGFR2-Fc with the full extracellular domain.

### Example 18 - Detection experiments for the binding of LFV-R to PDGF family molecules

The binding of the LFV-R to PDGF family molecules was detected using the surface plasmon resonance method, with specific implementation steps consistent with those described in Example 9 above. PDGF was loaded through a coupling reaction followed by influx of the fusion protein molecules to be detected, to remove non-specific binding of the PDGF molecules to the chip. The relevant detection results were shown in Table 18 below:

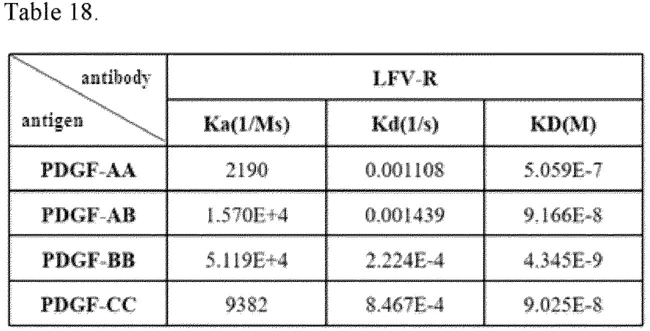

It could be seen that LFV-R had a clear binding ability to all of PDGF-AA, AB, BB, and CC.

### Example 19 - Detection experiments for cell proliferation inhibition on HUVEC

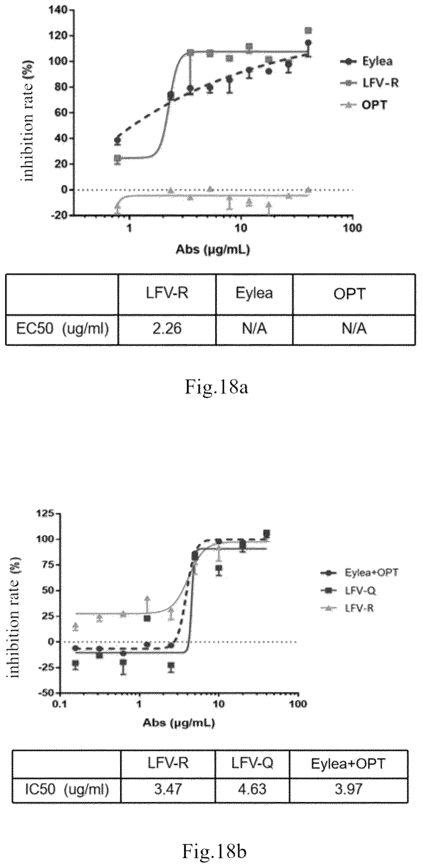

1:1 mixture of VEGF-A plus VEGF-C molecules was used to stimulate the proliferation of HUVEC cells, while different concentrations of LFV-R, Eylea, and OPT molecules were added. After the cells were cultured for 72 hours, MTS was used for chromogenic observation to detect the inhibitory potency of different drugs on cell proliferation. As shown in Figure 18a, it could be seen that only LFV-R exhibited a significant inhibitory potency, achieving a completely inhibitory effect at a concentration of about 3 ug/ml, whereas OPT exhibited no inhibitory effect, and Eylea exhibited a completely inhibitory potency only at ultra-high concentrations (above 30 ug/ml).

Furthermore, as a currently promising therapeutic approach, Eylea and OPT were mixed together (1:1 mixture) for proliferation inhibition detection on HUVEC, and LFV-R and LFV-Q were compared with the mixture. As shown in Figure 18b, Eylea mixed with OPT could completely inhibit the proliferation of HUVEC cells compared with using them separately. Moreover, LFV-R not only had a better IC₅₀ than Eylea + OPT and LFV-Q, but also exhibited a significantly higher inhibitory potency at lower concentrations compared to Eylea+OPT and LFV-Q, which would be beneficial in maintaining a sustained inhibitory efficacy even during the concentration decay period after intravitreal administration, thereby improving therapeutic benefit and prolonging injection interval.

### Example 20 - Treatment experiments on laser-induced choroidal neovascularization in rats

24 Brown-Norway rats were randomly divided into 4 groups based on body weight, with 6 animals in each group, half male and half female, which were model control group, LFV-R group, LFV-Q group and Eylea group, respectively. First, the rats underwent laser modeling on bilateral fundi, then 0.9% sodium chloride, LFV-R, LFV-Q or Eylea was given to vitreous bodies of bilateral eyes by a single injection on day 3 of modeling, respectively, at an administration volume of 4 µL/eye. The administration concentration of all groups was 10 mg/mL and at a dosage of 40 ug/eye, and the day of administration was defined as day 1 of the experiment. During the experiment, the status of surviving rats in each group was observed daily. Fundus photography and fluorescence fundus angiography examination were performed before modeling and on day 18 after administration to observe neovascular growth and leakage profiles in each group, and the drug efficacy of each group was compared.

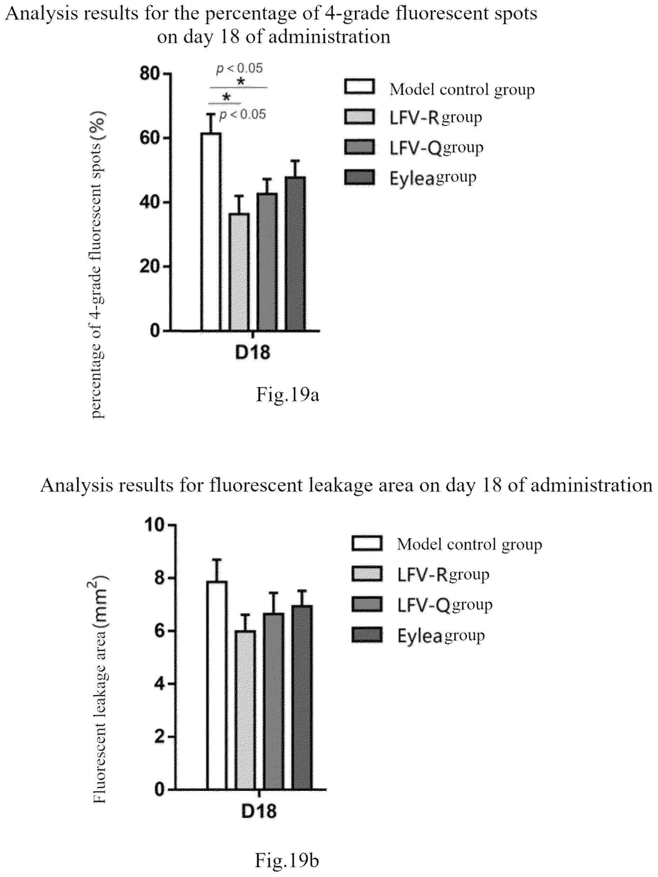

As shown in Figure 19, all three treatment groups of LFV-R, LFV-Q, and Eylea demonstrated lower 4-grade spot proportion and fluorescence leakage area compared to the model control group, and the 4-grade fluorescent spot proportion in the LFV-R and LFV-Q treatment groups revealed significantly statistical differences compared to the control group and the Eylea group.

Those skilled in the art will further appreciate that the present invention may be embodied in other specific forms without departing from the spirit or central attributes thereof. In that the foregoing description of the present invention discloses only exemplary embodiments thereof, it is to be understood that other variations are recognized as being within the scope of the present invention. Accordingly, the present invention is not limited to the particular embodiments which have been described in detail herein. Rather, reference should be made to the appended claims as indicative of the scope and content of the present invention.

## Claims

1. An isolated ligand-binding molecule comprising a VEGFR2-derived sequence, the VEGFR2-derived sequence is an amino acid sequence that has at least 95% identity to any of the following amino acid sequences:
(a) Amino acid sequence as set forth at positions 123-327 of SEQ ID NO: 2;
(b) Amino acid sequence as set forth at positions 117-327 of SEQ ID NO: 2;
(c) Amino acid sequence as set forth at positions 117-421 of SEQ ID NO: 2;
(d) Amino acid sequence as set forth at positions 23-327 of SEQ ID NO: 2; and
(e) Amino acid sequence as set forth at positions 23-421 of SEQ ID NO: 2;
wherein the ligand-binding molecule can bind to VEGF-A, VEGF-C, VEGF-D, PDGF-AA, PDGF-AB, PDGF-BB and PDGF-CC.

2. The ligand-binding molecule of claim 1, having an enhanced binding ability to at least three of VEGF-A, VEGF-C, VEGF-D, PDGF-AA, PDGF-AB, PDGF-BB, and PDGF-CC compared to that of native VEGFR2.

3. The ligand-binding molecule of claim 1 or 2, wherein the VEGFR2-derived sequence is an N-terminal and/or C-terminal truncated fragment of any one of (a)-(e), for example, with 20, 15, 10, 9, 8, 7, 6, 5, 4, 3, 2 or 1 amino acid(s) truncated.

4. The ligand-binding molecule of any one of claims 1-3, wherein the VEGFR2-derived sequence comprises an insertion, substitution or deletion of one or more amino acids compared to the sequence at the corresponding position in native VEGFR2, optionally an insertion, deletion or substitution at a glycosylation site to eliminate an NXT or NXS tri-amino acid, for example, N mutated to Q or A.

5. The ligand-binding molecule of claim 4, wherein the glycosylation site is selected from the group consisting of amino acid positions 46, 66, 96, 143, 158, 245 and 318 corresponding to SEQ ID NO: 2.

6. The ligand-binding molecule of claim 5, wherein the substitution at the glycosylation site is selected from the following: N143A, N143Q, N158A, N158Q, N245Q and N318Q.

7. The ligand-binding molecule of any one of the preceding claims, wherein the VEGFR2-derived sequence comprises or consists of an amino acid sequence selected from the group consisting of: SEQ ID Nos: 4-15 and 18.

8. The ligand-binding molecule of any one of the preceding claims, further comprising a VEGFR1-derived sequence or a VEGFR3-derived sequence.

9. The ligand-binding molecule of claim 8, wherein the VEGFR1-derived sequence is selected from the group consisting of:
(i) an amino acid sequence having at least 95% identity to the amino acid sequence as set forth at positions 132-230 of SEQ ID NO: 1; and
(ii) an N-terminal and/or C-terminal truncated amino acid sequence of the amino acid sequence of (i), for example, with 20, 15, 10, 9, 8, 7, 6, 5, 4, 3, 2 or 1 amino acid(s) truncated.

10. The ligand-binding molecule of claim 8 or 9, comprising an amino acid sequence having at least 95% identity to the amino acid sequence as set forth at positions 117-327 or 123-327 of SEQ ID NO: 2 operably linked to the VEGFR1-derived sequence.

11. The ligand-binding molecule of claim 10, comprising or consisting of an amino acid sequence selected from the group consisting of: SEQ ID Nos: 16-17 and 19-21.

12. The ligand-binding molecule of claim 8, wherein the VEGFR3-derived sequence is selected from the group consisting of:
(i) an amino acid sequence having at least 95% identity to the amino acid sequence as set forth at positions 25-216 of SEQ ID NO: 3; and
(ii) an N-terminal and/or C-terminal truncated amino acid sequence of the amino acid sequence of (i), for example, with 20, 15, 10, 9, 8, 7, 6, 5, 4, 3, 2 or 1 amino acid(s) truncated.

13. The ligand-binding molecule of claim 8 or 12, comprising an amino acid sequence having at least 95% identity to the amino acid sequence as set forth at positions 117-327, 123-327, or 221-327 of SEQ ID NO: 2, operably linked to the VEGFR3-derived sequence.

14. The ligand-binding molecule of claim 13, comprising or consisting of an amino acid sequence selected from the group consisting of: SEQ ID Nos: 22-30.

15. The ligand-binding molecule of any one of the preceding claims, further comprising an Fc region of an immunoglobulin, preferably an Fc region of human IgG1 or a variant thereof, optionally the ligand-binding molecule also comprising a hinge region.

16. The ligand-binding molecule of claim 15, wherein the Fc region of the immunoglobulin is linked to the C-terminal of the VEGFR2-derived sequence, the VEGFR1-derived sequence, or the VEGFR3-derived sequence via a linker.

17. The ligand-binding molecule of claim 15 or 16, wherein the Fc region is as set forth in the sequence of amino acids 104-330 of SEQ ID No: 31.

18. The ligand-binding molecule of any one of claims 15-17, comprising or consisting of any sequence of SEQ ID Nos: 32-59.

19. An isolated nucleic acid molecule comprising a polynucleotide sequence encoding the ligand-binding molecule of any one of claims 1-18.

20. A vector comprising the nucleic acid molecule of claim 19.

21. A host cell transformed or transfected with the nucleic acid molecule of claim 19 or the vector of claim 20.

22. A method for preparing a ligand-binding molecule comprising the steps of:
- culturing the host cell of claim 21 under suitable conditions to express the ligand-binding molecule; and
- isolating the ligand-binding molecule from the culture supernatant of the host cell.

23. A pharmaceutical composition comprising the ligand-binding molecule of any one of claims 1-18 and a pharmaceutically acceptable carrier.

24. The pharmaceutical composition of claim 23, formulated for topical administration, intravitreal injection or intravitreal implant administration.

25. The ligand-binding molecule of any one of claims 1-18 for use in treating neovascularization or a disease or condition caused by neovascularization in a subject.

26. The ligand-binding molecule for use according to claim 25, wherein ocular diseases caused by neovascularization include ocular diseases caused by retinal neovascularization and choroidal neovascularization.

27. The ligand-binding molecule for use according to claim 25, wherein the disease or condition is selected from the group consisting of choroidal vasculopathy or neovascularization, retinal neovascularization, macular neovascularization and ocular disorders associated with fundus leakage.

28. The ligand-binding molecule for use according to any one of claims 25-27, wherein the ligand-binding molecule is administered by intravitreal injection or by intravitreal implant administration.

29. A method for preventing, inhibiting or treating neovascularization or a disease or condition caused by neovascularization in a subject, the method comprises administering the ligand-binding molecule of any one of claims 1-18 or the pharmaceutical composition of claim 23 or 24 to the subject in an amount effective to inhibit neovascularization in the subject.

30. A kit comprising the ligand-binding molecule of any one of claims 1-18 or the pharmaceutical composition of claim 23 or 24 in a container.
